(19) Europäisches Patentamt
European Patent Office
Office européen des brevets

(11) EP 0 642 530 B1

(12) EUROPEAN PATENT SPECIFICATION

(45) Date of publication and mention of the grant of the patent:
**12.08.1998 Bulletin 1998/33**

(21) Application number: **93909931.3**

(22) Date of filing: **10.05.1993**

(51) Int Cl.6: **C07K 5/10**, C07K 7/06, A61K 38/04

(86) International application number:
**PCT/EP93/01138**

(87) International publication number:
**WO 93/23424 (25.11.1993 Gazette 1993/28)**

# (54) DERIVATIVES OF DOLASTATIN

Derivate des Dolastatin

DERIVES DE DOLASTATINE

(84) Designated Contracting States:
**AT BE CH DE DK ES FR GB GR IE IT LI LU NL PT SE**

(30) Priority: **20.05.1992 US 885788**
**25.11.1992 US 985696**

(43) Date of publication of application:
**15.03.1995 Bulletin 1995/11**

(73) Proprietor: **BASF Aktiengesellschaft**
**67063 Ludwigshafen (DE)**

(72) Inventors:
- **HAUPT, Andreas**
  **D-6700 Ludwigshafen (DE)**
- **EMLING, Franz**
  **D-6700 Ludwigshafen (DE)**
- **ROMERDAHL, Cynthia**
  **Wayland, MA 0177 (US)**

(56) References cited:
**EP-A- 0 398 558**

- **J.AM.CHEM.SOC. vol. 113, 1991, pages 6692 - 6692 PETTIT, G.R. ET AL. 'Antineoplastic Agents. 220. Synthesis of Natural (-)-Dolastatin 15'**
- **BIOCHEMICAL PHARMACOLOGY vol. 40, no. 8, 1990, pages 1859 - 1864 BAI, R. ET AL. 'Structure-activity studies with chiral isomers and with segments of the antimitotic marine peptide dolastatin 10'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The invention described herein provides novel peptides and derivatives thereof which offer potentially improved therapeutic utilities for the treatment of neoplastic diseases as compared to Dolastatin-10 and -15 (US Patent No 4,879,278, Nov. 7, 1989; US Patent No 4,816,444, Mar. 28, 1989). Furthermore, unlike dolastatin-10 and -15 which must be laboriously purified from scarce natural sources, the compounds of this invention may be conveniently synthesized as described in detail below. In addition, Dolastatin-10 is unstable to acid. It was described that even minor changes in the structure can cause complete loss of activity (Biochemical Pharmacology, vol. 40, no. 8, 1859-64, 1990).

Compounds of this invention include novel peptides of the formula I

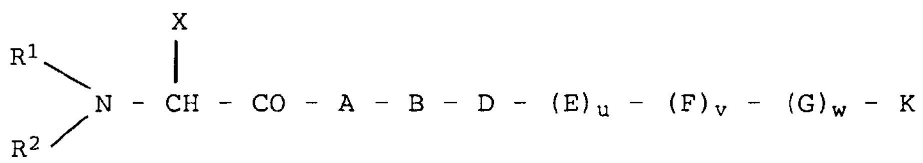

I

where

$R^1$ alkyl, preferably $C_{1-7}$; cycloalkyl, preferably $C_{3-6}$; alkylsulfonyl, preferably $C_{1-6}$; fluoroalkyl, preferably fluoroethyl, difluoroethyl, trifluoroethyl, fluoroisopropyl, trifluoroisopropyl; or $NR^3R^4$ where $R^3$ and $R^4$ may each be either hydrogen or alkyl, preferably $C_{1-4}$;

$R^2$ is hydrogen; alkyl, preferably $C_{1-4}$; fluoroalkyl, preferably fluoroethyl, difluoroethyl, trifluoroethyl, fluoroisopropyl, trifluoroisopropyl; cycloalkyl, preferably $C_{3-7}$;

$R^1$-N-$R^2$ together are

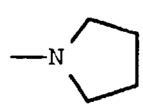 or 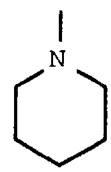 ;

A is a valyl, isoleucyl, leucyl, allo-isoleucyl, 3-tert-butylalanyl, 2-tert-butylglycyl, 3-cyclohexylalanyl, 2-ethylglycyl, 2-cyclohexylglycyl, norleucyl, norvalyl residue;

B is a N-alkyl-valyl, norvalyl, -leucyl, -isoleucyl, -2-tert-butylglycyl, -3-tert-butylalanyl, -3-cyclohexylalanyl, -phenylalanyl or -2-cyclohexylglycyl residue where N-alkyl is preferably N-methyl or N-ethyl;

D and E are independently selected from the group consisting of prolyl, homoprolyl, hydroxyprolyl and thiadiazolidinyl-4-carbonyl;

F and G are independently selected from the group consisting of prolyl, homo-prolyl, hydroxyprolyl, thiazolidinyl-4-carbonyl, 1-aminopentyl-1-carbonyl, valyl, 2-tert-butylglycyl, isoleucyl, leucyl, 3-cyclohexylalanyl, phenylalanyl, N-methylphenylalanyl, tetrahydroisoquinolyl-2-carbonyl, 3-thiazolylalanyl, 3-thienylalanyl, histidyl, 1-aminoindyl-1-carbonyl, 3-pyridylalanyl, 3-tert-butylalanyl, 2-cyclohexylglycyl, norvalyl, norleucyl and 3-naphthylalanyl residues

X is alkyl (preferably linear or branched $C_{1-5}$), cycloalkyl (preferably cyclohexyl), -$CH_2$-cyclohexyl or arylalkyl (preferably benzyl or phenethyl);

E and F together, may be

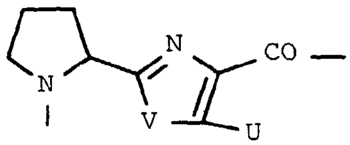

where V is oxygen or sulfur and U is hydrogen, $C_1$-$C_4$-alkyl, phenyl, or cycloalkyl (preferably cyclohexyl);

u,v, and w are independently 0 or 1; and

K is hydroxy, alkoxy (preferably $C_{1-4}$), phenoxy, benzyloxy or a substituted or unsubstituted amino moiety;

and the salts thereof with physiologically tolerated acids.

This invention also provides methods for preparing the compounds of formula I, pharmaceutical compositions containing such compounds together with a pharmaceutically acceptable carrier and methods for using same for treating cancer in mammals.

One subclass of compounds of this invention includes compounds of formula I wherein $R^1$-N-$R^2$ is

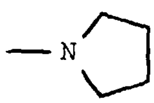

or

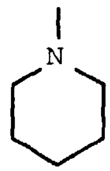

Another subclass of compounds of this invention includes compounds of formula I wherein K is an amino moiety of the formula $R^5$-N-$R^6$ wherein

$R^5$ is hydrogen, or hydroxy, or $C_{1-7}$-alkoxy, or benzyloxy, or $C_{1-7}$-alkyl which may be substituted by one or more fluoro atoms, or $C_{3-7}$-cycloalkyl, or benzyl which may be substituted by up to three substituents which may independently be $CF_3$, nitro, $C_{1-7}$-alkylsulfonyl, $C_{1-4}$-alkoxy, phenoxy, benzoxy, halogen or $C_{1-4}$-alkyl

$R^6$ is hydrogen, or $C_{1-7}$-alkyl which may be substituted by one or more fluoro atoms, or $C_{3-7}$-cycloalkyl, or phenyl (which may be substituted by up to three substituents which may independently be $CF_3$, nitro, halogen, CONHBzl, $CON(Bzl)_2$, $C_{1-4}$-alkyl which may form a cyclic system, $C_{1-4}$-alkoxy, phenoxy, benzoxy, or $C_{1-7}$-alkyl-sulfonyl), or benzyl (which may be substituted by up to three substituents which may independently be $CF_3$, nitro, halogen, CONHBzl, $CON(Bzl)_2$, $C_{1-4}$-alkyl which may form a cyclic system, $C_{1-4}$-alkoxy, phenoxy, benzoxy, or $C_{1-7}$-alkyl-sulfonyl), or
naphthyl (which may be substituted by up to two substituents which may independently be $CF_3$, nitro, halogen, CONHBzl, $CON(Bzl)_2$, $C_{1-4}$-alkyl, $C_{1-4}$-alkoxy, benzoxy, phenoxy, or $C_{1-7}$-alkyl-sulfonyl), or
benzhydryl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or
biphenyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or
triphenylmethyl (which may be substituted by up to three substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or
benzhydrylethyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or
benzhydrylmethyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy or $C_{1-7}$-alkyl-sulfonyl), or
naphthylmethyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or
acenaphthyl (which may be substituted by up to two substituents which may independently be $CF_3$, nitro, halogen,

CONHBzl, $CON(Bzl)_2$, $C_{1-4}$-alkyl, $C_{1-4}$-alkoxy, phenoxy, benzoxy, or $C_{1-7}$-alkyl-sulfonyl), or acenaphthylmethyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or pyridyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or picolyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or benzothiazolyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or benzisothiazolyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or benzopyrazolyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or benzoxazolyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or fluorenyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or aminofluorenyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or pyrimidyl (which may be substituted by up to two substituents which may independently be $CF_3$, nitro, halogen, COOEt, CONHBzl, $CON(Bzl)_2$, $C_{1-4}$-alkyl which may form a cyclic system, $C_{1-4}$-alkoxy, phenoxy, benzoxy, or $C_{1-7}$-alkyl-sulfonyl), or 5-membered heteroaryl [which may be substituted by up to three substituents which may independently be $CF_3$, nitro, halogen, cyano, COOMe, COOEt, thiomethyl, thioethyl, thiophenyl, picolyl,acetyl, $-CH_2$-COOEt, $CONH_2$ CONHBzl, $CON(Bzl)_2$, $C_{1-4}$-alkyl, $C_{3-6}$-cycloalkyl, $C_{3-4}$-alkylen group forming a bicyclic system with the heterocycle, $C_{1-4}$-alkoxy, phenoxy, benzoxy, phenyl (which may be substituted by up to four substituents which may independently be nitro, $CF_3$, halogen, or $C_{1-4}$-alkyl), benzyl (which may be substituted by up to four substituents which may independently be nitro, $CF_3$, halogen, $C_{1-4}$-alkyl, naphthyl, $C_{1-7}$-alkyl-sulfonyl, phenylsulfonyl, or $C_{1-4}$-dialkylamino)], or $-CHR^7$-5-membered heteroaryl (which may be substituted by up to two substituents which may independently be $CF_3$, nitro, halogen, CONHBzl, $CON(Bzl)_2$, COOMe, COOEt, $COOCH(CH_3)_2$, $CONH_2$, COOBzl, $C_{1-4}$-alkyl, $C_{1-4}$-alkoxy, phenoxy, benzoxy, phenyl, benzyl, naphthyl, or $C_{1-7}$-alkyl-sulfonyl [$R^7$ = hydrogen, linear or branched $C_{1-5}$-alkyl, benzyl; or $R^7$ and $R^5$ together form a group $-(CH_2)_3$- or $-(CH_2)_4$-]).

This subclass includes compounds of formula I wherein u, v and w are independently 0 or 1; $R^1$ is $C_{1-7}$-alkyl, $R^2$ is $C_{1-4}$-alkyl, A and F are lower alkyl amino acids, B is a N-loweralkylated lower alkyl amino acid; D, E, G and K are as previously defined. With the foregoing in mind, one set of such compounds can thus be depicted by the following formula II:

$$R^1R^2N—CXH—CO—A—B—Pro\text{-}Pro—(F)_v—(G)_w—K \qquad II$$

and another by the following formula III

$$R^1R^2N—CXH—CO—A—B—Pro—(F)_v—(G)_w—K. \qquad III$$

In another subclass of compounds of this invention $R^5$-N-$R^6$ together may form structures selected from the group consisting of

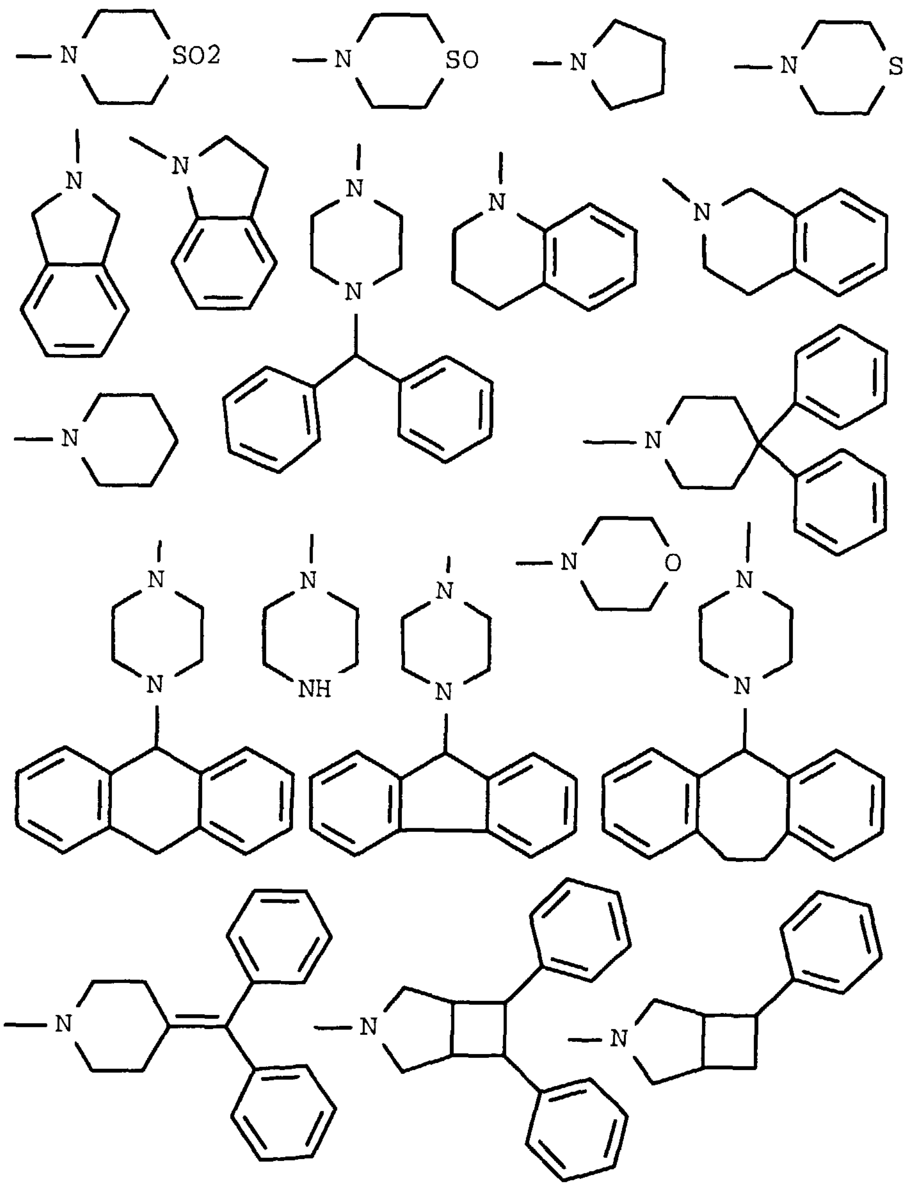

which may be unsubstituted or substituted with one or more substituents independently selected from the group consisting of $CF_3$, nitro, halogen, oxo, cyano, N,N-dimethylamino, CONHBzl, $CON(Bzl)_2$, $C_{1-6}$-alkyl, $C_{3-6}$-cycloalkyl, $C_{3-4}$-alkylen group forming a bicyclic system with the heterocycle,
$C_{1-4}$-alkoxy, phenoxy, benzoxy, naphthyl, pyrimidyl, COOEt, COOBzl, $C_{3-6}$-cycloalkyl, pyrolidinyl, piperidinyl, thienyl, pyrolyl, $-CH_2-CO-NCH(CH_3)_2$, $-CH_2-CO-N(CH_2)_4$, $-CH_2-CO-N(CH_2)_4O$, benzyl (which may be substituted by up to three substituents independently selected from the group consisting of nitro, halogen, $CF_3$, thiomethyl or the corresponding sulfoxide or sulfone, thioethyl or the corresponding sulfoxide or sulfone, $C_{1-4}$-alkyl, and $C_{1-4}$-alkoxy), and phenyl (which may be substituted by up to three substituents independently selected from the group consisting of nitro, halogen, $CF_3$, thiomethyl, thioethyl, $C_{1-4}$-alkyl, and $C_{1-4}$-alkoxy).

Another subclass of compounds of this invention includes for example compounds of formula I wherein u, v, and w are zero and K is not an hydroxy, benzoxy, phenoxy or alkoxy moiety.

Another subclass of compounds of this invention includes for example compounds of formula I wherein u, and v are zero and K is not an hydroxy or alkoxy moiety.

Still another subclass of compounds of this invention includes for example compounds of formula I wherein u, v and w are 1 and K is a hydroxy, alkoxy, phenoxy or benzyloxy moiety.

Yet another subclass of compounds of this invention includes for example compounds of formula I wherein u and v are 1, w is O and K is a hydroxy, alkoxy, phenoxy or benzyloxy moiety.

Another subclass of compounds of this invention includes for example compounds of formula I wherein u is 1, v and w are 0 and K is a hydroxy, alkoxy, phenoxy or benzyloxy moiety.

Preferred are compounds of formula I where the substituents have the following meanings:

$R^1$ is ethyl, methyl, 2-fluoroethyl, 2,2-difluoroethyl, 2,2,2-trifluoroethyl, 2-fluoroisopropyl, trifluoroisopropyl, isopropyl, propyl, butyl, pentyl, cyclopropyl, cyclopentyl, mesyl, 2,4,6-trimethylsulfonyl, $NH_2$, $N(CH_3)_2$, $N(CH_2CH_3)_2$, $N[CH(CH_3)_2]_2$

$R^2$ is hydrogen, methyl, ethyl, 2-fluoroethyl, 2,2-difluoroethyl, 2,2,2-trifluoroethyl, 2-fluoroisopropyl, trifluoroisopropyl, isopropyl, propyl, butyl, cyclopropyl

or $R^1$-N-$R^2$ together is one of the following residues:

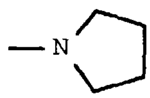
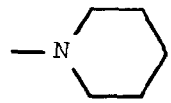

A, B, D, E, F, G and X have the meanings as described above;
u, v and w are independently 0 or 1;
E and F together are

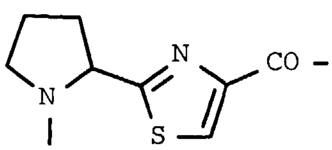
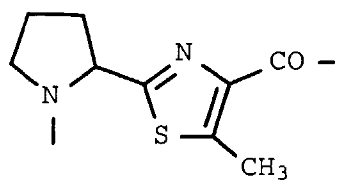
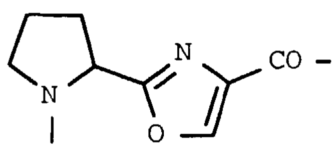
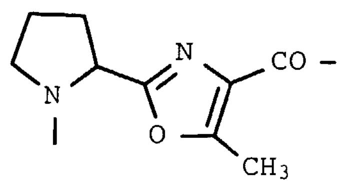
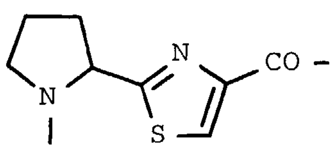
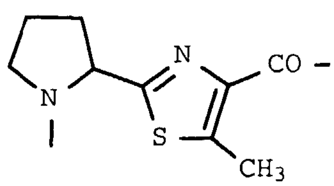
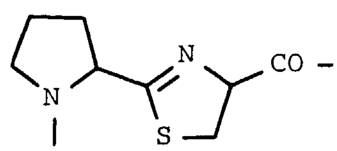
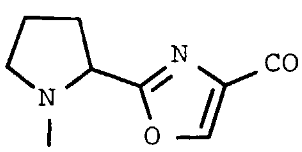
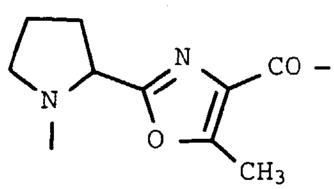

K is $-NR^5R^6$, wherein
$R^5$ is hydrogen, methyl, ethyl, 2-fluoroethyl, 2,2-difluoroethyl, trifluoroisopropyl, propyl, isoproypyl, or

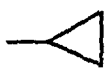
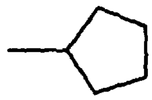
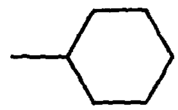
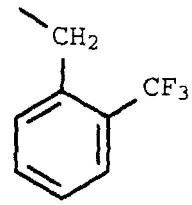

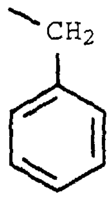
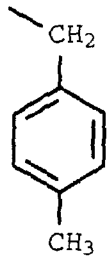
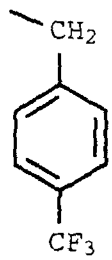
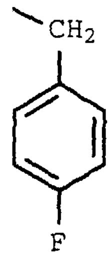
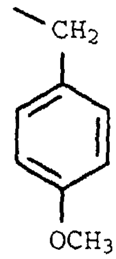
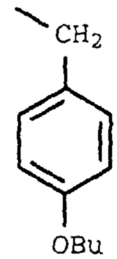

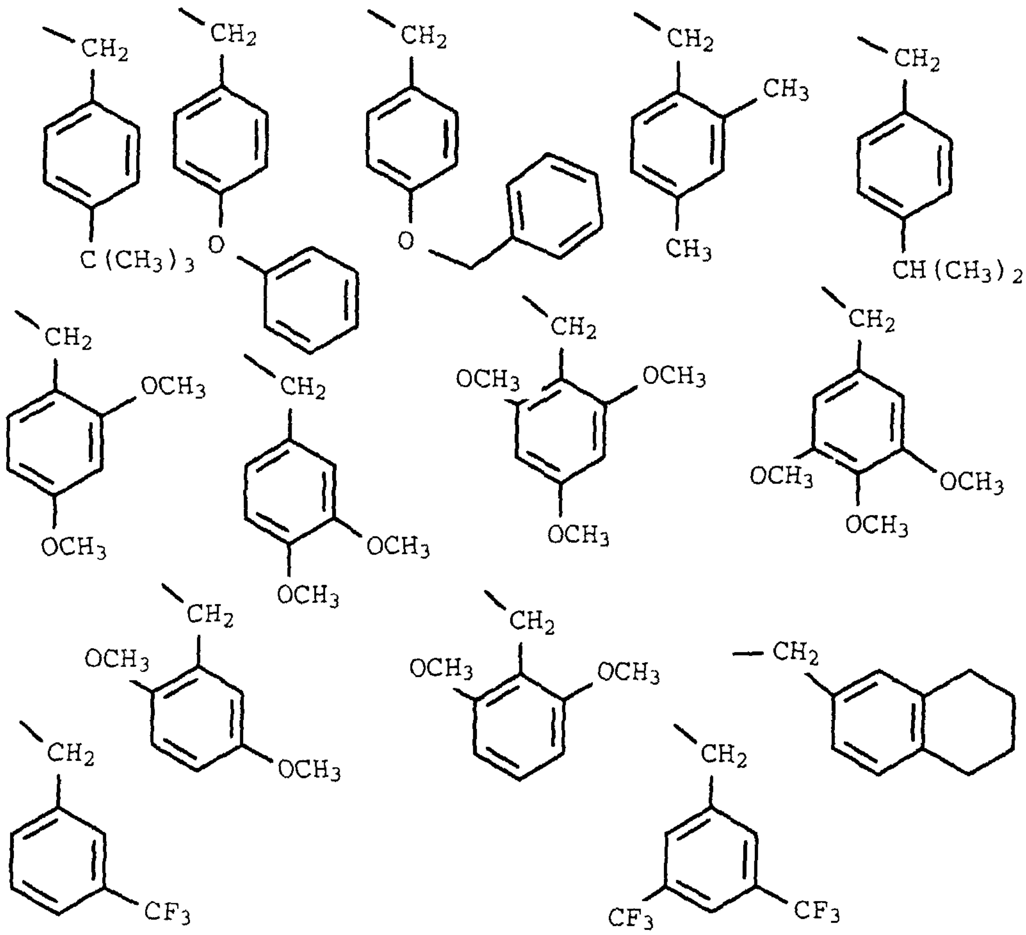

$R^6$ is hydrogen, methyl, ethyl, 2-fluoroethyl, 2,2-difluoroethyl, trifluoroethyl, trifluoroisopropyl, propyl, isopropyl, tert-butyl, or

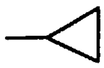
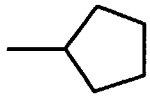
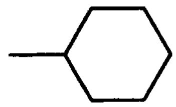
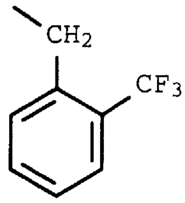

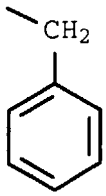
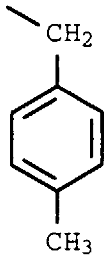
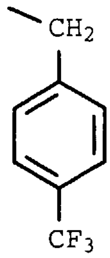
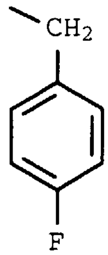
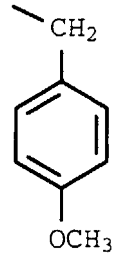
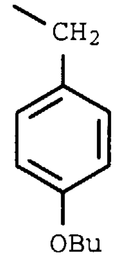

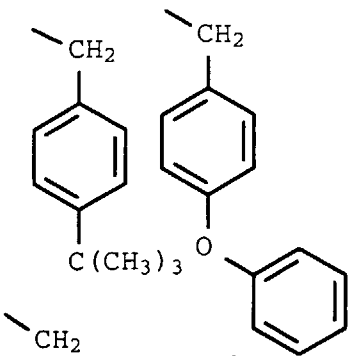
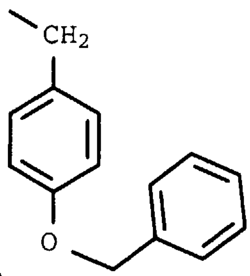
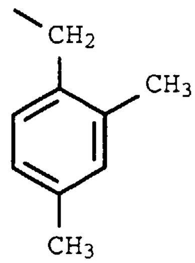
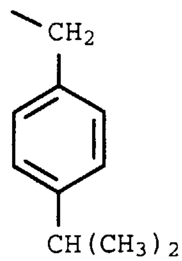

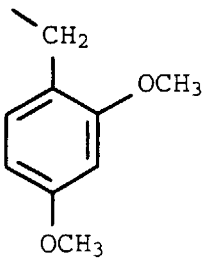
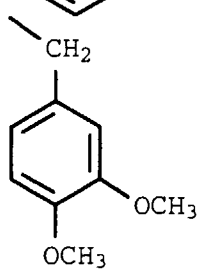
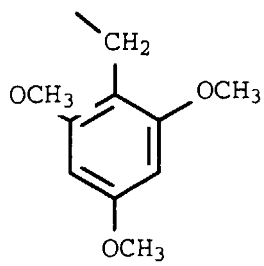
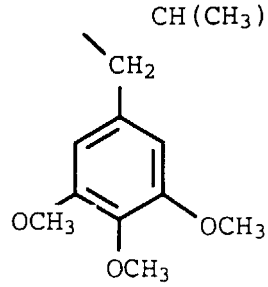

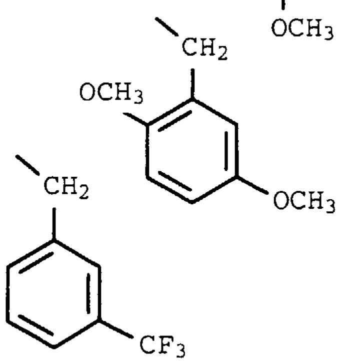
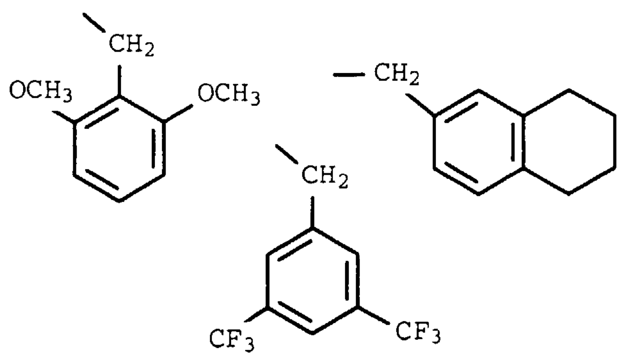

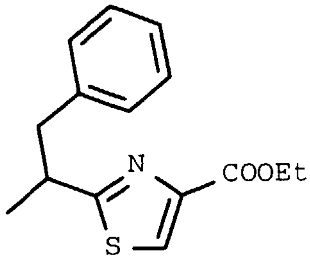
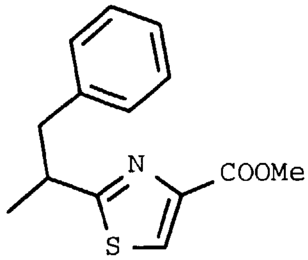
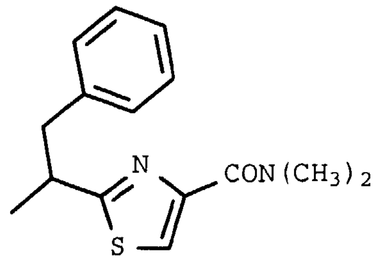

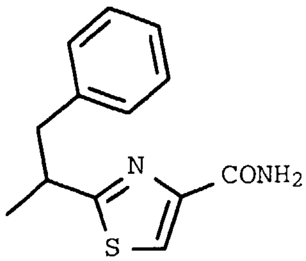
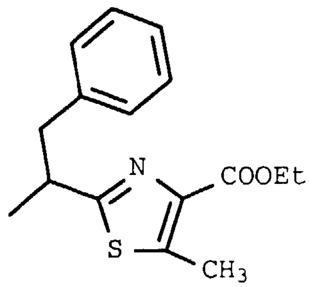
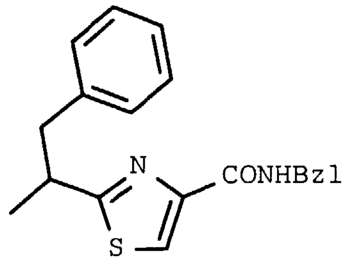

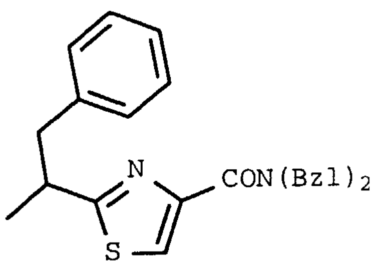
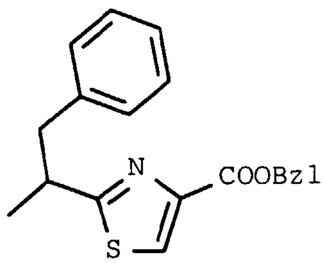
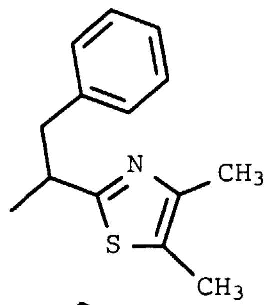

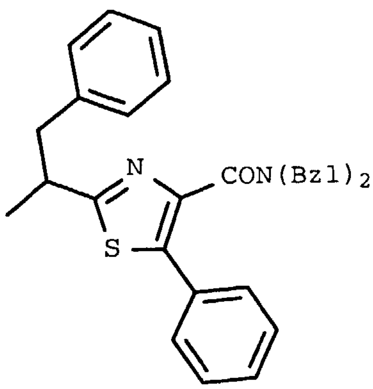
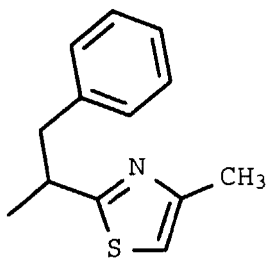
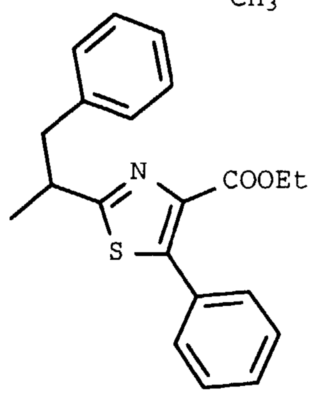

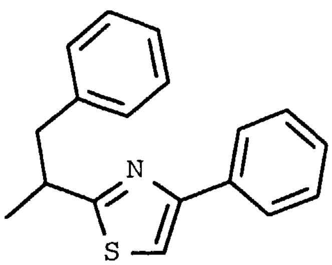
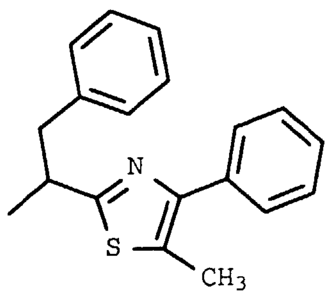
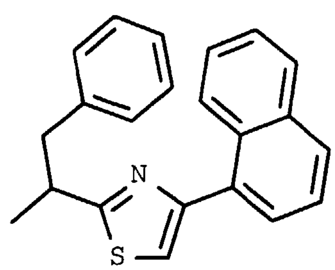

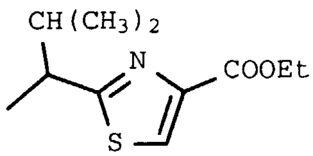
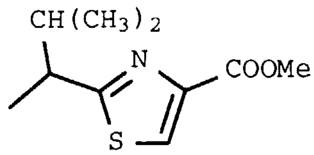
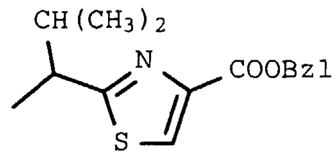

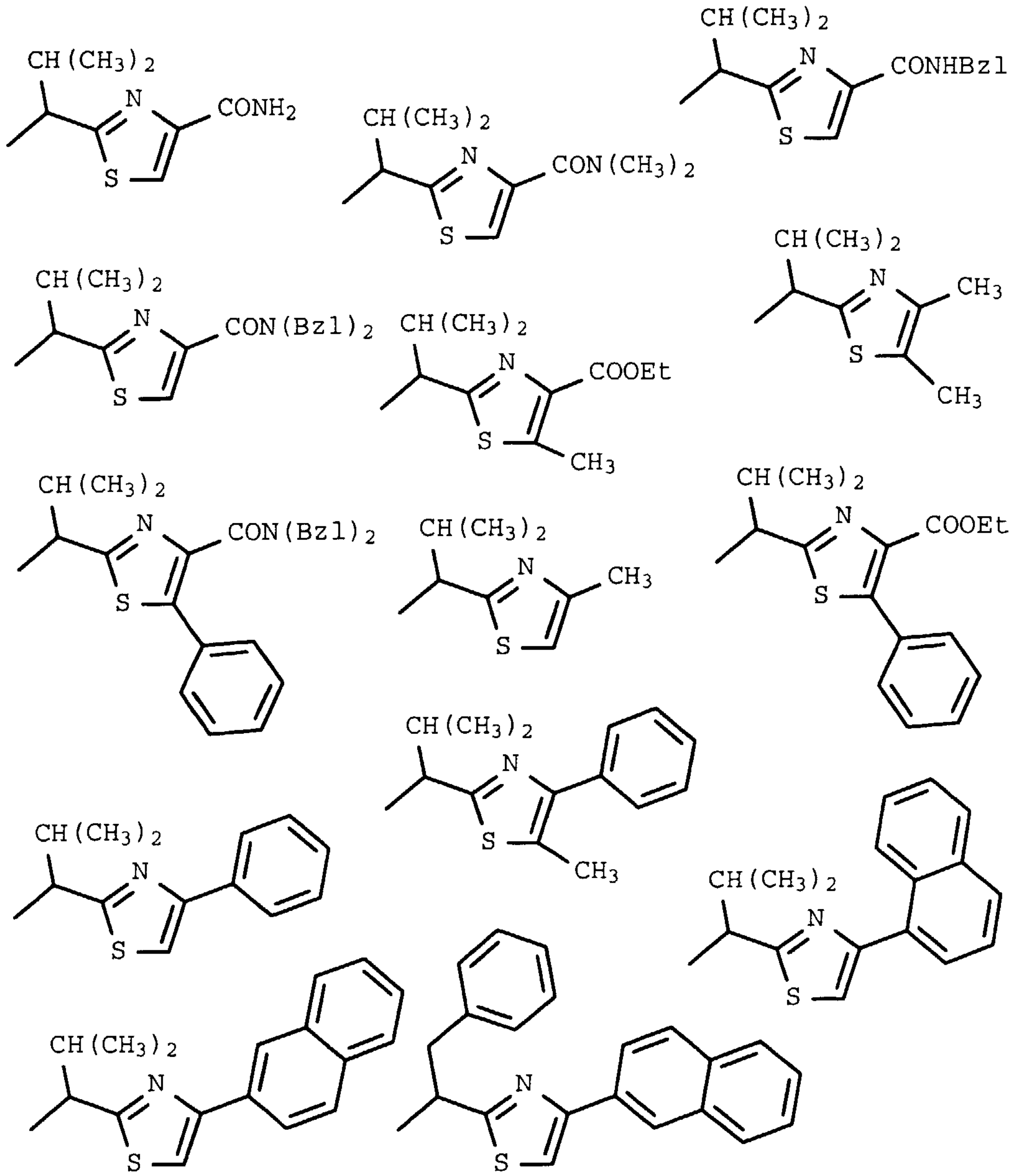

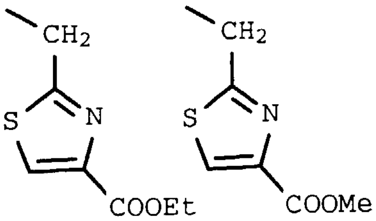
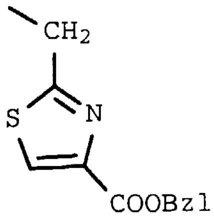
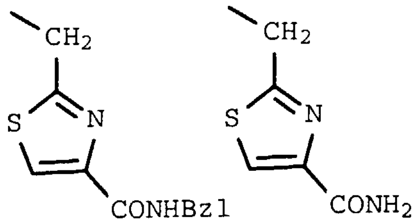
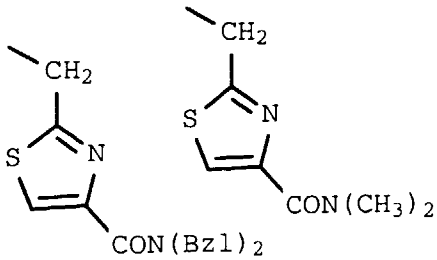
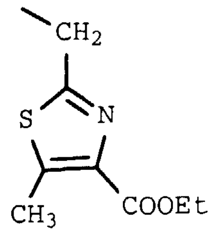
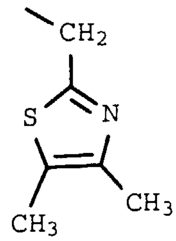
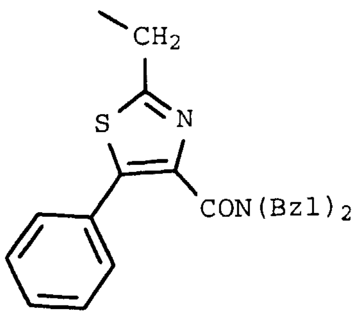
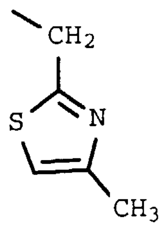
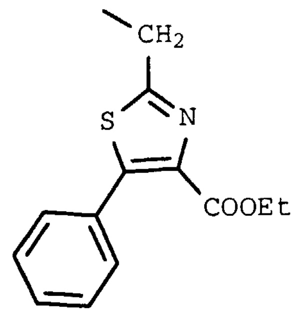
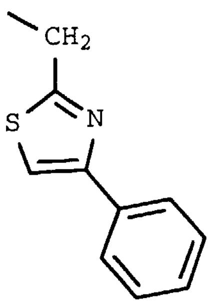
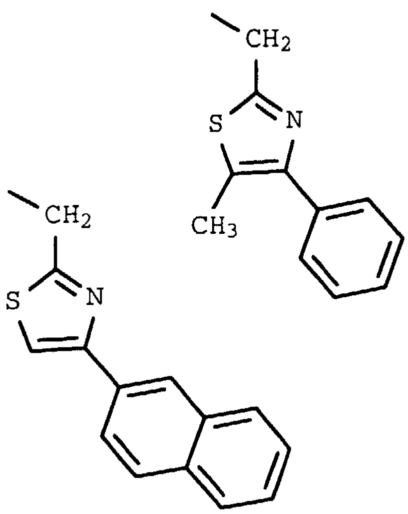
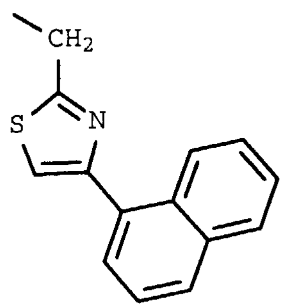

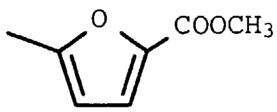
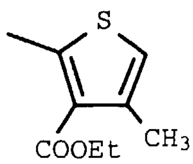
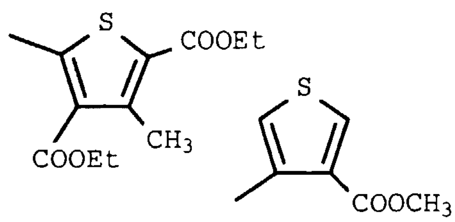
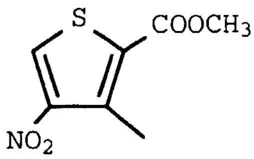
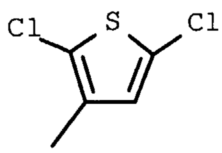
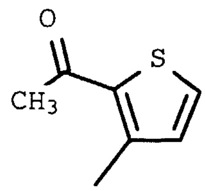
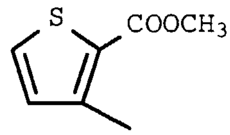
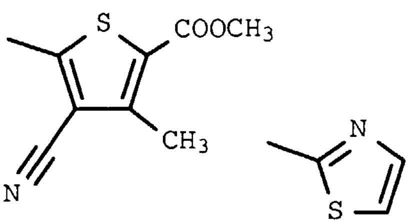
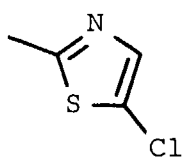
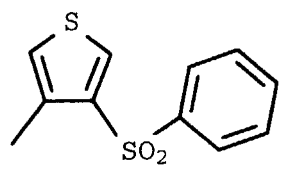
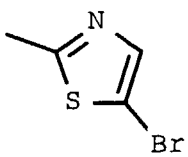
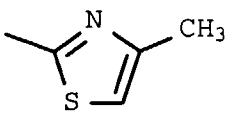
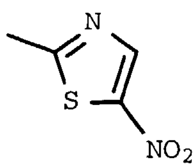
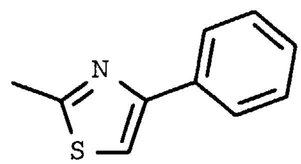
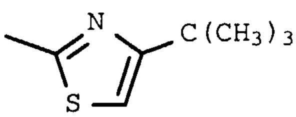
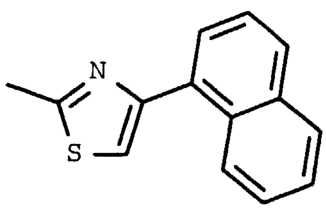
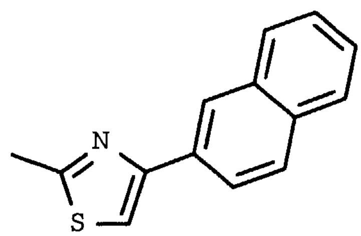
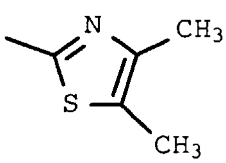
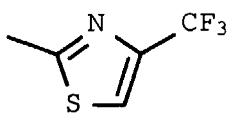
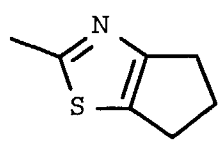
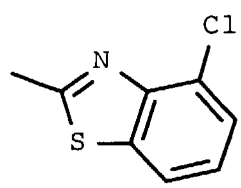
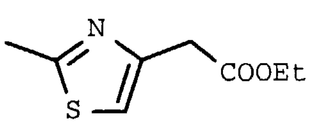
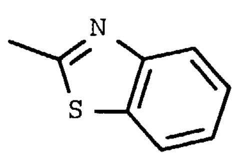
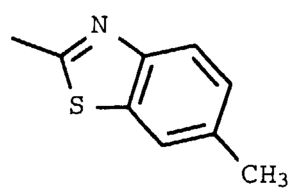

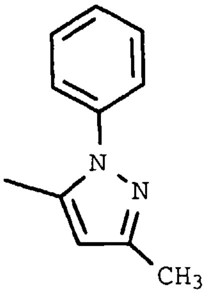
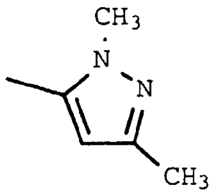
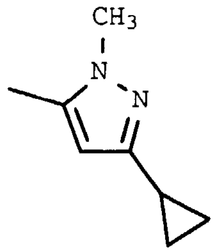
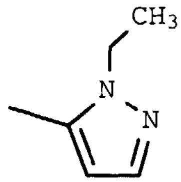
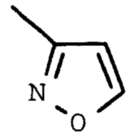
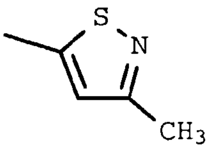
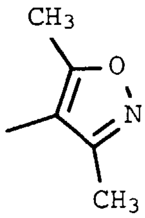
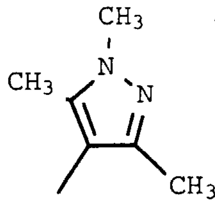
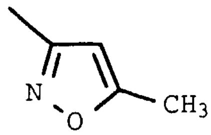
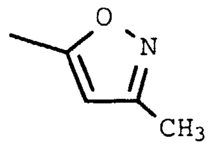
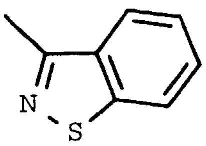
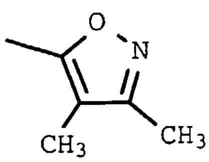
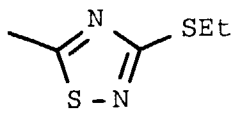
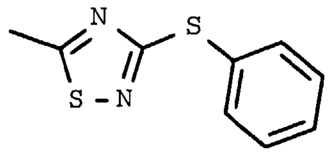
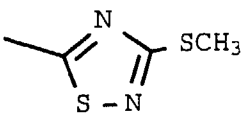
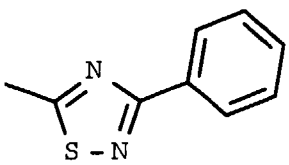
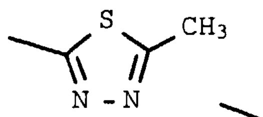
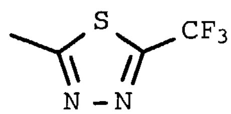
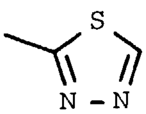
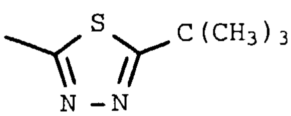
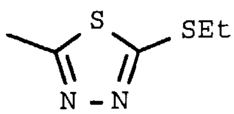
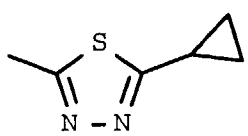
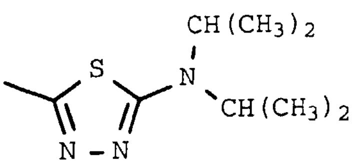
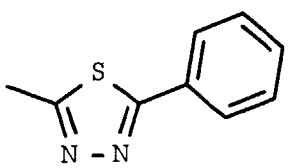
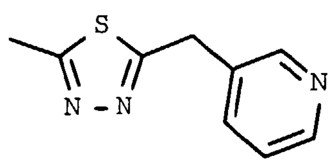

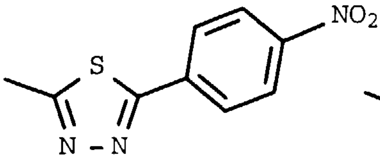
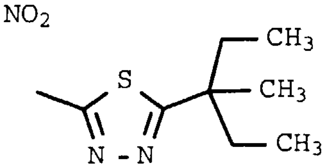
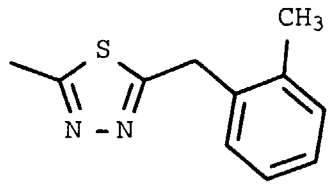

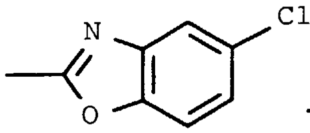
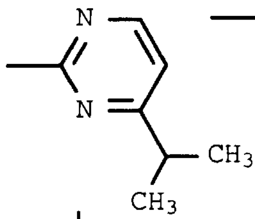
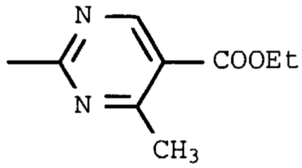
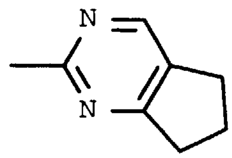

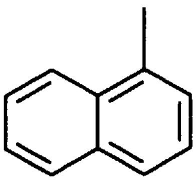
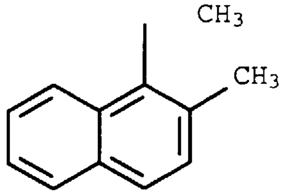
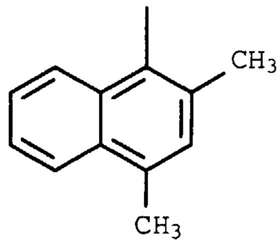
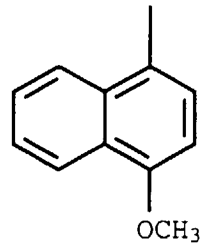

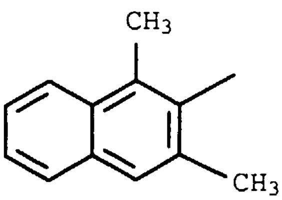
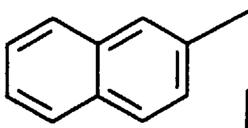
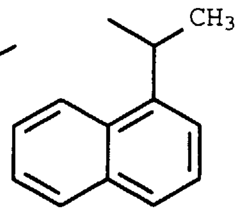
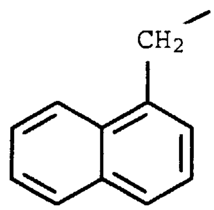

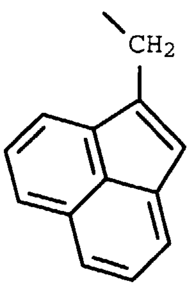
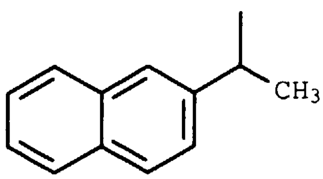
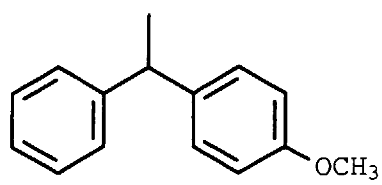

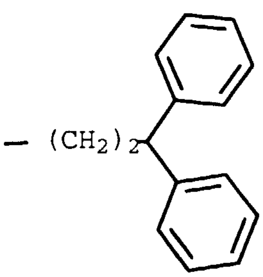
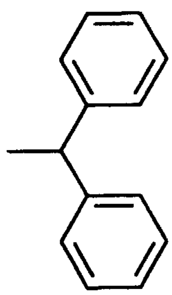
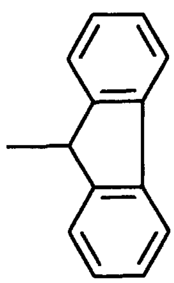
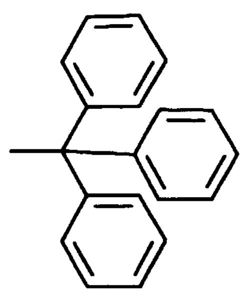

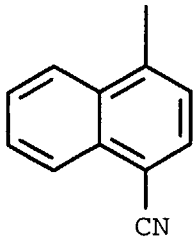
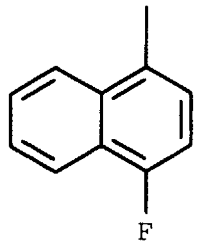
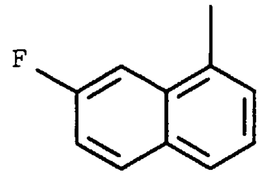
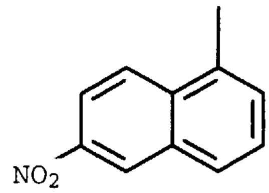

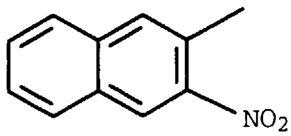
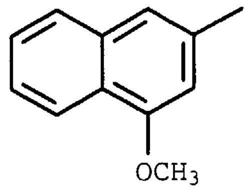
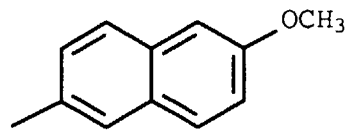

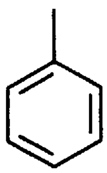
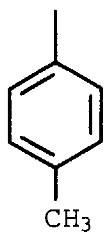
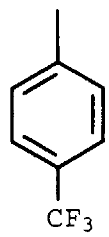
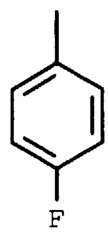
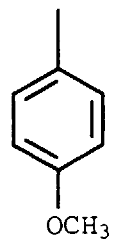
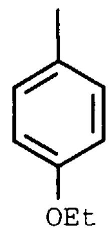
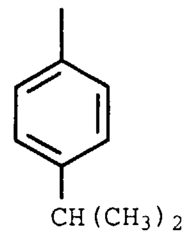

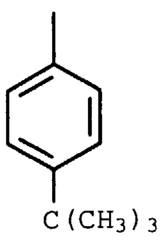
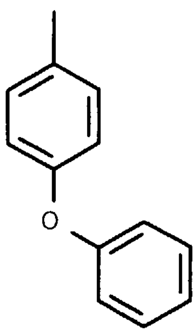
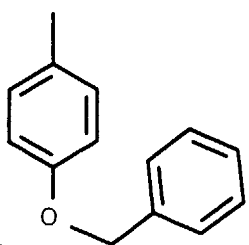
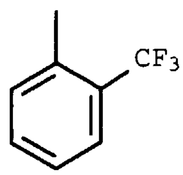
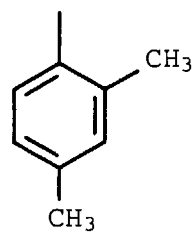

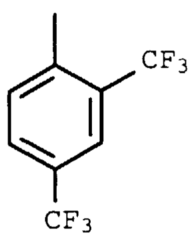
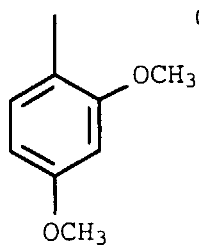
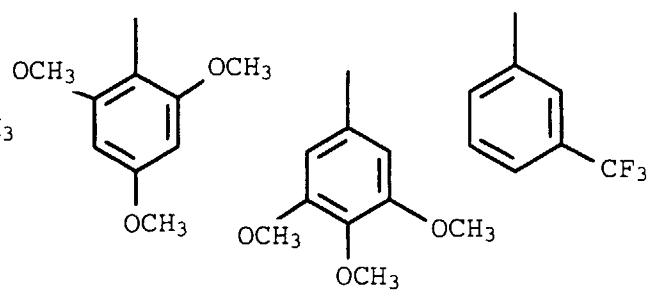

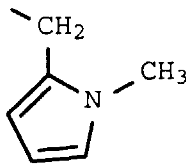 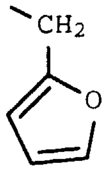 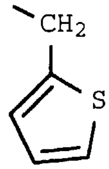 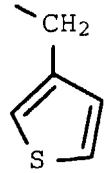 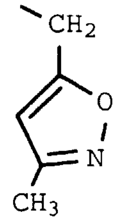

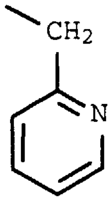 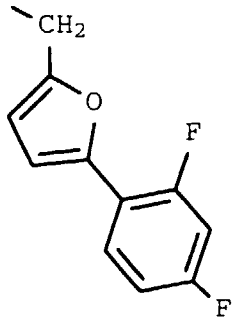 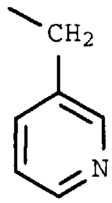 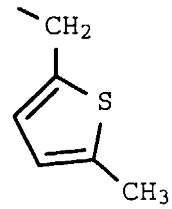 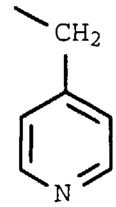

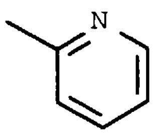 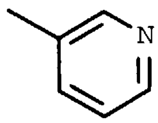 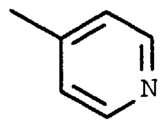 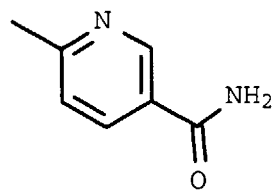

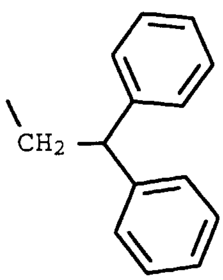 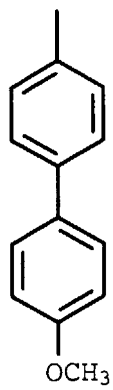 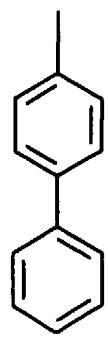 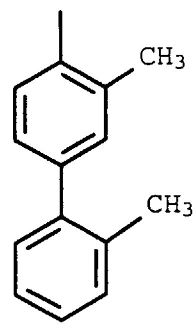 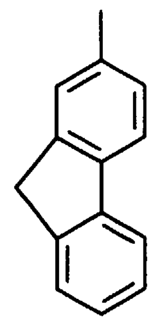

$R^5$-N-CHR$^7$-5-membered

heteroaryl are

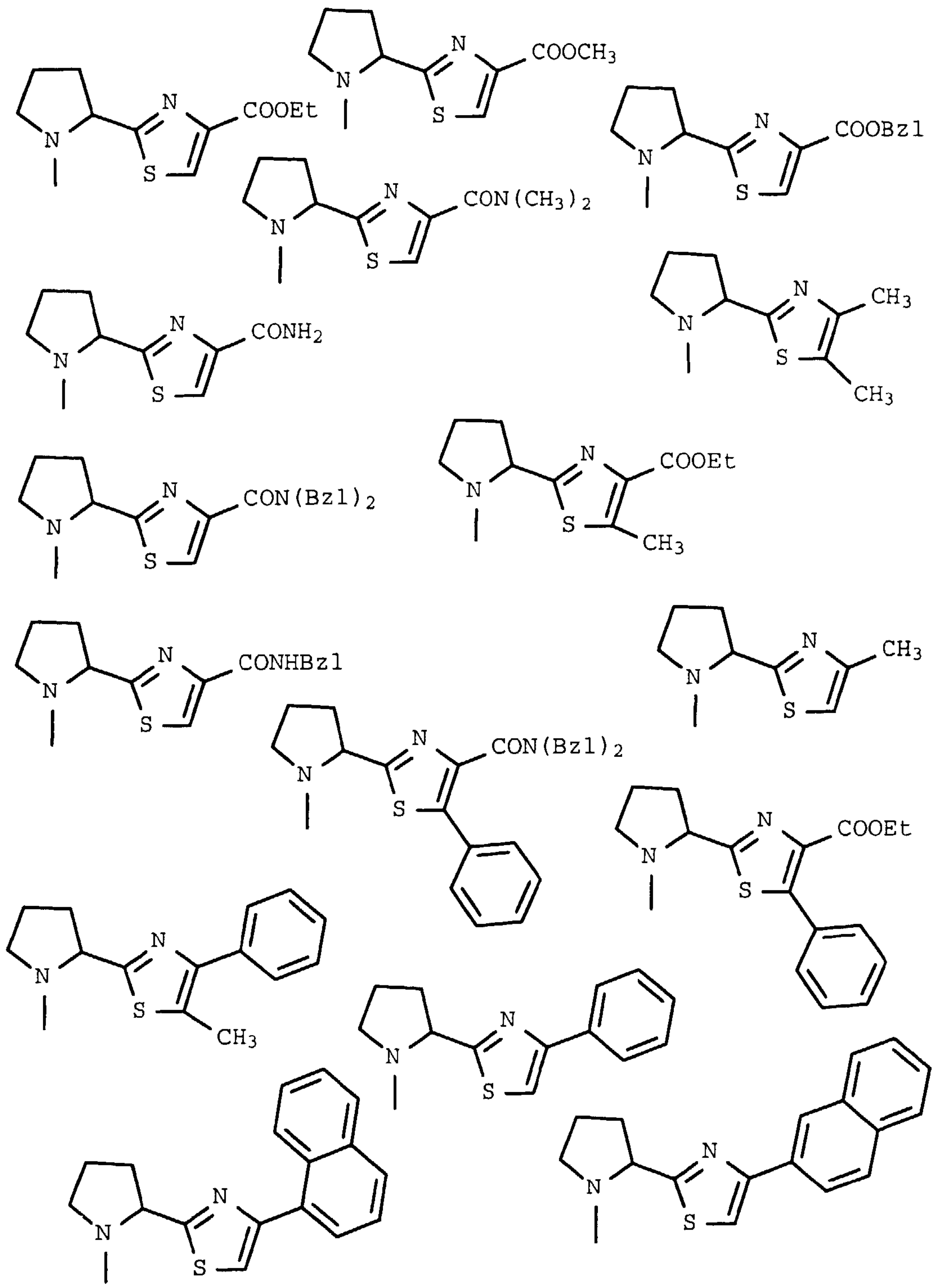

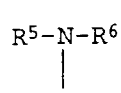

together are

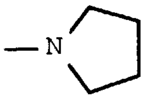
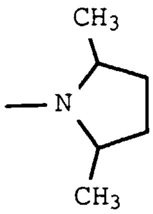
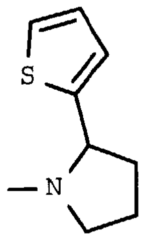
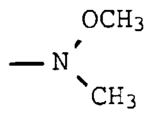
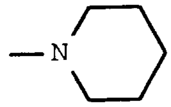
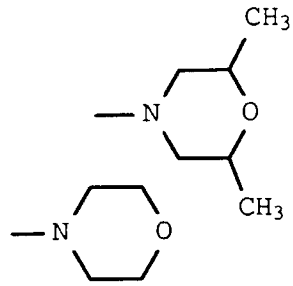

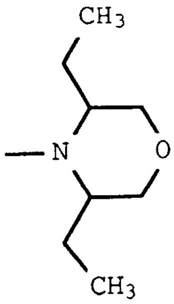
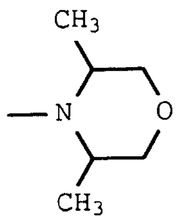
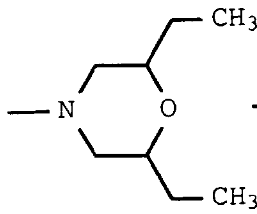
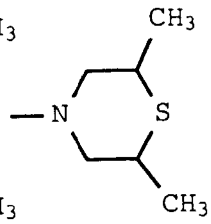
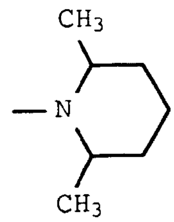

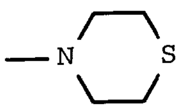
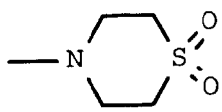
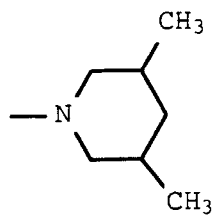
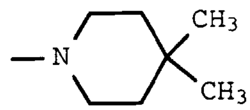

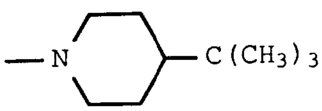
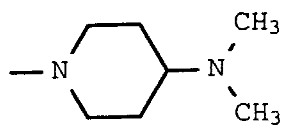
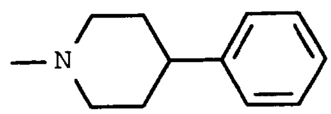

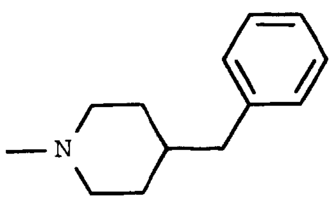
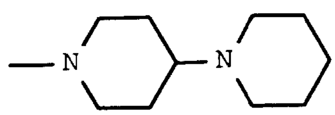
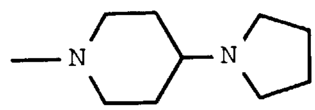

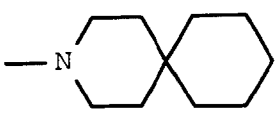
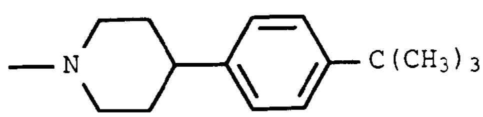

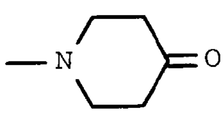
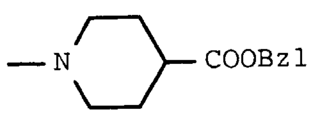
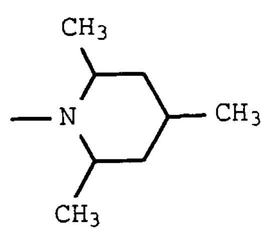

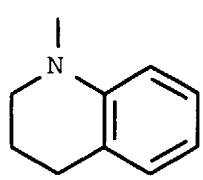
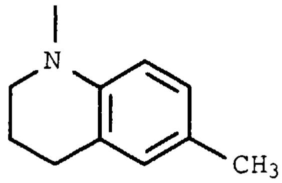
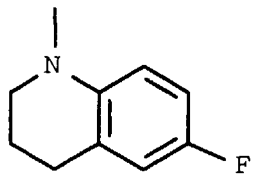
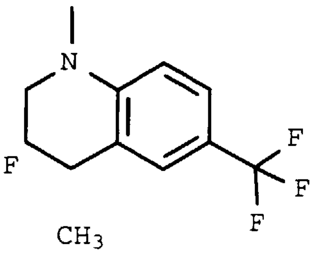
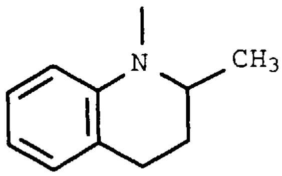
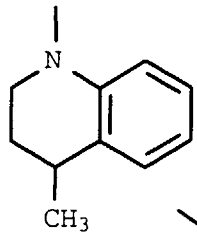
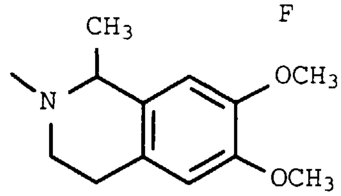
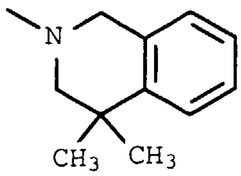
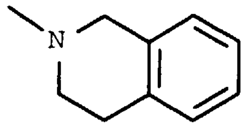
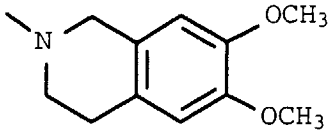
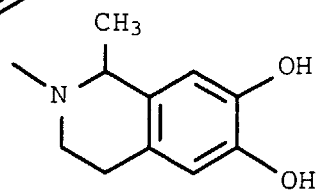
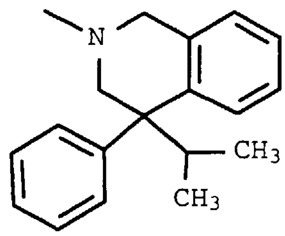
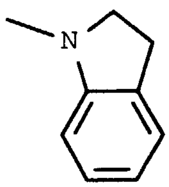
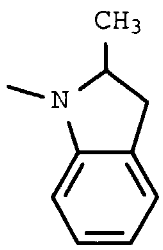
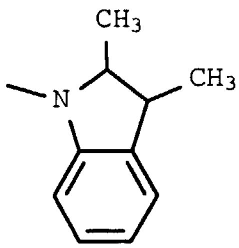
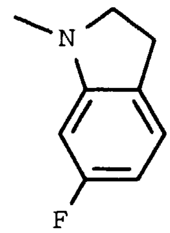
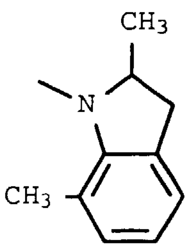
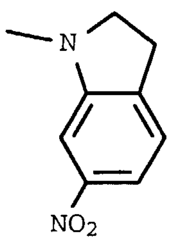
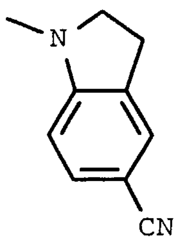
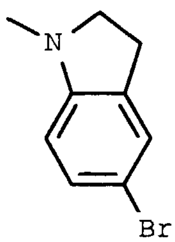
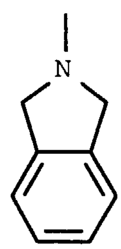
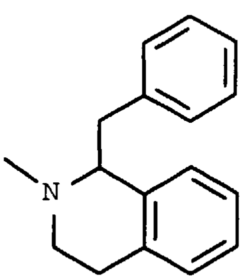

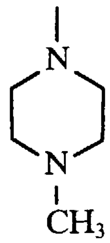
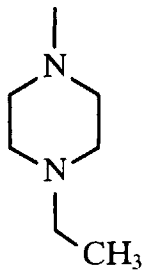
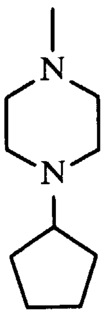
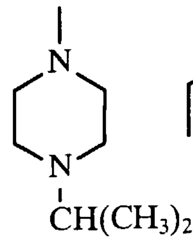
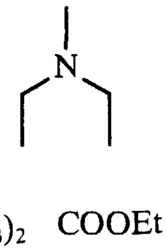
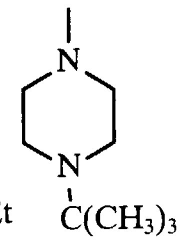
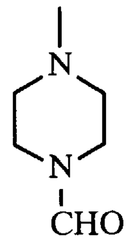

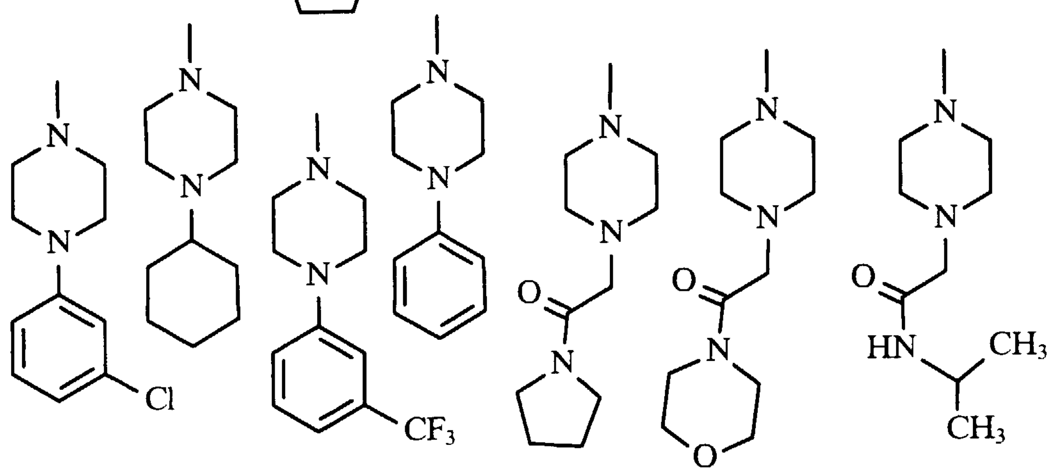

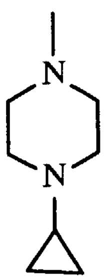
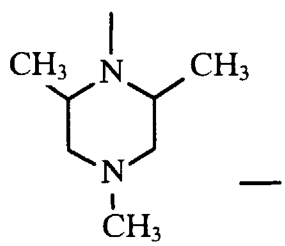
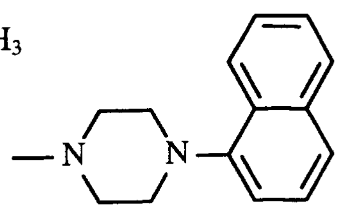

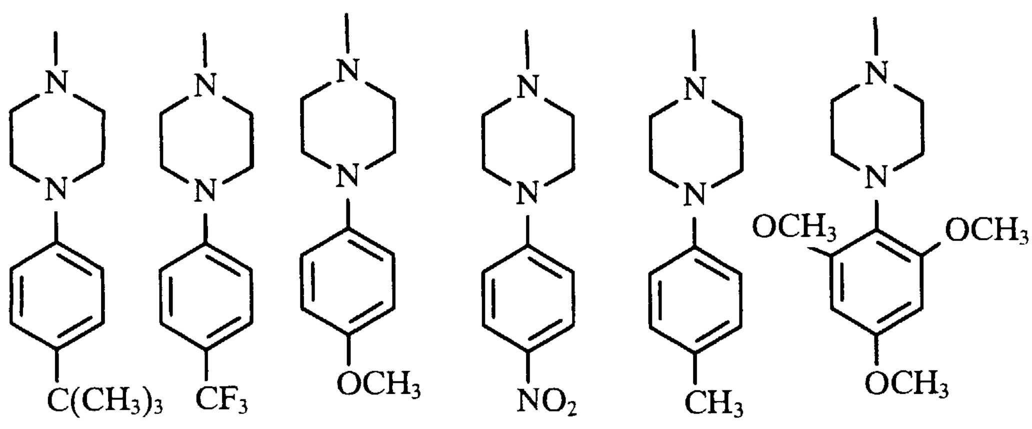

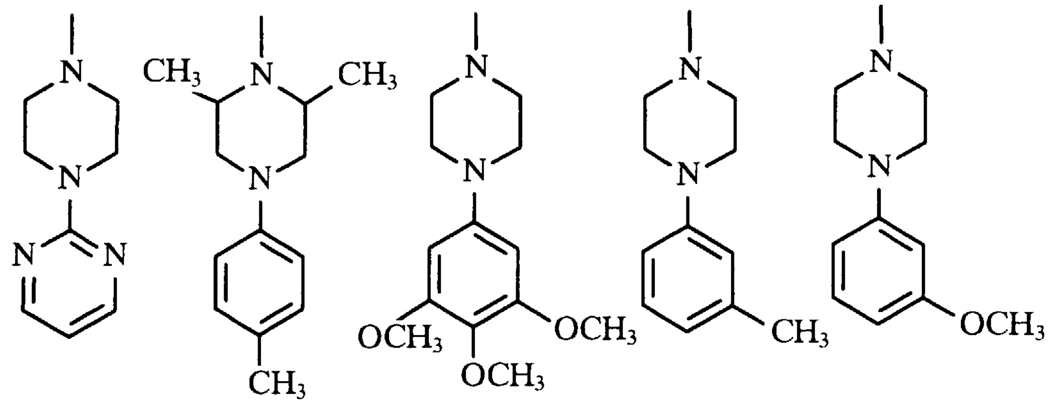

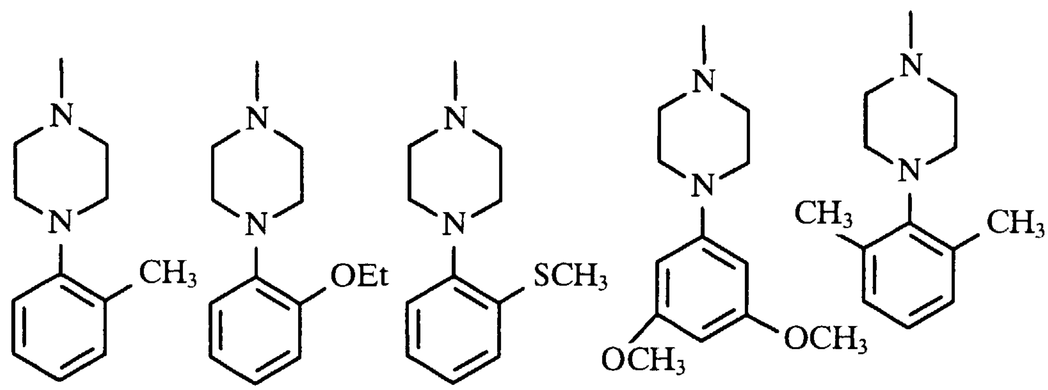

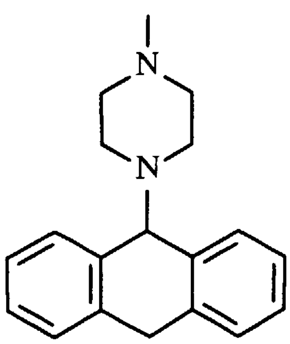

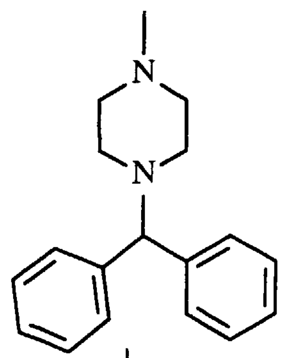

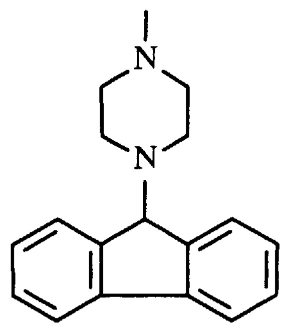

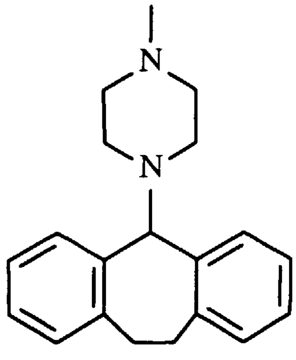

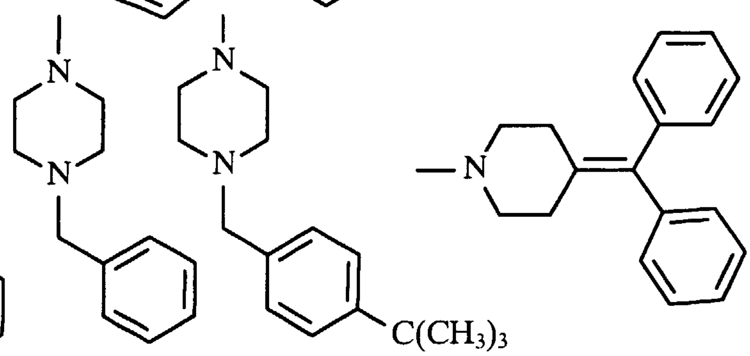

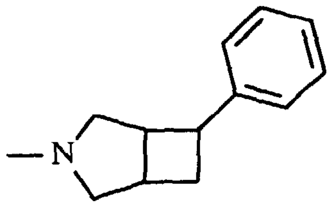 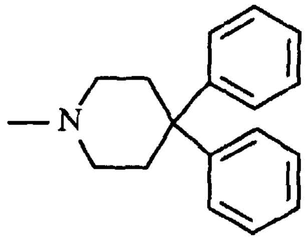 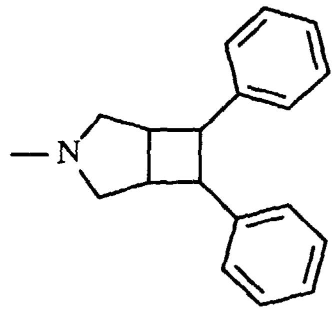

K is a hydroxy, alkoxy (preferably $C_{1-4}$), phenoxy or benzoxy moiety.

More preferred are compounds where the substituents have the following meanings:

$R^1$ is ethyl, methyl, 2-fluoroethyl, 2,2-difluoroethyl, 2,2,2-trifluoroethyl, 2-fluoroisopropyl, trifluoroisopropyl, isopropyl, propyl, cyclopropyl, or $N(CH_3)_2$

$R^2$ is hydrogen, methyl, ethyl, 2-fluoroethyl, 2,2-difluoroethyl, 2,2,2-trifluoroethyl, isopropyl, propyl, butyl, or cyclopropyl

$R^1$-N-$R^2$ together are

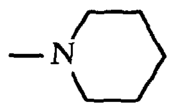

A, B, D, E, F, G and X have the meanings as described above;

u, v and w are independently 0 or 1;

E and F together are

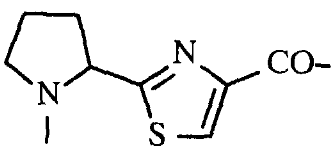 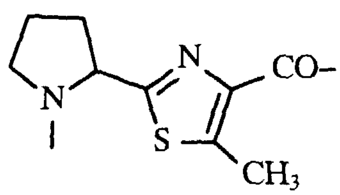 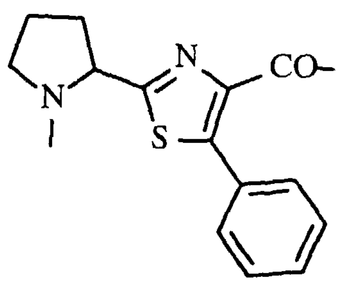

K is a hydroxy, $C_{1-4}$-alkoxy or benzyloxy moiety;

$R^5$ is hydrogen, methyl, ethyl, 2-fluoroethyl, 2,2-difluoroethyl, propyl, isoproypyl, cyclopropyl, cyclopentyl, cyclohexyl, benzyl, 4-phenoxybenzyl, 4-benzyloxybenzyl or 3,4,5-trimethoxybenzyl

$R^6$ is hydrogen, methyl, ethyl, 2-fluoroethyl, 2,2-trifluoroethyl, propyl, isopropyl, tert-butyl, cyclopropyl, cyclopentyl, cyclohexyl, benzyl, 4-phenoxybenzyl, 4-benzyloxybenzyl, 3,4,5-trimethoxybenzyl, phenyl, 4-phenoxyphenyl, 4-benzyloxyphenyl, 3,4,5-trimethoxyphenyl or

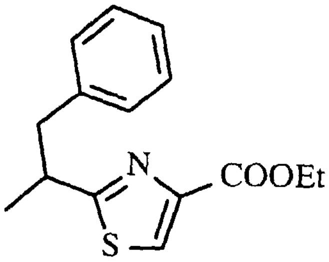

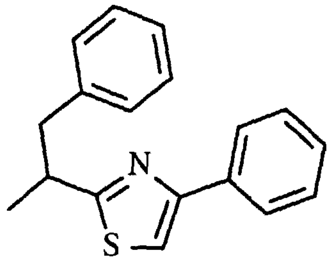

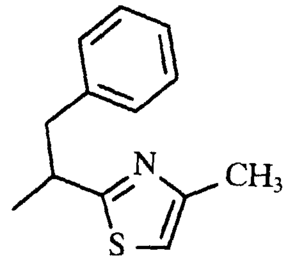

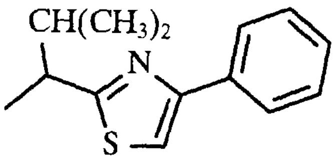

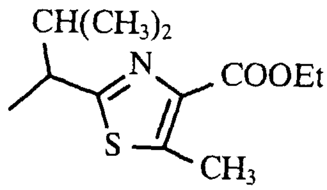

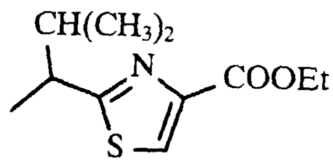

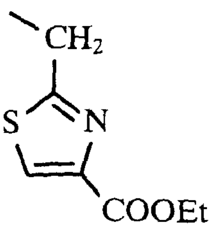

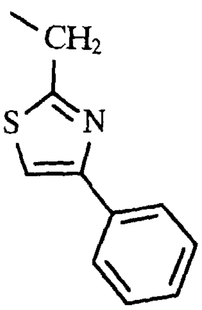

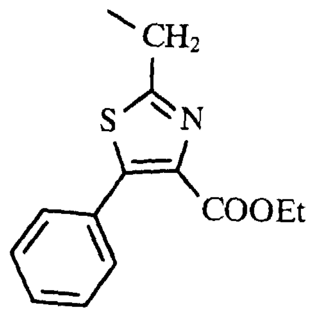

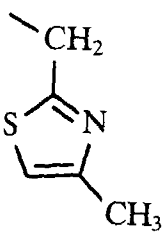

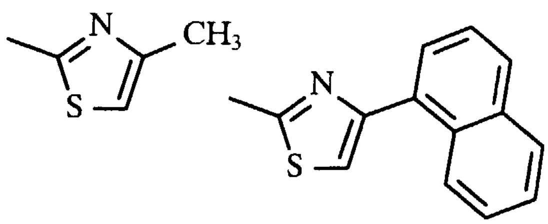
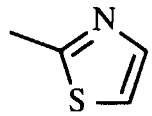
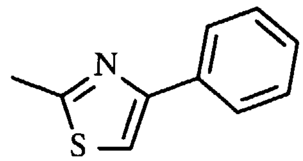
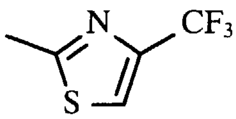
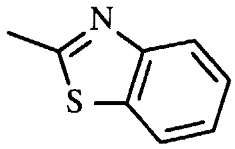
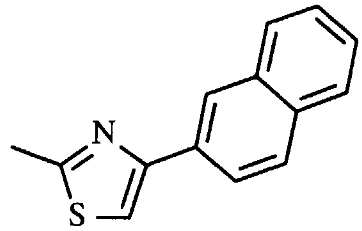
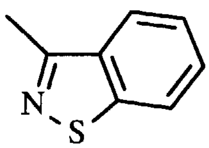
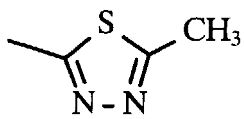
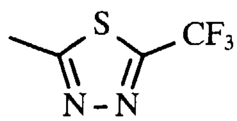
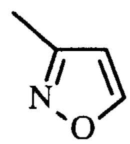
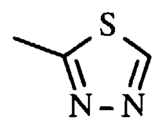
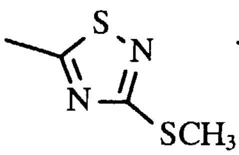
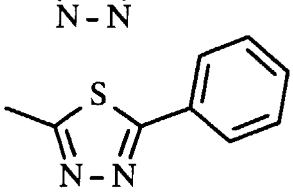
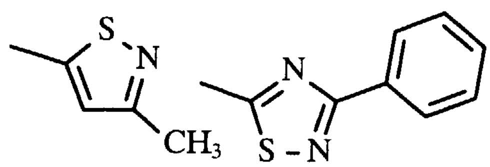
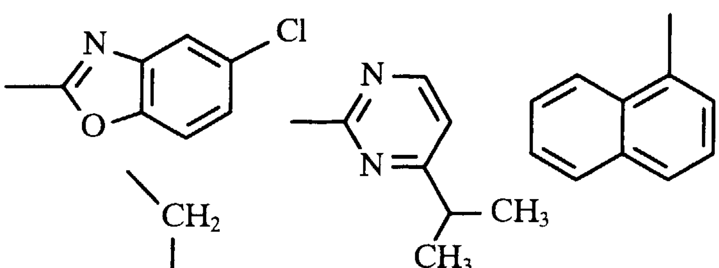
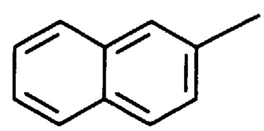
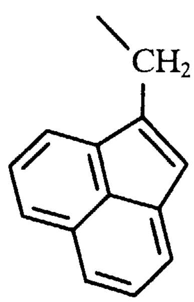
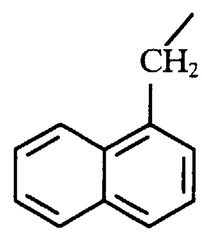
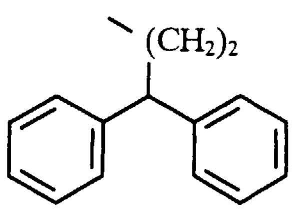
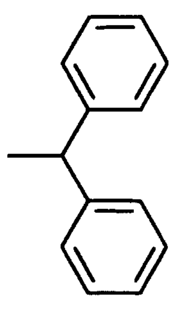
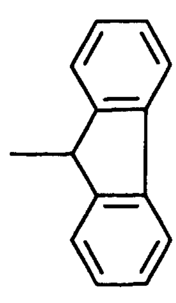
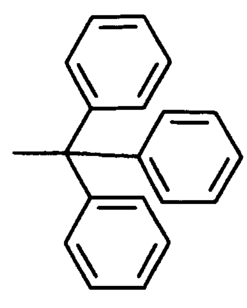

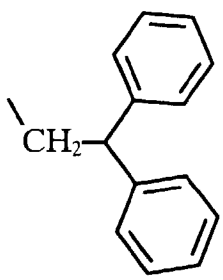
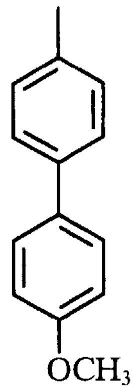
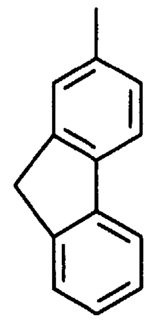
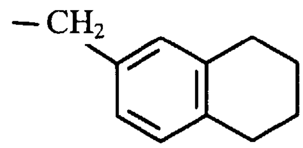

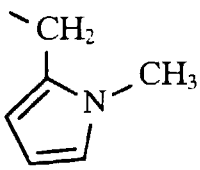
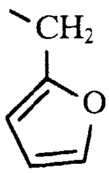
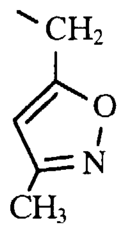
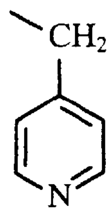
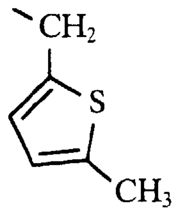
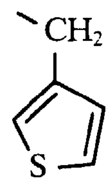
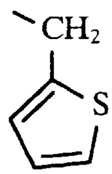
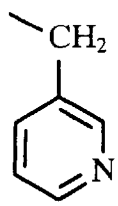
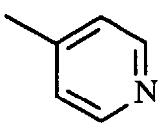
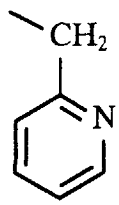
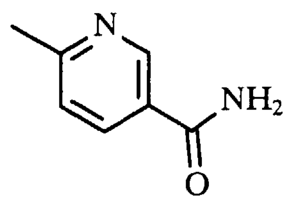

$R^5$-N-$CHR^7$-5-membered heteroaryl is

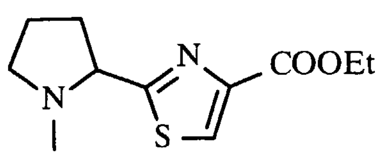
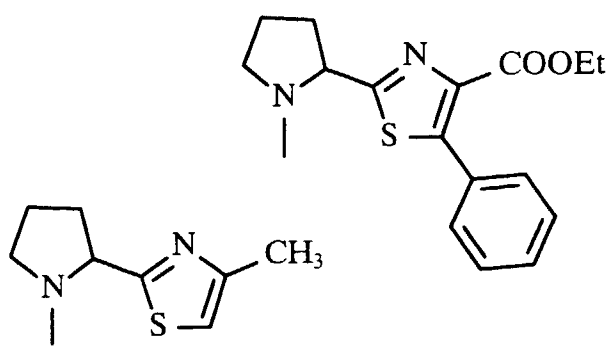
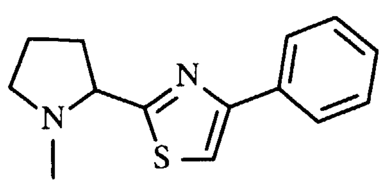

$R^5$-N-$R^6$ together are

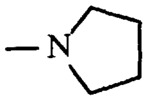
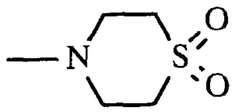
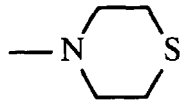
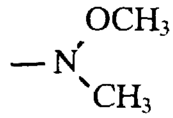

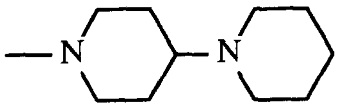
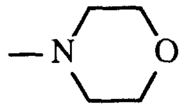
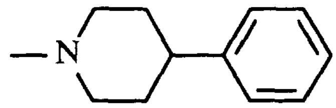

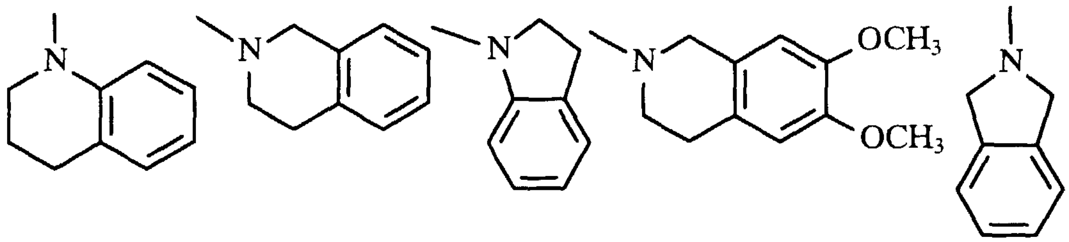

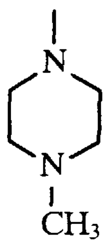
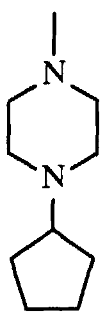
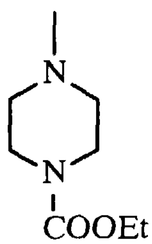
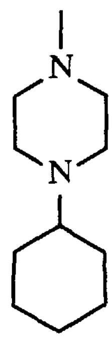
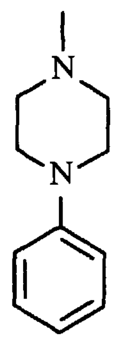

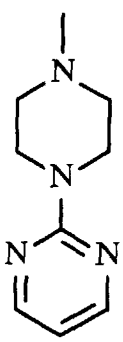
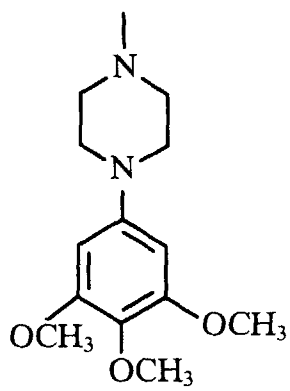

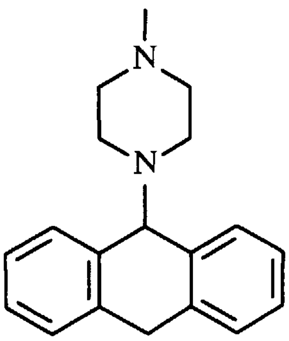
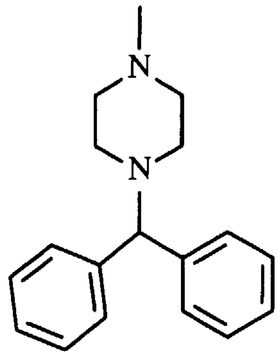
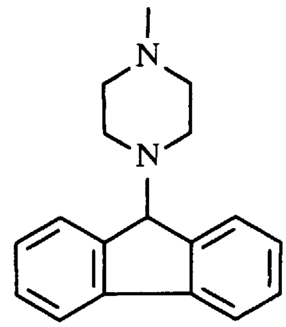

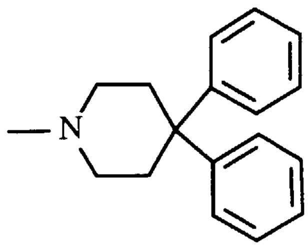
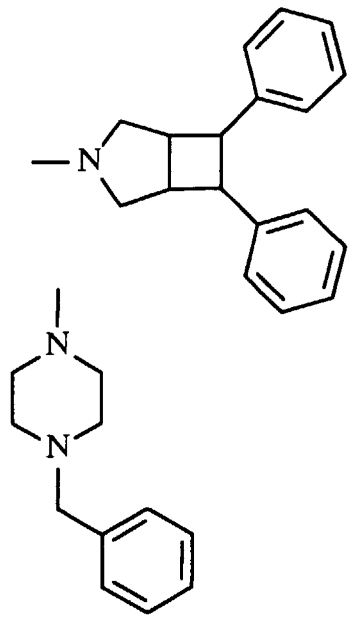
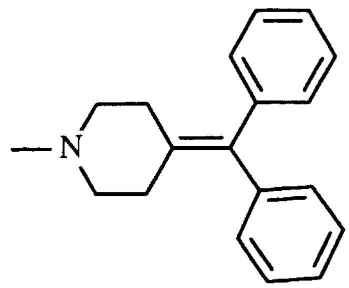

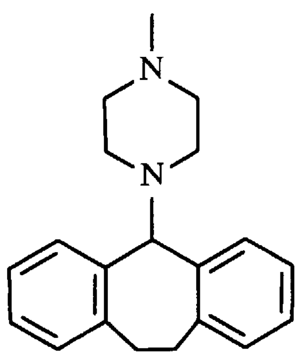

These examples illustrate but do not limit the scope of the present invention.

The peptides of the formula I are composed preferably of L-amino acids but they may contain one or more D-amino acids.

The new compounds may be present as salts with physiologically tolerated acids such as: hydrochloric acid, citric acid, tartaric acid, lactic acid, phosphoric acid, methanesulfonic acid, acetic acid, formic acid, maleic acid, fumaric acid, malic acid, succinic acid, malonic acid, sulfuric acid, L-glutamic acid, L-aspartic acid, pyruvic acid, mucic acid, benzoic acid, glucuronic acid, oxalic acid, ascorbic acid and acetylglycine.

The novel compounds can be prepared by known methods of peptide chemistry. Thus, the peptides can be assembled sequentially from amino acids or by linking suitable small peptide fragments. In the sequential assemblage, starting at the C terminus the peptide chain is extended stepwise by one amino acid each time. In fragment coupling it is possible to link together fragments of different lengths, and the fragments in turn can be obtained by sequential assemblage from amino acids or themselves by fragment coupling.

Both in the sequential assemblage and in the fragment coupling it is necessary to link the units by forming an amide linkage. Enzymatic and chemical methods are suitable for this.

Chemical methods for forming the amide linkage are described in detail by Muller, Methoden der organischen Chemie Vol. XV/2, pp 1 to 364, Thieme Verlag, Stuttgart, 1974; Stewart, Young, Solid Phase Peptide Synthesis, pp 31 to 34, 71 to 82, Pierce Chemical Company, Rockford, 1984; Bodanszky, Klausner, Ondetti, Peptide Synthesis, pp 85 to 128, John Wiley & Sons, New York, 1976 and other standard works on peptide chemistry. Particular preference is given to the azide method, the symmetric and mixed anhydride method, in situ generated or preformed active esters, the use of urethane protected N-carboxy anhydrides of amino acids and the formation of the amide linkage using coupling reagents (activators, especially dicyclohexylcarbodiimide (DCC), diisopropylcarbodiimide (DIC), 1-ethoxycarbonyl-2-ethoxy-1,2-dihydroquinoline (EEDQ), 1-ethyl-3-(3-dimethylaminopropyl)carbodiimide hydrochloride (EDCI), n-propanephosphonic anhydride (PPA), N,N-bis(2-oxo-3-oxazolidinyl)- midophosphoryl chloride (BOP-Cl), bromo-tris-pyrrolidinophosphonium hexa-fluorophosphate (PyBrop), diphenylphosphoryl azide (DPPA), Castro's reagent (BOP,

PyBop), O-benzotriazolyl-N,N,N',N'-tetramethyluronium salts (HBTU), diethylphosphoryl cyanide (DEPCN), 2,5-diphenyl-2,3-dihydro-3-oxo-4-hydroxythiophene dioxide (Steglich's reagent; HOTDO) and 1,1'-carbonyldiimidazole (CDI). The coupling reagents can be employed alone or in combination with additives such as N,N-dimethyl-4-aminopyridine (DMAP), N-hydroxy-benzotriazole (HOBt), N-hydroxybenzotriazine (HOOBt), N-hydroxysuccinimide (HOSu) or 2-hydroxypyridine.

Whereas it is normally possible to dispense with protective groups in enzymatic peptide synthesis, reversible protection of reactive groups not involved in formation of the amide linkage is necessary for both reactants in chemical synthesis. Three conventional protective group techniques are preferred for the chemical peptide synthesis: the benzyloxycarbonyl (Z), the t-butoxycarbonyl (Boc) and the 9-fluorenylmethoxycarbonyl (Fmoc) techniques. Identified in each case is the protective group on the $\alpha$-amino group of the chain-extending unit. A detailed review of amino-acid protective groups is given by Müller, Methoden der organischen Chemie Vol. XV/1, pp 20 to 906, Thieme Verlag, Stuttgart, 1974. The units employed for assembling the peptide chain can be reacted in solution, in suspension or by a method similar to that described by Merrifield in J. Amer. Chem. Soc. 85 (1963) 2149. Particularly preferred methods are those in which peptides are assembled sequentially or by fragment coupling using the Z, Boc or Fmoc protective group technique, with one of the reactants in the said Merrifield technique being bonded to an insoluble polymeric support (also called resin hereinafter). This typically entails the peptide being assembled sequentially on the polymeric support using the Boc or Fmoc protective group technique, the growing peptide chain being covalently bonded at the C terminus to the insoluble resin particles (cf. Fig. 1 and 2). This procedure makes it possible to remove reagents and byproducts by filtration, and thus recrystallization of intermediates is unnecessary.

The protected amino acids can be linked to any suitable polymers, which merely have to be insoluble in the solvents used and to have a stable physical form which makes filtration easy. The polymer must contain a functional group to which the first protected amino acid can be firmly attached by a covalent bond. Suitable for this purpose are a wide variety of polymers, eg. cellulose, polyvinyl alcohol, polymethacrylate, sulfonated polystyrene, chloromethylated styrene/divinylbenzene copolymer (Merrifield resin), 4-methylbenzhydrylamine resin (MBHA-resin), phenylacetamidomethyl-resin (Pam-resin), p-benzyloxy-benzyl-alcohol-resin, benzhydryl-amine-resin (BHA-resin), 4-(hydroxymethyl)benzoyloxy-methyl-resin, the resin of Breipohl et al. (Tetrahedron Letters 28 (1987) 565; supplied by BACHEM), 4-(2,4-dimethoxyphenylaminomethyl)phenoxy-resin (supplied by Novabiochem) or o-chlorotrityl-resin (supplied by Biohellas).

Suitable for peptide synthesis in solution are all solvents which are inert under the reaction conditions, especially water, N,N-dimethylformamide (DMF), dimethyl sulfoxide (DMSO), acetonitrile, dichloromethane (DCM), 1,4-dioxane, tetrahydrofuran (THF), N-methyl-2-pyrrolidone (NMP) and mixtures of the said solvents. Peptide synthesis on the polymeric support can be carried out in all inert organic solvents in which the amino-acid derivatives used are soluble; however, preferred solvents additionally have resin-swelling properties, such as DMF, DCM, NMP, acetonitrile and DMSO, and mixtures of these solvents. After synthesis is complete, the peptide is cleaved off the polymeric support. The conditions under which cleavage off the various resin types is possible are disclosed in the literature. The cleavage reactions most commonly used are acid- and palladium-catalyzed, especially cleavage in liquid anhydrous hydrogen fluoride, in anhydrous trifluoromethanesulfonic acid, in dilute or concentrated trifluoroacetic acid, palladium-catalyzed cleavage in THF or THF-DCM mixtures in the presence of a weak base such as morpholine or cleavage in acetic acid/dichloromethane/trifluoroethanol mixtures. Depending on the chosen protective groups, these may be retained or likewise cleaved off under the cleavage conditions. Partial deprotection of the peptide may also be worthwhile when certain derivatization reactions are to be carried out. Peptides dialkylated at the N-terminus can be prepared either by coupling on the appropriate N,N-di-alkylamino acids in solution or on the polymeric support or by reductive alkylation of the resin-bound peptide in DMF/1% acetic acid with $NaCNBH_3$ and the appropriate aldehydes. The various non-naturally occurring amino acids as well as the various non-amino acid moieties disclosed herein may be obtained from commercial sources or synthesized from commercially-available materials using methods known in the art. For example, amino acids building blocks with $R^1$ and $R^2$ moieties can be prepared according to E. Wünsch, Houben Weyl, Meth. d. Org. Chemie, Bd. XV, 1, p. 306 following, Thieme Verlag Stuttgart 1974 and Literature cited therein. Peptides with $\gamma$- or $\delta$-lactam bridges can be prepared by incorporating the appropriate lactam-bridged dipeptide units (R. Freidinger, J. Org. Chem. (1982) 104-109) into the peptide chain. Peptides with thiazole-, oxazol-, thiazolin- or oxazolin-containing dipeptide building blocks can be prepared by incorporating the appropriate dipeptidic units (P. Jouin et al., Tetrahedron Letters (1992), 2807-2810; P. Wipf et al., Tetrahedron Letters (1992), 907-910; W.R. Tully, J. Med. Chem. (1991), 2065; Synthesis (1987), 235) into the peptide chain.

The compounds of this invention may be used to inhibit or otherwise treat solid tumors (e.g. tumors of the lung, breast, colon, prostate, bladder, rectum, or endometrial tumors) or hematological malignancies (e.g. leucemias, lymphomas) by administration of the compound to the mammal. Administration may be by any of the means which are conventional for pharmaceutical, preferably onco-logical, agents, including oral and parenteral means such as subcutaneously, intravenously, intramuscularly and intraperitoneally. The compounds may be administered alone or in the form of pharmaceutical compositions containing a compound of formula I together with a pharmaceutically accepted

carrier appropriate for the desired route of administration. Such pharmaceutical compositions may be combination products, i.e., may also contain other therapeutically active ingredients.

The dosage to be administered to the mammal will contain an effective tumor-inhibiting amount of active ingredient which will depend upon conventional factors including the biological activity of the particular compound employed; the means of administration; the age, health and body weight of the recipient; the nature and extent of the symptoms; the frequency of treatment; the administration of other therapies; and the effect desired. A typical daily dose will be about 5 to 250 milligrams per kilogram of body weight on oral administration and about 1 to 100 milligrams per kilogram of body weight on parenteral administration.

The novel compounds can be administered in conventional solid or liquid pharmaceutical administration forms, eg. uncoated or (film-)coated tablets, capsules, powders, granules, suppositories or solutions. These are produced in a conventional manner. The active substances can for this purpose be processed with conventional pharmaceutical aids such as tablet binders, fillers, preservatives, tablet disintegrants, flow regulators, plasticizers, wetting agents, dispersants, emulsifiers, solvents, sustained release compositions, antioxidants and/or propellant gases (cf. H. Sucker et al.: Pharmazeutische Technologie, Thieme-Verlag, Stuttgart, 1978). The administration forms obtained in this way normally contain 1-90% by weight of the active substance.

The following examples are intended to illustrate the invention. The proteinogenous amino acids are abbreviated in the examples using the known three-letter code. Other meanings are: TFA = trifluoroacetic acid, Ac = acetic acid, Bu = butyl, Et = ethyl, Me = methyl, Bzl = benzyl.

A. General procedures

I. The peptides claimed in claim 1 are either synthesized by classical solution synthesis using standard Z- and Boc-methodology as described above or by standard methods of solid-phase synthesis on a completely automatic model 431A synthesizer supplied by APPLIED BIOSYSTEMS. The apparatus uses different synthetic cycles for the Boc and Fmoc protective group techniques.

a) Synthetic cycle for the Boc protective group technique

| | | |
|---|---|---|
| 1. | 30% trifluoroacetic acid in DCM | 1 x 3 min |
| 2. | 50% trifluoroacetic acid in DCM | 1 x 1 min |
| 3. | DCM washing | 5 x 1 min |
| 4. | 5% diisopropylethylamine in DCM | 1 x 1 min |
| 5. | 5% diisopropylethylamine in NMP | 1 x 1 min |
| 6. | NMP washing | 5 x 1 min |
| 7. | Addition of preactivated protected amino acid (activation with 1 equivalent of DCC and 1 equivalent of HOBt in NMP/DCM); | |
| | Peptide coupling (1st part) | 1 x 30 min |
| 8. | Addition of DMSO to the reaction mixture until it contains 20% DMSO by volume | |
| 9. | Peptide coupling (2nd part) | 1 x 16 min |
| 10. | Addition of 3.8 equivalents of diisopropylethylamine to the reaction mixture | |
| 11. | Peptide coupling (3rd part) | 1 x 7 min |
| 12. | DCM washing | 3 x 1 min |
| 13. | if conversion is incomplete, repetition of coupling (back to 5.) | |
| 14. | 10% acetic anhydride, | |
| | 5% diisopropylethylamine in DCM | 1 x 2 min |
| 15. | 10% acetic anhydride in DCM | 1 x 4 min |
| 16. | DCM washing | 4 x 1 min |
| 17. | back to 1. | |

BOP-Cl and PyBrop were used as reagents for coupling of the amino acid following N-methylamino acids. The reaction times were correspondingly increased. In solution synthesis, the use of either Boc-protected amino acid NCAs (N-tert.-butyloxycarbonylamino acid-N-carboxy-anhydrides) or Z-protected amino acid NCAs (N-benzyloxycarbonyl-amino acid-N-carboxy-anhydrides) respectively is most advantageous for this type of coupling.

b) Synthetic cycle for the Fmoc protective group technique

| | | |
|---|---|---|
| 1. | DMF washing | 1 x 1 min |
| 2. | 20% piperidine in DMF | 1 x 4 min |
| 3. | 20% piperidine in DMF | 1 x 16 min |
| 4. | DMF washing | 5 x 1 min |
| 5. | Addition of the preactivated protected amino acid (activation by 1 equivalent of TBTU and 1.5 equivalent of DIPEA in DMF); Peptide coupling | 1 x 61 min |
| 6. | DMF washing | 3 x 1 min |
| 7. | if conversion is incomplete, repetition of coupling (back to 5.) | |
| 8. | 10% acetic anhydride in DMF | 1 x 8 min |
| 9. | DMF washing | 3 x 1 min |
| 10. | back to 2. | |

BOP-Cl and PyBrop were used as reagents for coupling on the amino acid following the N-methylamino acids. The reaction times were correspondingly increased.

II. Reductive alkylation of the N terminus

The peptide-resin prepared as in AIa or AIb was deprotected at the N terminus (steps 2-4 in AIb or 1-6 in AIa) and then reacted with a 3-fold molar excess of aldehyde or ketone in DMF/1% acetic acid with addition of 3 equivalents of NaCNBH3. After reaction was complete (negative Kaiser test) the resin was washed several times with water, isopropanol, DMF and dichloromethane.

III. Workup of the peptide-resins obtained as in Ia and II

The peptide-resin was dried under reduced pressure and transferred into a reaction vessel of a TEFLON HF apparatus (supplied by PENINSULA). Addition of a scavenger, preferably anisole (1 ml/g of resin), and in the case of tryptophan-containing peptides of a thiol to remove the indolic formyl group, preferably ethanedithiol (0.5 ml/g of resin), was followed by condensing in hydrogen fluoride (10 ml/g of resin) while cooling with liquid $N_2$. The mixture was left to warm to 0°C and stirred at this temperature for 45 min. The hydrogen fluoride was then stripped off under reduced pressure, and the residue was washed with ethyl acetate in order to remove remaining scavenger. The peptide was extracted with 30% strength acetic acid and filtered, and the filtrate was lyophilized.

IV. Work-up of the peptide-resins obtained as in Ib and II

The peptide-resin was dried under reduced pressure and then subjected to one of the following cleavage procedures, depending on the amino-acid composition (Wade, Tregear, Howard Florey Fmoc Workshop Manual, Melbourne 1985).

| Cleavage conditions | | | |
|---|---|---|---|
| | TFA | Scavenger | Reaction time |
| 1 | 95% | 5% H2O | 1.5 h |
| 2 | 95% | 5% ethanedithiol/anisol (1:3) | 1,5 h |

The suspension of the peptide-resin in the suitable TFA mixture was stirred at room temperature for the stated time and then the resin was filtered off and washed with TFA and DCM. The filtrate and the washings were concentrated, and the peptide was precipitated by addition of diethyl ether. After cooling in an ice bath, the precipitate was filtered off, taken up in 30% acetic acid and lyophilized.

V. When an o-chlorotrityl-resin (supplied by Biohellas) is used, the suspension of the peptide-resin in an acetic acid/trifluoroethanol/dichloromethane mixture (1:1:3) is stirred at room temperature for 1 h. The resin is then filtered off with suction and thoroughly washed with the cleavage solution. The combined filtrates are concentrated in acuo and treated with water. The precipitated solid is removed by filtration or centrifugation, washed with diethyl ether and dried under reduced pressure.

VI. Purification and characterization of the peptides

Purification was carried out by gel chromatography (SEPHADEX G-10, G-15/10% HOAc, SEPHADEX LH20/MeOH) with or without subsequent medium pressure chromatography (stationary phase: HD-SIL C-18, 20-45 μ, 100 Å; mobile phase: gradient with A = 0.1% TFA/ MeOH, B = 0.1% TFA/$H_2O$).

The purity of the resulting products was determined by analytical HPLC (stationary phase: 100 2.1 mm VYDAC C-18, 5 l, 300 Å; mobile phase: $CH_3CN/H_2O$ gradient, buffered with 0.1% TFA, 40°C). Characterization was by amino-acid analysis and fast atom bombardment mass spectroscopy.

B. Specific procedures

EXAMPLE 1 (SEQ ID NO: 1)

N,N-Dimethyl-Val-Val-N-methyl-Val-Pro-Pro-Val-Phe-$NH_2$

1.98 g of Fmoc-RINK-resin (substitution 0.46 mmol/g), corresponding to a batch size of 0.84 mmol, were reacted as in AIb with 1.26 mmol each of

| | |
|---|---|
| Fmoc-Phe-OH | Fmoc-N-methyl-Val-OH |
| Fmoc-Val-OH | Fmoc-Val-OH |
| Fmoc-Pro-OH | Fmoc-Val-OH |
| Fmoc-Pro-OH | |

The amino acid following the N-methylamino acid was coupled on with PyBrop as coupling reagent. After the iterative synthetic cycles were completed, the peptide-resin underwent N-terminal deprotection (steps 2-4 in AIb), and was further reacted with aqueous formaldehyde solution as in AII and then dried under reduced pressure. The resulting resin was subjected to TFA cleavage as in AIV. The crude product (590 mg) was purified by gel filtration (SEPHADEX-LH-20). The yield was 295 mg.

EXAMPLE 2 (SEQ ID NO: 2)

N,N-Dimethyl-Val-Val-N-Me-Val-Pro 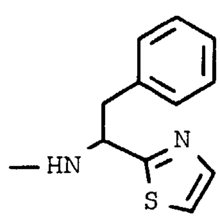

4.11 g of Fmoc-Pro-p-alkoxybenzyl-alcohol-resin (substitution 0.73 mmol/g), corresponding to a batch size of 3 mmol, were reacted as in AIb with 4.5 mmol each of

Fmoc-N-MeVal-OH
Fmoc-Val-OH
Fmoc-Val-OH.

The amino acid following the N-methylamino acid was in this case reacted with double coupling using PyBrop or Bop-Cl with increased reaction times. After the synthesis was complete, the peptide-resin underwent N-terminal deprotection (steps 2-4 in AIb), and was further reacted with aqueous formaldehyde solution as in AII and then dried under reduced pressure. The resin obtained in this way was subjected to TFA cleavage as in AIV. The crude product (750 mg) was employed directly for the next coupling. 100 mg of this compound were reacted with 45 mg of (S)-2-[1-amino-2-phenylethyl]thiazole and 230 mg of PyBop with the addition of 192 µl of DIPEA in DMF at room temperature for 2 d. The reaction mixture was purified by gel chromatography (Sephadex LH-20, methanol) and the product fractions were combined. 83 mg of product were obtained.

The following compounds were prepared and can be prepared according to examples 1 and 2:

3. Xaa Val Xan Pro Pro Val Phe
4. Xaa Val Xan Pro Pro Val Xac
5. Xaa Val Xan Pro Pro Val Xad
6. Xaa Val Xan Pro Pro Val Xae
7. Xaa Val Xan Pro Pro Val Xaf
8. Xaa Val Xan Pro Pro Val His-$NH_2$
9. Xbo Val Xan Pro Pro Val Phe-$NH_2$
10. Xaa Val Xan Pro Pro Val Xag-$NH_2$
11. Xaa Val Xan Pro Pro Val Xah
12. Xaa Xbe Xan Pro Pro Val Trp-$NH_2$
13. Xaa Val Xan Pro Pro Xai Phe-$NH_2$
14. Xaa Val Xan Pro Pro Ile Phe-$NH_2$
15. Xaa Val Xan Pro Xal Val Phe-$NH_2$
16. Xaa Val Xan Pro Xak Val Phe-$NH_2$
17. Xaa Val Xan Xak Pro Val Phe-$NH_2$
18. Xaa Val Xao Pro Pro Val Phe-$NH_2$
19. Xaa Val Xam Pro Pro Val Phe-$NH_2$
20. Xaa Ile Xan Pro Pro Val Phe-$NH_2$
21. Xaa Xai Xan Pro Pro Val Phe-$NH_2$
22. Xaa Leu Xan Pro Pro Val Phe-$NH_2$
23. Xar Val Xan Pro Pro Val Phe-$NH_2$
24. Xas Val Xan Pro Pro Val Phe-$NH_2$
25. Xat Val Xan Pro Pro Val Phe-$NH_2$
26. Xau Val Xan Pro Xal Val Phe-$NH_2$
27. Xan Val Xan Pro Pro Val Phe-$NH_2$
28. Xax Val Xan Pro Pro Val Phe-$NH_2$
29. Xaa Val Xan Pro Pro Phe Phe-$NH_2$
30. Xaa Val Xan Pro Pro Val-$NH_2$
31. Xaa Val Xan Pro Xbb

32. Xaa Val Xan Pro Xbc
33. Xaa Val Xan Pro Pro Xbd
34. Xax Val Xan Pro Pro Val-$NH_2$
35. Xat Val Xan Pro Pro Val-$NH_2$
36. Xaa Xai Xan Pro Pro Val-$NH_2$
37. Xaa Val Xan Pro Pro Xai-$NH_2$
38. Xaa Val Xan Xak Pro Val-$NH_2$
39. Xaa Val Xan Pro Xak Val-$NH_2$
40. Xaa Val Xan Pro Pro Val
41. Xaa Val Xan Pro Pro-$NH_2$
42. Xaa Val Xan Pro Pro
43. Xaa Val Xan Pro Xbf
44. Xaa Val Xan Xbb
45. Xaa Val Xan Xbc
46. Xaa Val Xan Xbg
47. Xaa Val Xan Xbh
48. Xaa Val Xan Xbi
49. Xaa Val Xan Xbk
50. Xaa Val Xan Xbl
51. Xaa Val Xan Xbm
52. Xaa Val Xan Xbn
53. Xax Val Xan Pro Pro-$NH_2$
54. Xbo Val Xan Pro Pro-$NH_2$
55. Xat Val Xan Pro Pro-$NH_2$
56. Xaa Xai Xan Pro Pro-$NH_2$
57. Xat Xai Xan Pro Pro-$NH_2$
58. Xaa Val Xan Pro
59. Xaa Val Xan Xbf
60. Xax Val Xan Pro-$NH_2$
61. Xbo Val Xan Pro-$NH_2$
62. Xaa Val Xan Pro Xbn
63. Xaa Val Xan Pro Xbg
64. Xaa Val Xan Pro Xbi
65. Xaa Val Xan Pro Xbl
66. Xbo Val Xan Pro Xbg
67. Xbo Val Xan Pro Xbl
68. Xaa Val Xan Pro Xbx
69. Xby Val Xan Pro Pro Val Phe $NH_2$
70. Xed Val Xan Pro Pro Val Phe $NH_2$
71. Xee Val Xan Pro Pro Val Phe $NH_2$
72. Xef Val Xan Pro Pro Val Phe $NH_2$
73. Xbz Val Xan Pro Pro Val Phe $NH_2$
74. Xeg Val Xan Pro Pro Val Phe $NH_2$
75. Xca Val Xan Pro Pro Val Phe $NH_2$
76. Xcb Val Xan Pro Pro Val Phe $NH_2$
77. Xcb Val Xao Pro Pro Val Phe $NH_2$
78. Xci Val Xan Pro Pro Val Phe $NH_2$

79. Xcl Val Xan Pro Pro Val Phe $NH_2$
80. Xby Val Xan Pro Pro Val $NH_2$
81. Xed Val Xan Pro Pro Val $NH_2$
82. Xee Val Xan Pro Pro Val $NH_2$
83. Xef Val Xan Pro Pro Val $NH_2$
84. Xbz Val Xan Pro Pro Val $NH_2$
85. Xeg Val Xan Pro Pro Val $NH_2$
86. Xca Val Xan Pro Pro Val $NH_2$
87. Xcb Val Xan Pro Pro Val $NH_2$
88. Xci Val Xan Pro Pro Val $NH_2$
89. Xcl Val Xan Pro Pro Val $NH_2$
90. Xby Val Xan Pro Pro $NH_2$
91. Xed Val Xan Pro Pro $NH_2$
92. Xee Val Xan Pro Pro $NH_2$
93. Xef Val Xan Pro Pro $NH_2$
94. Xbz Val Xan Pro Pro $NH_2$
95. Xeg Val Xan Pro Pro $NH_2$
96. Xca Val Xan Pro Pro $NH_2$
97. Xcb Val Xan Pro Pro $NH_2$
98. Xci Val Xan Pro Pro $NH_2$
99. Xcl Val Xan Pro Pro $NH_2$
100. Xaa Val Xan Pro Pro Val Xei
101. Xaa Val Xan Pro Pro Val Xem
102. Xaa Val Xan Pro Pro Val Xeo
103. Xaa Val Xan Pro Pro Val Xep
104. Xaa Val Xan Pro Pro Val Xeq
105. Xaa Val Xan Pro Pro Val Xex
106. Xaa Val Xan Pro Pro Val Xey
107. Xaa Val Xan Pro Pro Val Xfb
108. Xaa Val Xan Pro Pro Val Xfe
109. Xaa Val Xan Pro Pro Val Xfh
110. Xaa Val Xan Pro Pro Val Xfu
111. Xaa Val Xan Pro Pro Val Xfv
112. Xaa Val Xan Pro Pro Val Xft
113. Xaa Val Xan Pro Pro Val Xfw
114. Xaa Val Xan Pro Pro Val Xfx
115. Xaa Val Xan Pro Pro Val Xga
116. Xaa Val Xan Pro Pro Val Xgd
117. Xaa Val Xan Pro Pro Val Xgg
118. Xaa Val Xan Pro Pro Val Xgh
119. Xaa Val Xan Pro Pro Val Xgi
120. Xaa Val Xan Pro Pro Val Xgl
121. Xaa Val Xan Pro Pro Val Xgs
122. Xaa Val Xan Pro Pro Val Xgv
123. Xaa Val Xan Pro Pro Val Xhe
124. Xaa Val Xan Pro Pro Val Xgy
125. Xaa Val Xan Pro Pro Val Xhd

126. Xaa Val Xan Pro Pro Val Xhb
127. Xaa Val Xan Pro Pro Val Xhc
128. Xaa Val Xan Pro Pro Val Xhl
129. Xaa Val Xan Pro Pro Xeh
130. Xaa Val Xan Pro Pro Xen
131. Xaa Val Xan Pro Pro Xeo
132. Xaa Val Xan Pro Pro Xep
133. Xaa Val Xan Pro Pro Xeq
134. Xaa Val Xan Pro Pro Xer
135. Xaa Val Xan Pro Pro Xet
136. Xaa Val Xan Pro Pro Xeu
137. Xaa Val Xan Pro Pro Xes
138. Xaa Val Xan Pro Pro Xew
139. Xaa Val Xan Pro Pro Xez
140. Xaa Val Xan Pro Pro Xfc
141. Xaa Val Xan Pro Pro Xff
142. Xaa Val Xan Pro Pro Xfi
143. Xaa Val Xan Pro Pro Xfs
144. Xaa Val Xan Pro Pro Xfz
145. Xaa Val Xan Pro Pro Xgc
146. Xaa Val Xan Pro Pro Xgf
147. Xaa Val Xan Pro Pro Xgm
148. Xaa Val Xan Pro Pro Xgr
149. Xaa Val Xan Pro Pro Xgu
150. Xaa Val Xan Pro Pro Xgs
151. Xaa Val Xan Pro Pro Xgx
152. Xaa Val Xan Pro Pro Xha
153. Xaa Val Xan Pro Pro Xhk
154. Xaa Val Xan Pro Xek
155. Xaa Val Xan Pro Xen
156. Xaa Val Xan Pro Xer
157. Xaa Val Xan Pro Xep
158. Xaa Val Xan Pro Xeq
159. Xaa Val Xan Pro Xer
160. Xaa Val Xan Pro Xet
161. Xaa Val Xan Pro Xeu
162. Xaa Val Xan Pro Xes
163. Xaa Val Xan Pro Xfa
164. Xaa Val Xan Pro Xfd
165. Xaa Val Xan Pro Xfg
166. Xaa Val Xan Pro Xfl
167. Xaa Val Xan Pro Xfk
168. Xaa Val Xan Pro Xfm
169. Xaa Val Xan Pro Xfn
170. Xaa Val Xan Pro Xfo
171. Xaa Val Xan Pro Xfp
172. Xaa Val Xan Pro Xfq

173. Xaa Val Xan Pro Xfr
174. Xaa Val Xan Pro Xfy
175. Xaa Val Xan Pro Xgb
176. Xaa Val Xan Pro Xge
177. Xaa Val Xan Pro Xgk
178. Xaa Val Xan Pro Xgn
179. Xaa Val Xan Pro Xhi
180. Xaa Val Xan Pro Xgo
181. Xaa Val Xan Pro Xgp
182. Xaa Val Xan Pro Xgq
183. Xaa Val Xan Pro Xgt
184. Xaa Val Xan Pro Xgw
185. Xaa Val Xan Pro Xgz
186. Xaa Val Xan Pro Xhm
187. Xaa Val Xhf Pro Pro Val Phe $NH_2$
188. Xaa Val Xhg Pro Pro Val Phe $NH_2$
189. Xaa Val Xhh Pro Pro Val Phe $NH_2$
190. Xaa Val Xhf Pro Pro Val $NH_2$
191. Xaa Val Xhg Pro Pro Val $NH_2$
192. Xaa Val Xhh Pro Pro Val $NH_2$
193. Xaa Val Xhf Pro Pro $NH_2$
194. Xaa Val Xhg Pro Pro $NH_2$
195. Xaa Val Xhh Pro Pro $NH_2$
196. Xaa Val Xhf Pro Xfy
197. Xaa Val Xhg Pro Xfy
198. Xaa Val Xhh Pro Xfy
199. Xaa Val Xhf Pro Xgb
200. Xaa Val Xhg Pro Xgb
201. Xaa Val Xhh Pro Xgb
202. Xed Val Xan Pro Xfy
203. Xby Val Xan Pro Xfy
204. Xby Val Xan Pro Xhi
205. Xef Val Xan Pro Xfy
206. Xef Val Xan Pro Xhi
207. Xca Val Xan Pro Xfy
208. Xca Val Xan Pro Xhi
209. Xaa Val Xan Pro Xdp Phe $NH_2$
210. Xaa Val Xan Pro Xdq Phe $NH_2$
211. Xaa Val Xan Pro Xdr Phe $NH_2$
212. Xaa Val Xan Pro Xdp $NH_2$
213. Xaa Val Xan Pro Xdq $NH_2$
214. Xaa Val Xan Pro Xdr $NH_2$
215. Xaa Val Xan Pro Pro Xdi $NH_2$
216. Xaa Val Xan Pro Pro Xcs $NH_2$
217. Xaa Val Xan Pro Pro Xct $NH_2$
218. Xaa Val Xan Pro Pro Xcu $NH_2$
219. Xca Val Xan Pro Pro Phe Phe $NH_2$

220. Xby Val Xan Pro Pro Phe Phe $NH_2$
221. Xca Val Xan Pro Pro Phe Phe $NH_2$
222. Xef Val Xhf Pro Pro tLeu Phe $NH_2$
223. Xef Val Xhf Pro Pro tLeu Aic $NH_2$
224. Xef Val Xhf Pro Pro tLeu Tic $NH_2$
225. Xef Val Xan Pro Xfy
226. Xef Val Xan Pro Xbg
227. Xef Val Xan Pro Xbh
228. Xef Val Xan Pro Xgn
229. Xaa tLeu Xan Pro Pro Val Phe $NH_2$
230. Xaa Leu Xan Pro Pro Val Phe $NH_2$
231. Xaa Ile Xan Pro Pro Val Phe $NH_2$
232. Xaa Val Xan Pro Pro tLeu Phe $NH_2$
233. Xaa Val Xan Pro Pro Leu Phe $NH_2$
234. Xaa Val Xan Pro Pro Ile Phe $NH_2$
235. Xaa Val Xan Pro Pro Val Ala $NH_2$
236. Xht Val Xan Pro Pro Val Phe $NH_2$
237. Xhu Val Xan Pro Pro Val Phe $NH_2$
238. Xht Val Xan Pro Pro Val $NH_2$
239(a). Xhu Val Xan Pro Pro Val $NH_2$
240. Xht Val Xan Pro Pro $NH_2$
241. Xhu Val Xan Pro Pro $NH_2$
242. Xaa Val Xhy Pro Pro Val Phe $NH_2$
243. Xaa Val Xhz Pro Pro Val Phe $NH_2$
244. Xaa Val Xhy Pro Pro Val $NH_2$
245. Xaa Val Xhz Pro Pro Val $NH_2$
246. Xaa Val Xhy Pro Pro $NH_2$
247. Xaa Leu Xhz Pro Pro $NH_2$
248. Xaa Val Xhy Xfy
249. Xa Val Xhz Xfy
250. Xhv Val Xan Pro Pro Val Phe $NH_2$
251. Xhw Val Xan Pro Pro Val Phe $NH_2$
252. Xhx Val Xan Pro Pro Val Phe $NH_2$
253. Xhv Val Xan Pro Pro Val $NH_2$
254. Xhw Val Xan Pro Pro Val $NH_2$
255. Xhx Val Xan Pro Pro Val $NH_2$
256. Xhv Val Xan Pro Pro $NH_2$
257. Xhw Val Xan Pro Pro $NH_2$
258. Xhx Val Xan Pro Pro $NH_2$
259. Xaa Val Xan Pro Xia
260. Xaa Val Xan Pro Xib
261. Xaa Val Xan Pro Xic
262. Xaa Val Xan Pro Xid
263. Xaa Val Xan Pro Xie
264. Xby Val Xan Pro Xia
265. Xby Val Xan Pro Xib
266. Xby Val Xan Pro Xic

267. Xby Val Xan Pro Xid
268. Xby Val Xan Pro Xie
269. Xca Val Xan Pro Xia
270. Xca Val Xan Pro Xib
271. Xca Val Xan Pro Xic
272. Xca Val Xan Pro Xid
273. Xca Val Xan Pro Xie
274. Xed Val Xan Pro Xia
275. Xed Val Xan Pro Xib
276. Xed Val Xan Pro Xic
277. Xed Val Xan Pro Xid
278. Xed Val Xan Pro Xie
279. Xby Leu Xan Pro Xia
280. Xby Leu Xan Pro Xib
281. Xby Ile Xan Pro Xic
282. Xby Ile Xan Pro Xid
283. Xby Leu Xan Pro Xie
284. Xca Leu Xan Pro Xia
285. Xca Val Xao Pro Xib
286. Xca Val Xao Pro Xic
287. Xat Val Xhf Pro Xak Leu Phe $NH_2$
288. Xat Val Xhf Pro Xhr Leu Phe $NH_2$
289. Xed Val Xhf Pro Xak Leu Phe $NH_2$
290. Xed Val Xhf Pro Xhr Leu Phe $NH_2$
291. Xat Val Xhf Pro Xak Val $NH_2$
292. Xat Val Xhf Pro Xhr Val $NH_2$
293. Xed Val Xhf Pro Xak Val $NH_2$
294. Xed Val Xhf Pro Xhr Val $NH_2$
295. Xat Val Xhf Pro Xak $NH_2$
296. Xat Val Xhf Pro Xhr $NH_2$
297. Xed Val Xhf Pro Xak $NH_2$
298. Xat Val Xhf Pro Xia
299. Xat Val Xhf Pro Xib
300. Xed Val Xhf Pro Xic
301. Xed Val Xhf Pro Xid
302. Xat Val Xhf Pro Xie
303. Xat Val Xhf Pro Xhs $NH_2$
304. Xed Val Xhf Pro Xhs $NH_2$
305. Xat Val Xhf Pro Xak Xfz
306. Xat Val Xhf Pro Xhr Xfz
307. Xed Val Xhf Pro Xak Xfz
308. Xed Val Xhf Pro Xhr Xfz
309. Xat Val Xhf Pro Xak Xbw
310. Xat Val Xhf Pro Xhr Xbw
311. Xed Val Xhf Pro Xak Xbw
312. Xed Val Xhf Pro Xhr Xbw
313. Xat Val Xhf Pro Xak Xer

314. Xat Val Xhf Pro Xhr Xer
315. Xed Val Xhf Pro Xak Xer
316. Xed Val Xhf Pro Xhr Xer
317. Xat Val Xhf Pro Xak Xgi
318. Xat Val Xhf Pro Xhr Xgi
319. Xed Val Xhf Pro Xak Xgi
320. Xed Val Xhf Pro Xhr Xgi
321. Xat Val Xhf Pro Xak Xif
322. Xat Val Xhf Pro Xhr Xif
323. Xed Val Xhf Pro Xak Xif
324. Xed Val Xhf Pro Xhr Xif
325. Xat Val Xhf Pro Xak Xig
326. Xat Val Xhf Pro Xhr Xig
327. Xed Val Xhf Pro Xak Xig
328. Xed Val Xhf Pro Xhr Xig
329. Xaa Val Xan Pro Pro Xif
330. Xaa Val Xan Pro Xig
331. Xca Val Xan Pro Pro Xif
332. Xca Val Xan Pro Xig
333. Xby Val Xan Pro Pro Xif
334. Xby Val Xan Pro Xig
335. Xed Val Xan Pro Pro Xif
336. Xed Val Xan Pro Xig
337. Xaa Leu Xan Pro Pro Xif
338. Xaa Leu Xan Pro Xig
339. Xca Leu Xan Pro Pro Xif
340. Xca Leu Xan Pro Xig
341. Xby Leu Xan Pro Pro Xif
342. Xby Leu Xan Pro Xig
343. Xed Leu Xan Pro Pro Xif
344. Xed Leu Xan Pro Xig
345. Xaa Chg Xan Pro Pro Val $NH_2$
346. Xaa Chg Xan Pro Pro $NH_2$
347. Xaa Chg Xan Pro Pro Val Phe $NH_2$
348. Xaa Val Xii Pro Pro Val Phe $NH_2$
349. Xaa Val Xii Pro Pro Val $NH_2$
350. Xaa Val Xii Pro Pro $NH_2$
351. Xaa Val Xan Pro Xik
352. Xaa Val Xan Pro Pro Xab
353. Xim Val Xan Pro Pro Val Phe $NH_2$
354. Xin Val Xan Pro Pro Val Phe $NH_2$
355. Xim Val Xan Pro Pro Val $NH_2$
356. Xin Val Xan Pro Pro Val $NH_2$
357. Xim Val Xan Pro Pro $NH_2$
358. Xin Val Xan Pro Pro $NH_2$
359. Xaa Val Xan Pro Pro Val Xir
360. Xaa Val Xan Pro Pro Val Xis

361. Xaa Val Xan Pro Pro Val Xit
362. Xaa Val Xan Pro Pro Val Xiu
363. Xaa Val Xan Pro Pro Xiv
364. Xaa Val Xan Pro Pro Xiw
365. Xaa Val Xan Pro Pro Xiy
366. Xaa Val Xan Pro Pro Xix
367. Xaa Val Xan Pro Xiz
368. Xaa Val Xan Pro Xka
369. Xaa Val Xan Pro Xkb
370. Xaa Val Xan Pro Xkc
371. Xke Val Xan Pro Pro Val Phe $NH_2$
372. Xkf Val Xan Pro Pro Val Phe $NH_2$
373. Xke Val Xan Pro Pro Val $NH_2$
374. Xkf Val Xan Pro Pro Val $NH_2$
375. Xke Val Xan Pro Pro $NH_2$
376. Xkf Val Xan Pro Pro $NH_2$
377. Xed Val Xhf Pro Xhr $NH_2$

Examples for the MS-characterization of the synthesized novel compounds are given in the following table.

| EXAMPLE No. | Fast atom bombardment M S analysis Mol.-Weight (measured) | EXAMPLE No. | Fast atom bombardment M S analysis Mol.-Weight (measured) |
|---|---|---|---|
| 3 | 798 | 37 | 649 |
| 16 | 810 | 47 | 550 |
| 21 | 811 | 75 | 853 |
| 25 | 811 | 90 | 579 |
| 28 | 881 | 232 | 811 |
| 29 | 845 | | |

Table I -

| Sequence Identification of Compounds Prepared According to Examples 1 and 2 | |
|---|---|
| Compound Number(s) | Sequence ID Number |
| 31, 32, 43, 62-67, 68, 154-186, 196-208, 212-214, 225-228, 259-278, 285, 286, 295-304, 330, 332, 334, 336, 351, 367-370, 377 | 2 |
| 3, 9, 23-25, 27-28, 69-79, 187-189, 242, 243, 250-252, 348, 353, 354, 371, 372 | 3 |
| 4-7, 10, 11, 359-361 | 4 |
| 8 | 5 |
| 12 | 6 |
| 13, 222, 232 | 7 |
| 14 | 8 |
| 15, 16, 26 | 9 |
| 17 | 10 |
| 18, 19 | 11 |

Table I - (continued)

| Sequence Identification of Compounds Prepared According to Examples 1 and 2 | |
|---|---|
| Compound Number(s) | Sequence ID Number |
| 20, 231 | 12 |
| 21, 229 | 13 |
| 22, 230 | 14 |
| 29 | 15 |
| 30, 80-89, 190-192 | 16 |
| 33, 37, 129-153, 215-218, 329, 331, 333, 335, 352, 363-366 | 17 |
| 34, 35, 40, 349 | 18 |
| 36 | 19 |
| 38, 41, 42, 53-55, 90-99, 193-195, 240, 241, 246, 247, 256-258, 350, 357-358, 578-581 | 20 |
| 39 | 21 |
| 44-52, 59, 248, 249 | 22 |
| 56, 57, 346 | 23 |
| 58, 60-61 | 24 |
| 100-128 | 25 |
| 209-211 | 26 |
| 219-221 | 27 |
| 223, 224 | 28 |
| 233 | 29 |
| 234 | 30 |
| 235 | 31 |
| 236, 237, 244, 245, 253-255, 355, 356, 373, 374 | 32 |
| 238, 238(a), 279, 280, 283, 284, 338, 340, 342, 344 | 33 |
| 281, 282 | 34 |
| 291-294 | 35 |
| 286-290 | 36 |
| 305-328 | 37 |
| 341, 343 | 38 |
| 339 | 39 |
| 345 | 40 |
| 347 | 41 |

The symbols Xaa... in the summary have the following meanings:

Xaa: N,N-Dimethylvaline

Xab: 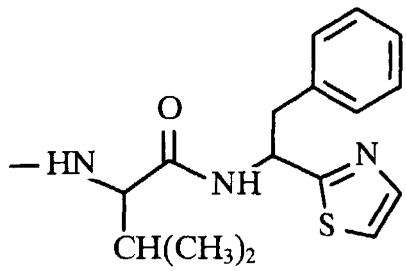

Xac: 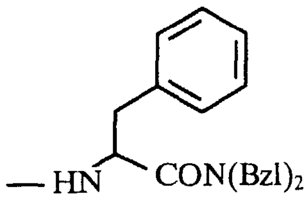

Xad: 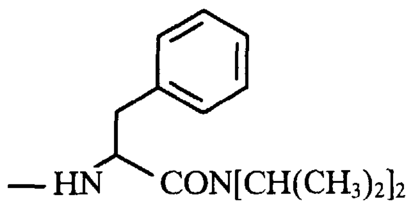

Xae: 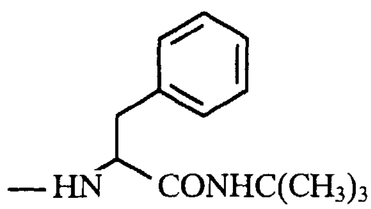

Xaf: 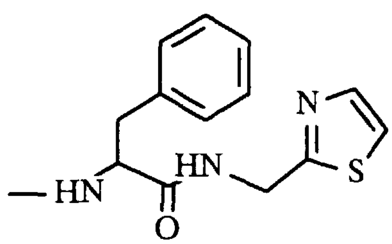

Xag: Tetrahydroisoquinoline carboxylic acid

Xah: 1-Aminoindane-1-carboxylic acid

Xai: tert-Leucine or 2-tert-butylglycine

Xak: Homoproline or pipecolic acid

Xal: 1-aminopentane-1-carboxylic acid

Xam: N-Methylisoleucine

Xan: N-Methylvaline

Xao: N-Methylleucine

Xar: N-N-Dimethylisoleucine

Xas: N,N - Dimethylleucine

Xat: N,N-Dimethyl-tert-leucine

Xau: N,N-Dimethyl-

Xax: N,N-Dibutylvaline

Xbb:

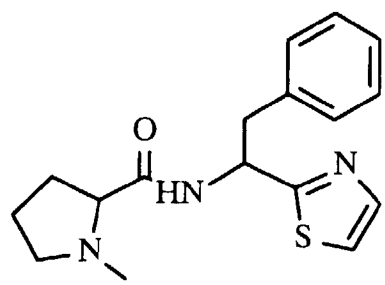

Xbc:

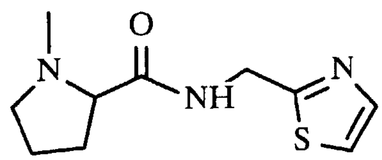

Xbd:

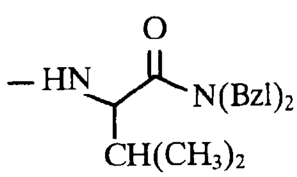

Xbf:

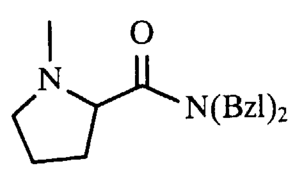

Xbg:

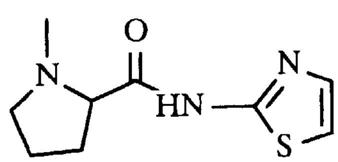

Xbh:

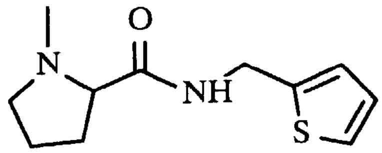

Xbi:

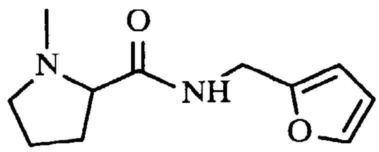

Xbk:

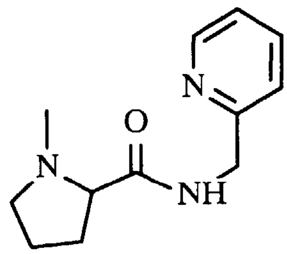

Xbl:

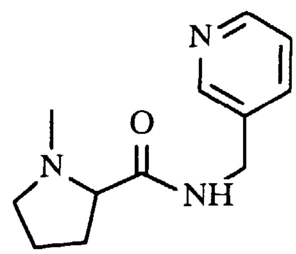

Xbm:

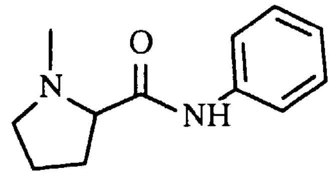

Xbn:

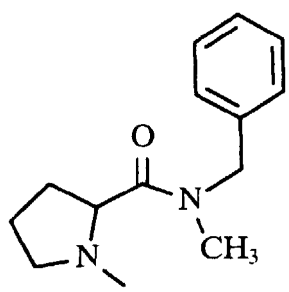

Xbo: N-Methyl-N-isopropylvaline

Xbw: 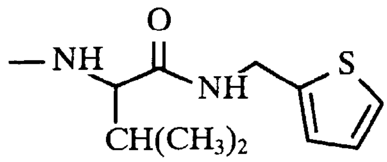

Xbx: 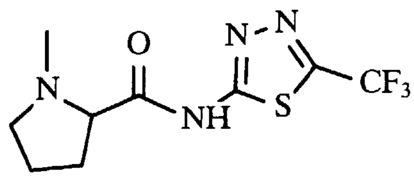

Xby: N,N-Diethylvaline

Xbz: N,N-Bis(2-fluoroethyl)valine

Xca: N,N-Dipropylvaline

Xcb: N-Cyclopropylvaline

Xci: 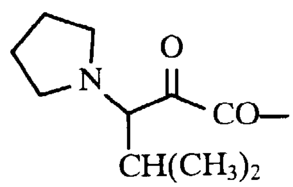

Xcl: 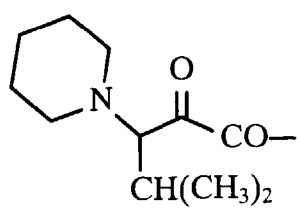

Xdp: 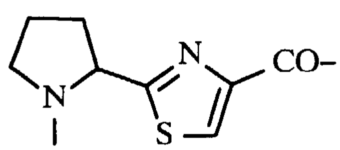

Xdq: 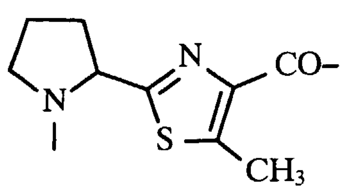

Xdr: 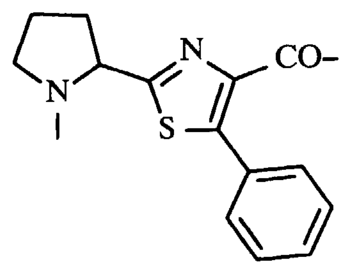

Xed: N,N-Diethyl-tert-leucin

Xee: N,N-Ditrifluoroethyl-tert-leucine

Xef: N,N-Dipropyl-tert-leucine

Xeg: N-Cyclopropyl-tert-leucine

Xei: 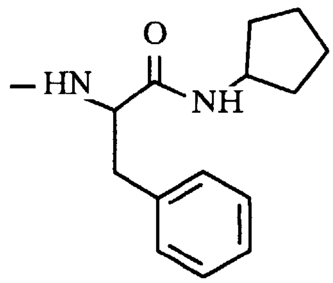

Xek: 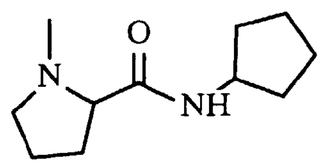

Xel: 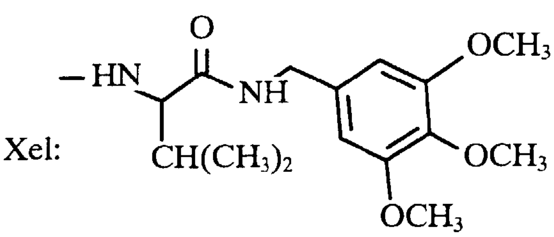

Xem: 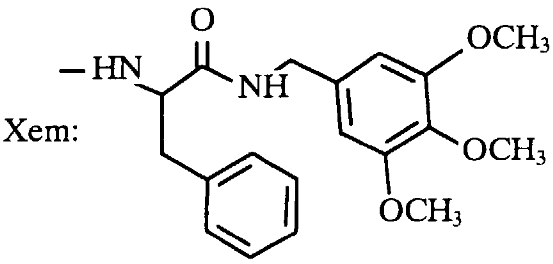

Xen: 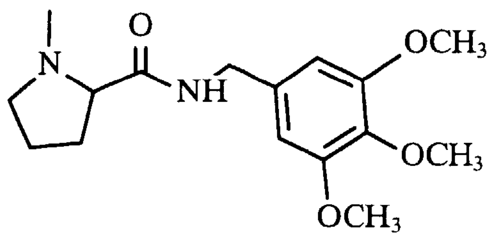

Xeo: 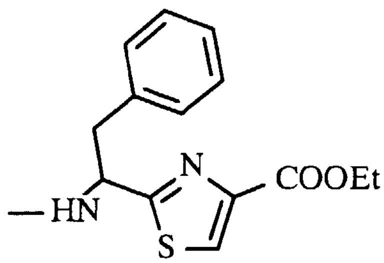

Xep: 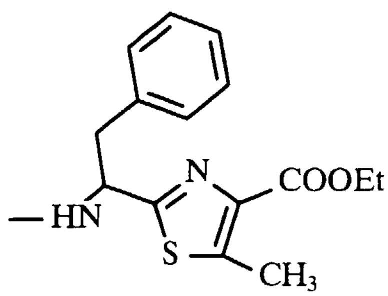

Xeq: 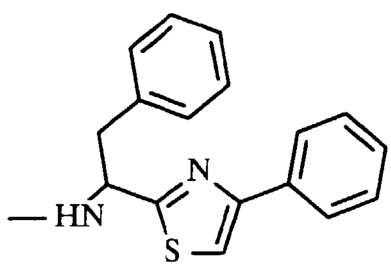

Xer: 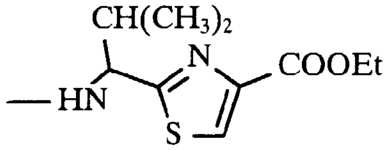

Xes: 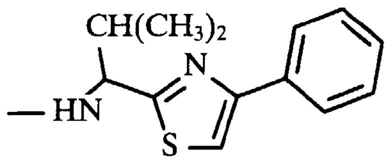

Xet: 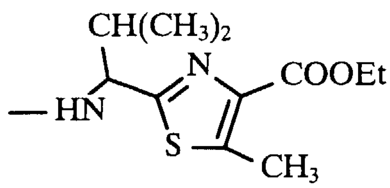

Xeu: 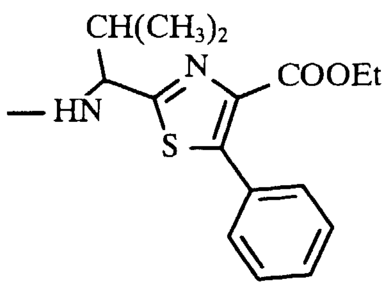

Xev: 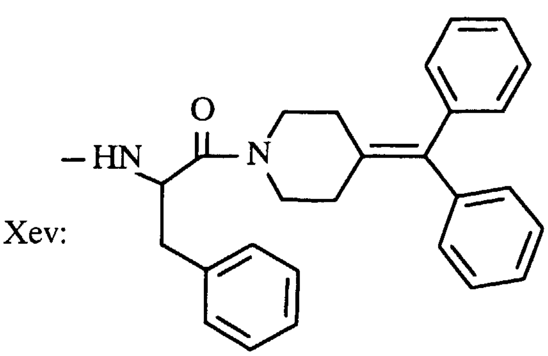

Xew: 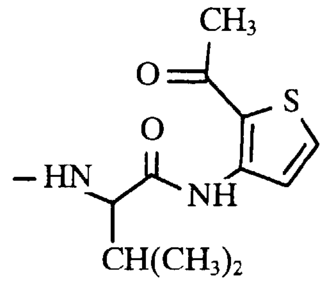

Xex: 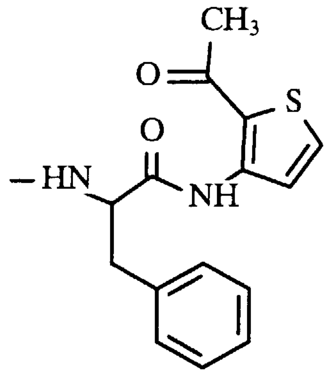

Xey: 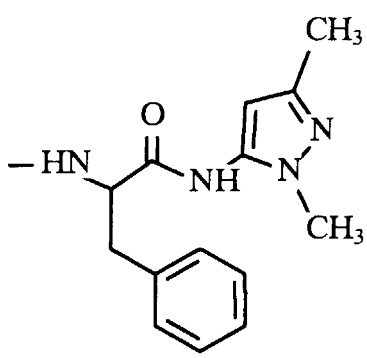

Xez: 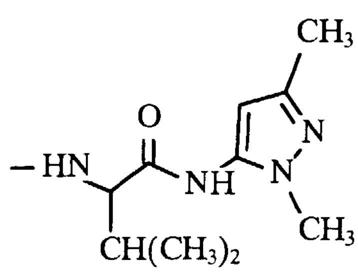

Xfa: 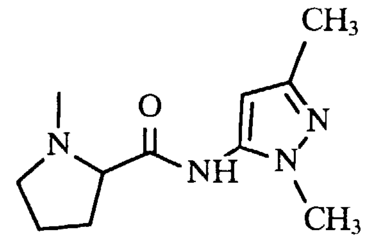

Xfb: 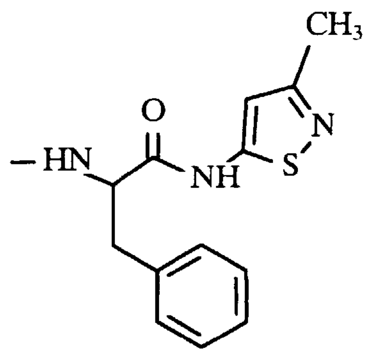

Xfc: 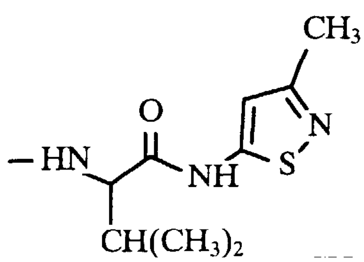

Xfd: 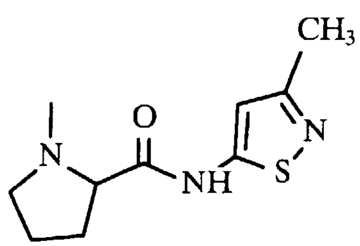

Xfe: 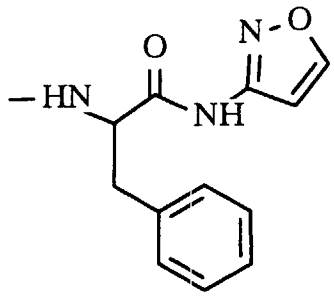

Xff: 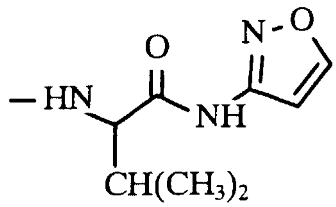

Xfg: 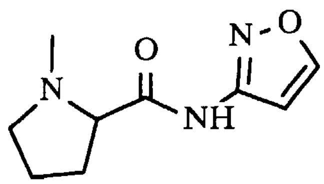

Xfh: 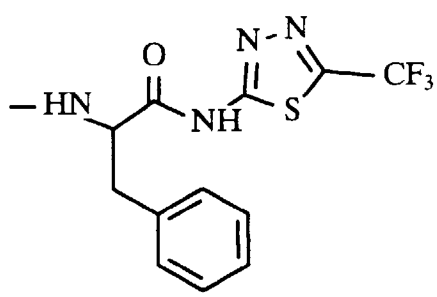

Xfi: 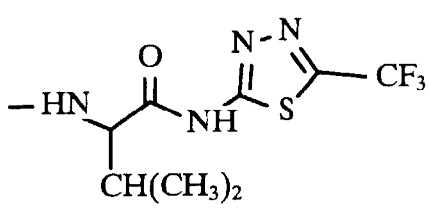

Xfk: 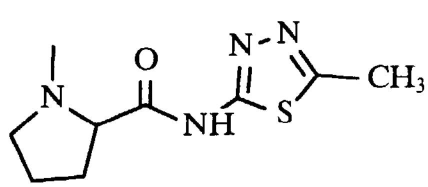

Xfl:

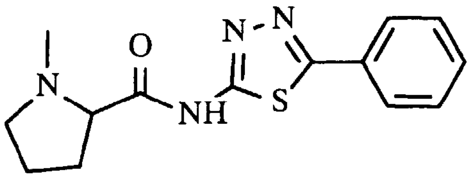

Xfm:

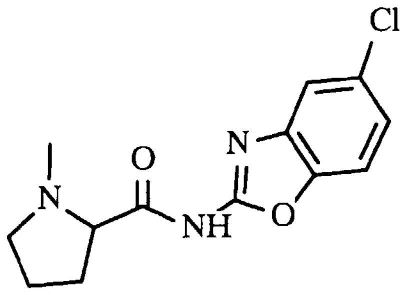

Xfn:

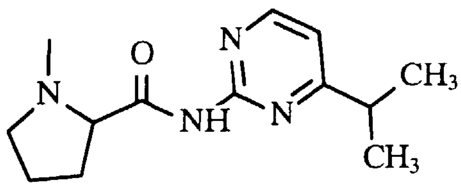

Xfo:

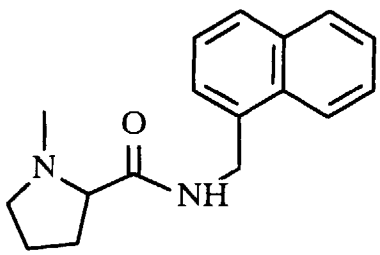

Xfp:

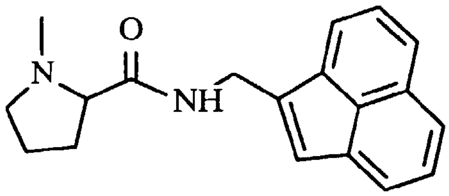

Xfq: 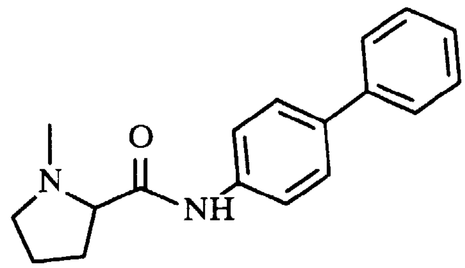

Xfr: 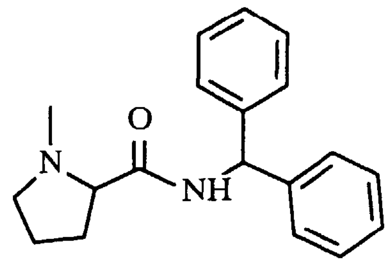

Xfs: 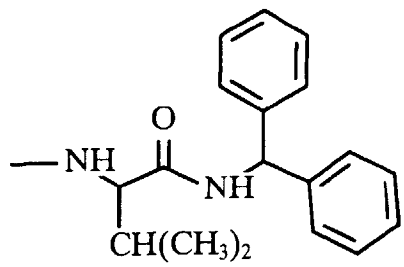

Xft: 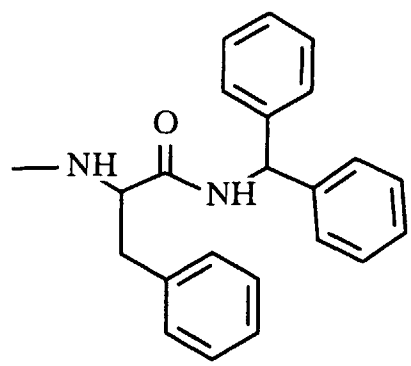

Xfu: 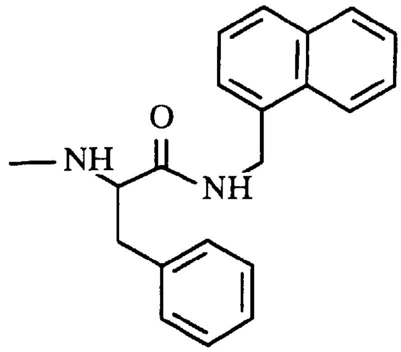

Xfv: 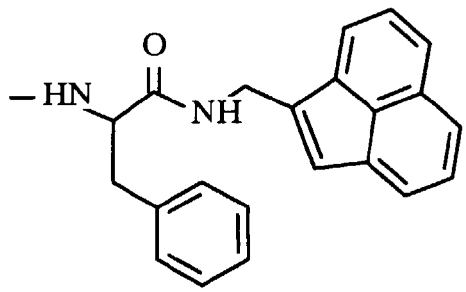

Xfw: 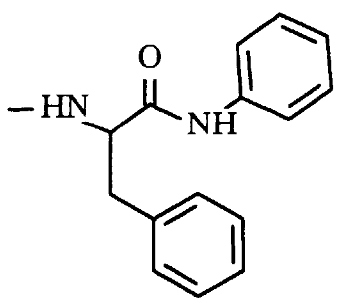

Xfx: 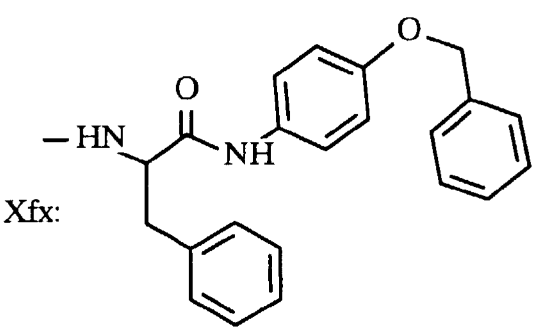

Xfy:

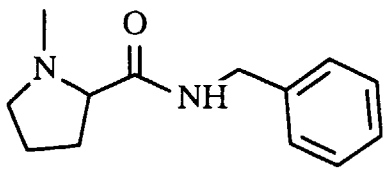

Xfz:

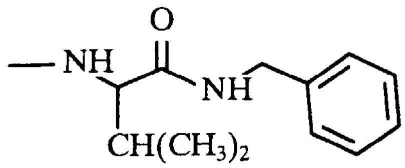

Xga:

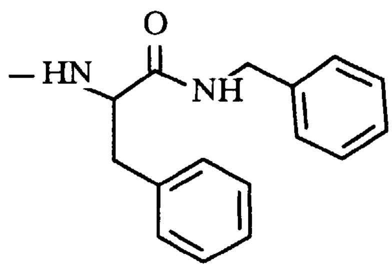

Xgb:

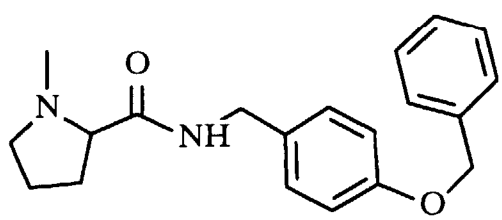

Xgc:

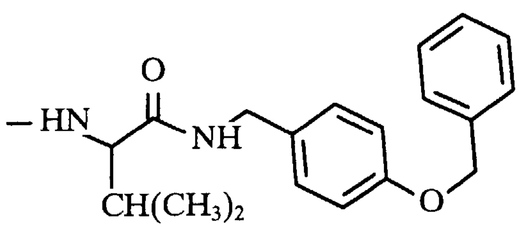

Xgd: 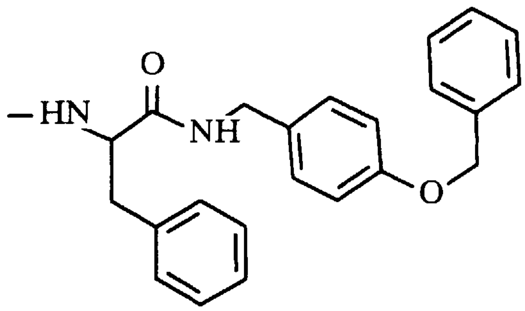

Xge: 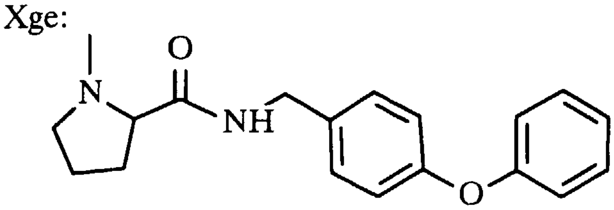

Xgf: 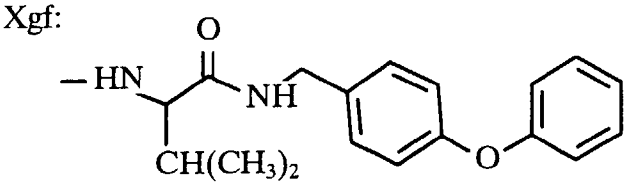

Xgg: 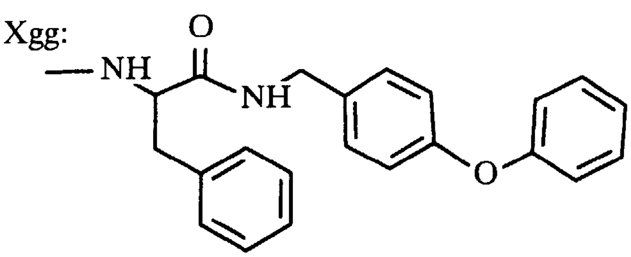

Xgh: 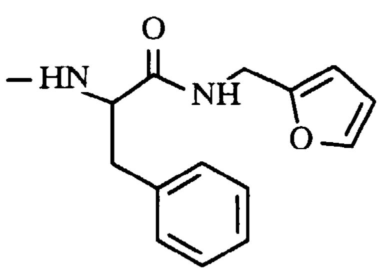

Xgi: 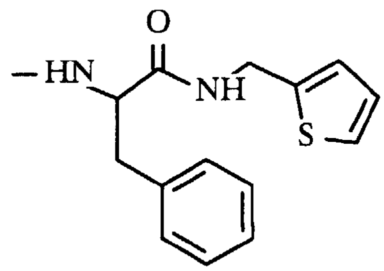

Xgk: 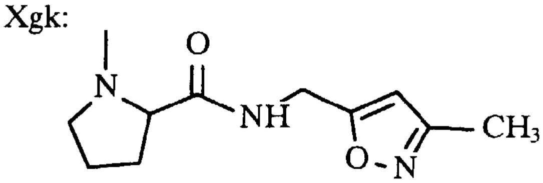

Xgl: 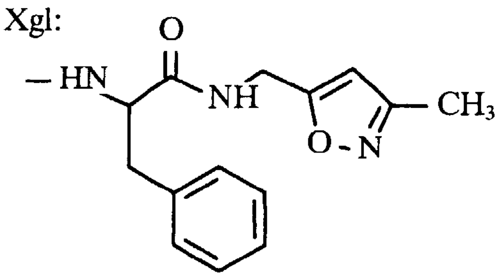

Xgm: 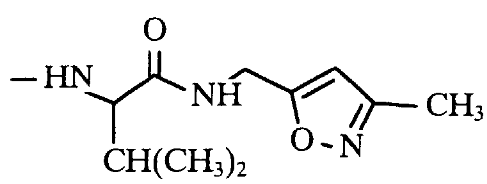

Xgn: 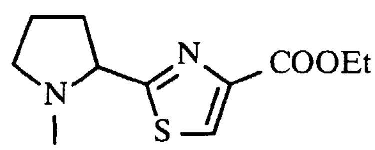

Xgo: 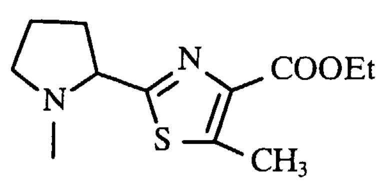

Xgp: 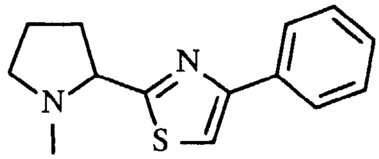

Xgq: 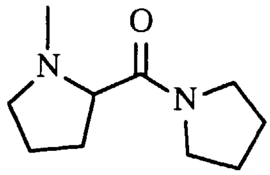

Xgr: 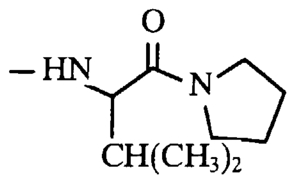

Xgs: 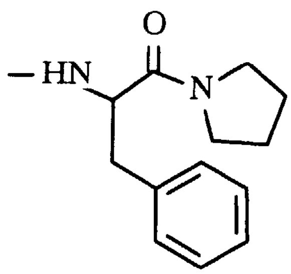

Xgt: 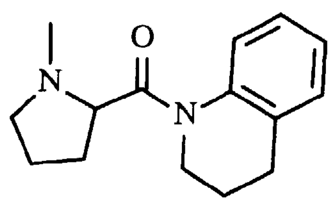

Xgu: 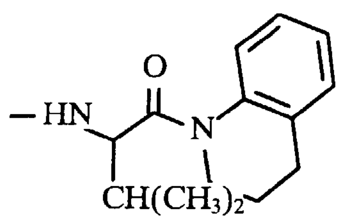

Xgv:

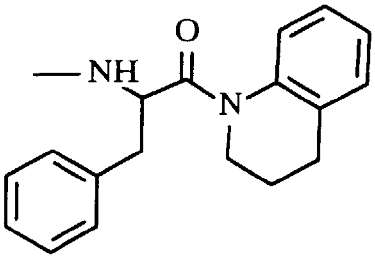

Xgw:

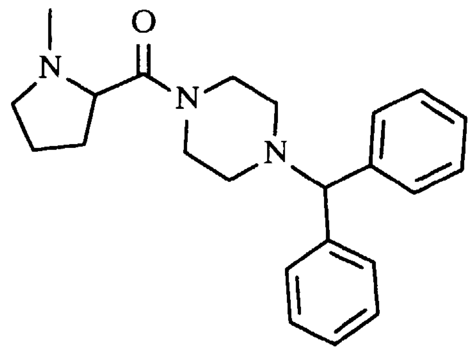

Xgx:

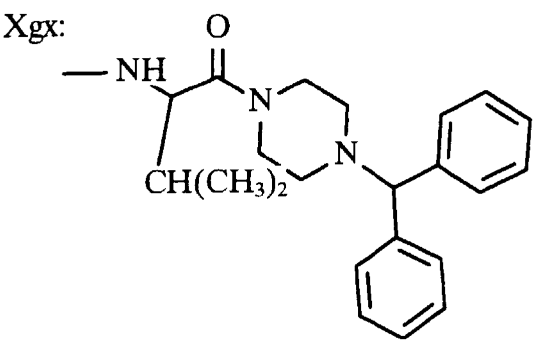

Xgy:

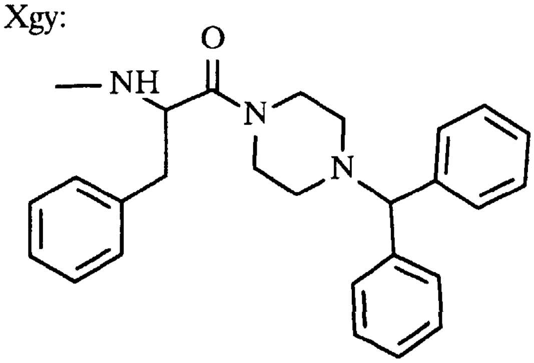

Xgz:

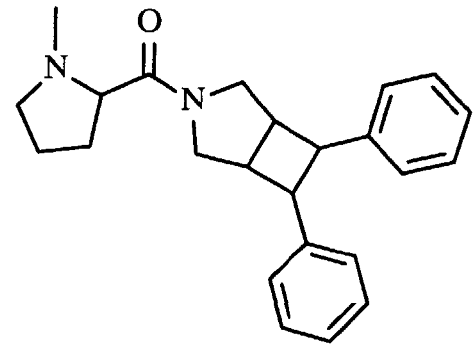

Xha:

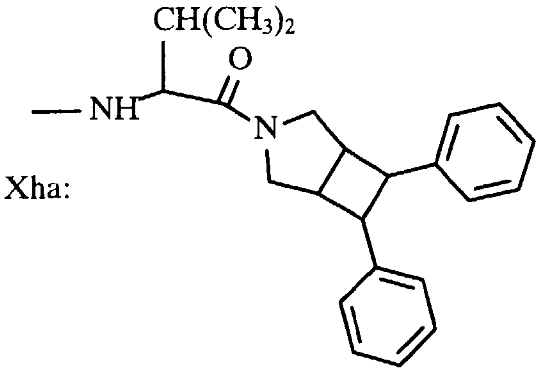

Xhb:

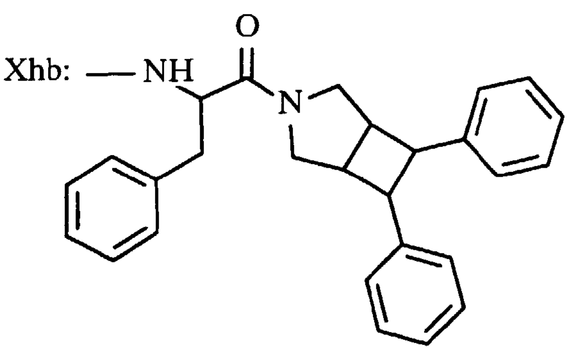

Xhc:

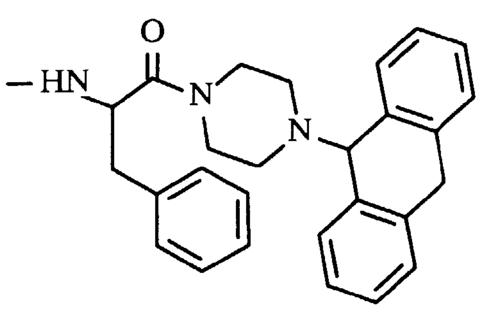

Xhd: 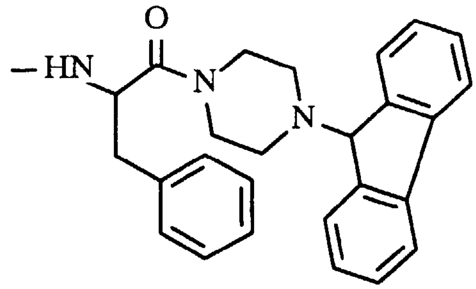

Xhe: 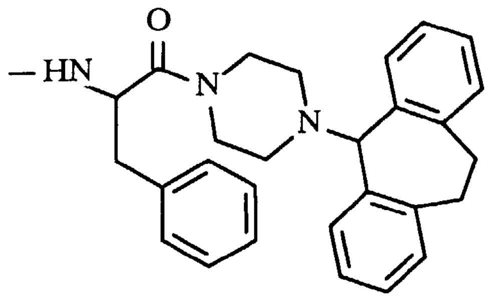

Xhf: N-Methyl-2-tert-butylglycine

Xhg: N-Methyl-3-tert-butylalanine

Xhh: N-Ethylvaline

Xhi: 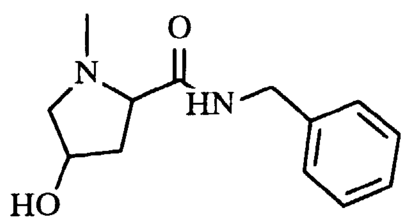

Xhk: 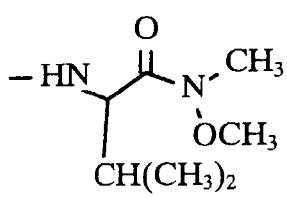

Xhl: 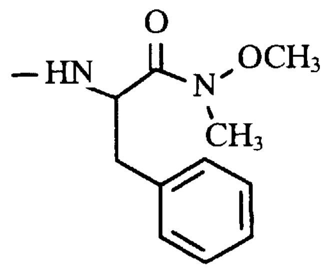

Xhm: 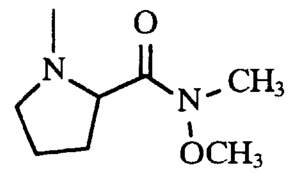

Xhr: Hydroxyproline

Xhs: 3-Thienylalanine

Xht: N,N-Dimethyl-3-cyclohexylalanine

Xhu: N,N-Diethyl-3-cyclohexylalanine

Xhv: N-Methyl-N-isopropyl-tert.-leucine

Xhw: N-Methyl-N-isopropyl-leucine

Xhx: N-Methyl-N-isopropyl-isoleucine

Xhy: N-Methyl-3-cyclohexyl-alanine

Xhz: N-Methyl-phenylalanine

Xia:

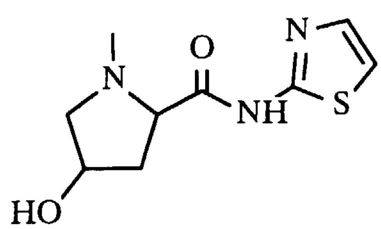

Xib:

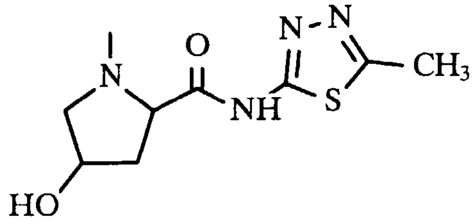

Xic:

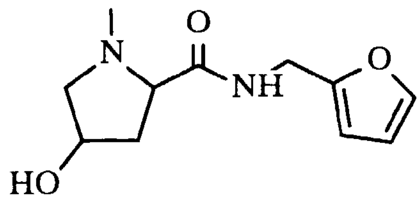

Xid:

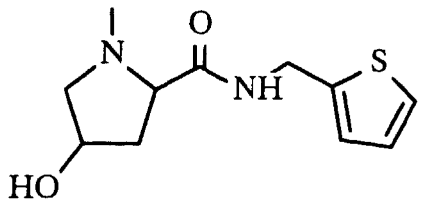

Xie:

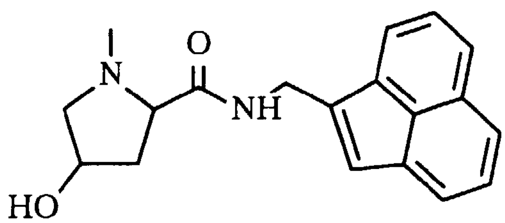

Xif:

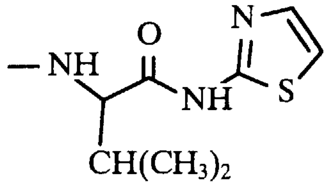

Xig: 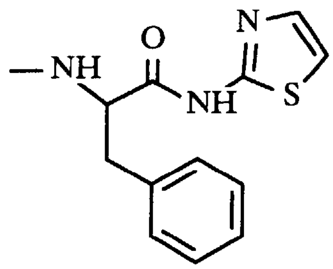

Xih: 2 -Cyclohexylglycine

Xii: N-Methyl-2-cyclohexylglycine

Xik: 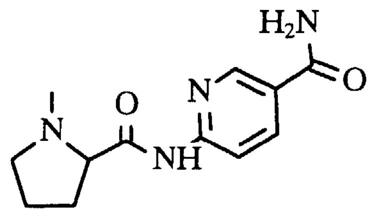

Xil: 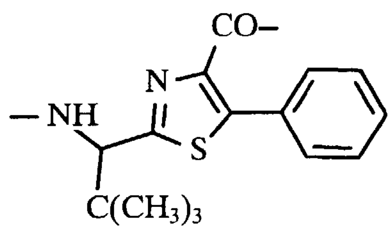

Xim: N-Methylaminosuflonyl-valine

Xin: N-tert.butylaminosulfonyl-valine

Xir: Phenylalanine-methylester

Xis: Phenylalanine-ethylester

Xit: Phenylalanine-benzylester

Xiu: Phenylalanine-tert.butylester

Xiv: Valine-benzylester

Xiw: Valine-methylester

Xix: Valine-ethylester

Xiy: Valine-tert.butylester

Xiz: Proline-benzylester

Xka: Proline-methylester

Xkb: Proline-ethylester

Xkc: Proline-tert.butylester

Xke: N-Methylsulfonyl-valine

Xkf: N -Methyl-N-methylsulfonyl-valine

The ending $-NH_2$ has the meaning that the C-terminal amino acid is in its amide form.

Compounds of this invention may be assayed for anti-cancer activity by conventional methods, including for example, the methods described below.

A. In vitro methodology

Cytotoxicity may be measured using a standard methodology for adherent cell lines such as the microculture tetrazolium assay (MTT). Details of this assay have been published (Alley, MC et al, Cancer Research 48:589-601, 1988). Exponentially growing cultures of tumor cells such as the HT-29 colon carcinoma or LX-1 lung tumor are used to make microtiter plate cultures. Cells are seeded at 5000-20,000 cells per well in 96-well plates (in 150 µl of media), and grown overnight at 37°C. Test compounds are added, in 10-fold dilutions varying from $10^{-4}$ M to $10^{-10}$ M. Cells are then incubated for 48 hours. To determine the number of viable cells in each well, the MTT dye is added (50 µl of 3 mg/ml solution of 3-(4,5-dimethylthiazol-2-yl)-2,5-diphenyltetrazolium bromide in saline). This mixture is incubated at 37°C for 5 hours, and then 50 µl of 25 % SDS, pH2 is added to each well. After an overnight incubation, the absorbance of each well at 550 nm is read using an ELISA reader. The values for the mean +/- SD of data from replicated wells are calculated, using the formula % T/C (% viable cells treated/control).

$$\frac{\text{OD of treated cells}}{\text{OD of control cells}} \times 100 = \%\ \text{T/C}$$

The concentration of test compound which gives a T/C of 50 % growth inhibition was designated as the $IC_{50}$

B. In vivo methodology

Compounds of this invention may be further tested in any of the various pre-clinical assays for in vivo activity which are indicative of clinical utility. Such assays are conducted with nude mice into which tumor tissue, preferably of human origin, has been transplanted ("xenografted"), as is well known in this field. Test compounds are evaluated for their anti-tumor efficacy following administration to the xenograft-bearing mice.

More specifically, human tumors which have been grown in athymic nude mice are transplanted into new recipient animals, using tumor fragments which are about 50 mg in size. The day of transplantation is designated as day 0. Six to ten days later, mice are treated with the test compounds given as an intravenous or intraperitoneal injection, in groups of 5-10 mice at each dose. Compounds were given daily for 5 days, 10 days or 15 days, at doses from 10-100 mg/kg body weight. Tumor diameters and body weights were measured twice weekly. Tumor volumes are calculated using the diameters measured with Vernier calipers, and the formula:

$$(\text{length} \times \text{width}^2)/2 = \text{mg of tumor weight}$$

Mean tumor weights are calculated for each treatment group, and T/C values determined for each group relative to the untreated control tumors.

The novel compounds of the present invention show good in vitro activity in the above mentioned assay systems and antitumor activity in the above mentioned in vivo system.

## Claims

**1.** A peptide with anticancer activity of the formula I

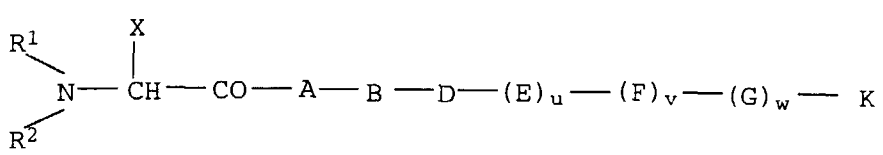 I

where

| | |
|---|---|
| $R^1$ | is alkyl; cycloalkyl; alkylsulfonyl; fluoroalkyl; or $NR^3R^4$ where $R^3$ and $R^4$ may each be either hydrogen or alkyl; |
| $R^2$ | is hydrogen; alkyl; fluoroalkyl; cycloalkyl; or |
| $R^1$-N-$R^2$ | together may be |

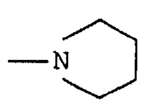 or 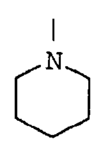

| | |
|---|---|
| A | is a valyl, isoleucyl, leucyl, allo-isoleucyl, 3-tert-butylalanyl, 2-tert-butylglycyl, 3-cyclohexylalanyl, 2-ethylglycyl, 2-cyclohexylglycyl, norleucyl or norvalyl residue; |
| B | is a N-alkyl-valyl, -norvalyl, -leucyl, -isoleucyl, -2-tert-butylglycyl, -3-tert-butylalanyl, -3-cyclohex- |

ylalanyl, -phenylalanyl, or -2-cyclohexylglycyl residue;

D and E are independently selected from the group consisting of prolyl, homo-prolyl, hydroxyprolyl, thiazolidinyl-4-carbonyl;

F and G are independently selected from the group consisting of prolyl, homo-prolyl, hydroxyprolyl, thiazolidinyl-4-carbonyl, 1-aminopentyl-1-carbonyl, valyl, 2-tert-butylglycyl, isoleucyl, leucyl, 3-cyclohexylalanyl, phenylalanyl, N-methylphenylalanyl, tetrahydroisoquinolyl-2-carbonyl, 3-thiazolylalanyl, 3-thienylalanyl, histidyl, 1-aminoindyl-1-carbonyl, 3-pyridylalanyl, 3-tert-butylalanyl, 2-cyclohexylglycyl, norvalyl, norleucyl and 3-naphthylalanyl residues

X is alkyl, cycloalkyl, $-CH_2$-cyclohexyl or arylalkyl

E and F together may be

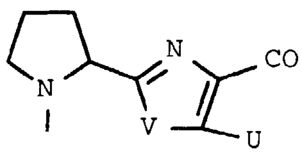

where V is oxygen or sulfur; U is hydrogen, $C_{1-4}$-alkyl, phenyl, or cycloalkyl;

u,v,and w are independently 0 or 1; and

K is hydroxy, alkoxy, phenoxy, benzyloxy or a substituted or unsubstituted amino moiety;

and the salts thereof with physiologically tolerated acids.

**2.** Compounds of formula I according to claim 1 wherein $R^1$-N-$R^2$ is

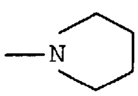

or

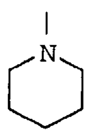

**3.** Compounds of formula I according to claim 1 wherein K is an amino moiety of the formula $R^5$-N-$R^6$ wherein

$R^5$ is hydrogen, or hydroxy, or $C_{1-7}$-alkoxy, or benzyloxy, or $C_{1-7}$-alkyl, or fluoroalkyl, or $C_{3-7}$-cycloalkyl, or benzyl which may be substituted by up to three substituents which may independently be $CF_3$, nitro, $C_{1-7}$-alkylsulfonyl, $C_{1-4}$-alkoxy, phenoxy, benzoxy, halogen or $C_{1-4}$-alkyl

$R^6$ is H, or $C_{1-7}$-alkyl, or $C_{3-7}$-cycloalkyl, or fluoroalkyl, or phenyl (which may be substituted by up to three substituents which may independently be $CF_3$, nitro, halogen, CONHBzl, CON(Bzl)$_2$, $C_{1-4}$-alkyl which may form a cyclic system, $C_{1-4}$-alkoxy, phenoxy, benzoxy, or $C_{1-7}$-alkyl-sulfonyl), or
benzyl (which may be substituted by up to three substituents which may independently be $CF_3$, nitro, halogen, CONHBzl, CON(Bzl)$_2$, $C_{1-4}$-alkyl which may form a cyclic system, $C_{1-4}$-alkoxy, phenoxy, benzoxy, or $C_{1-7}$-alkylsulfonyl), or
naphthyl (which may be substituted by up to two substituents which may independently be $CF_3$, nitro, halogen, CONHBzl, CON(Bzl)$_2$, $C_{1-4}$-alkyl, $C_{1-4}$-alkoxy, benzoxy, phenoxy, or $C_{1-7}$-alkyl-sulfonyl), or
benzhydryl (which may be substituted by up to two substituents which may independently be $CF_3$, nitro, halogen, CONHBzl, CON(Bzl)$_2$, $C_{1-4}$-alkyl, $C_{1-4}$-alkoxy, phenoxy, benzoxy, or $C_{1-7}$-alkyl-sulfonyl), or
biphenyl (which may be substituted by up to two substituents which may independently be $CF_3$, nitro, halogen, CONHBzl, CON(Bzl)$_2$, $C_{1-4}$-alkyl, $C_{1-4}$-alkoxy, phenoxy, benzoxy, or $C_{1-7}$-alkyl-sulfonyl), or
triphenylmethyl (which may be substituted by up to three substituents which may independently be $CF_3$,

nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or benzhydrylethyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or benzhydrylmethyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or naphthylmethyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or acenaphthyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or acenaphthylmethyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or pyridyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or picolyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or benzothiazolyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or benzisothiazolyl (which may be substituted by up to two substituents which may independently be $CF_3$, nitro, halogen, CONHBzl, $CON(Bzl)_2$, $C_{1-4}$-alkyl,$C_{1-4}$-alkoxy, phenoxy, benzoxy, or $C_{1-7}$-alkyl-sulfonyl), or benzopyrazolyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or benzoxazolyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or fluorenyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or aminofluorenyl (which may be substituted by up to two substituents which may independently be $CF_3$, nitro, halogen, CONHBzl, $CON(Bzl)_2$, $C_{1-4}$-alkyl, $C_{1-4}$-alkoxy, phenoxy, benzoxy, or $C_{1-7}$-alkyl-sulfonyl), or pyrimidyl (which may be substituted by up to two substituents which may independently be $CF_3$, nitro, halogen, COOEt, CONHBzl, $CON(Bzl)_2$, $C_{1-4}$-alkyl which may form a cyclic system, $C_{1-4}$-alkoxy, phenoxy, benzoxy, or $C_{1-7}$-alkyl-sulfonyl), or 5-membered heteroaryl [which may be substituted by up to three substituents which may independently be $CF_3$, nitro, halogen, cyano, COOMe, COOEt, thiomethyl, thioethyl, thiophenyl, picolyl, acetyl, -$CH_2$-COOEt, $CONH_2$ CONHBzl, $CON(Bzl)_2$, $C_{1-4}$-alkyl which may form a cyclic system, $C_{1-4}$-alkoxy, phenoxy, benzoxy, phenyl (which may be substituted by up to four substituents which may independently be nitro, $CF_3$, halogen, or $C_{1-4}$-alkyl), benzyl (which may be substituted by up to four substituents which may independently be nitro, $CF_3$, halogen, $C_{1-4}$-alkyl, naphthyl, $C_{1-7}$-alkyl-sulfonyl, phenylsulfonyl, or $C_{1-4}$-dialkylamino)], or -$CHR^7$-5-membered heteroaryl (which may be substituted by up to two substituents which may independently be $CF_3$, nitro, halogen, CONHBzl, $CON(Bzl)_2$, COOMe, COOEt, $COOCH(CH_3)_2$, $CONH_2$, COOBzl, $C_{1-4}$-alkyl, $C_{1-4}$-alkoxy, phenoxy, benzoxy, phenyl, benzyl, naphthyl, or $C_{1-7}$-alkylsulfonyl [$R^7$ = H, linear or branched $C_{1-5}$-alkyl, benzyl; or $R^7$ and $R^5$ together form a group -$(CH_2)_3$- or -$(CH_2)_4$-]).

**4.** Compounds of formula I according to claim 1 wherein K is $R^5$-N-$R^6$ which together may form structures selected from the group consisting of

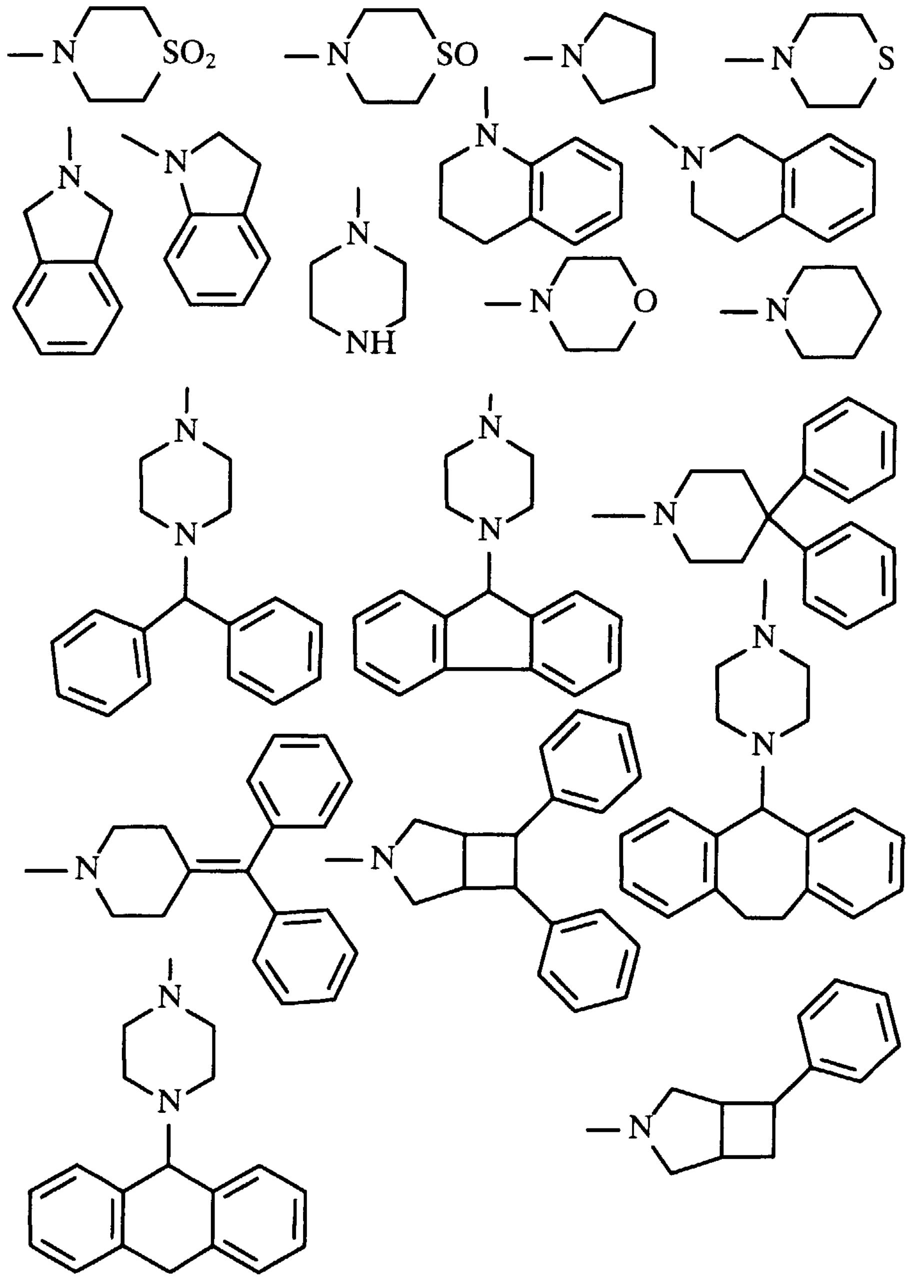

which may be unsubstituted or substituted with one or more substituents independently selected from the group consisting of $CF_3$, nitro, halogen, oxo, cyano, N,N-dimethylamino, CONHBzl, $CON(Bzl)_2$, $C_{1-6}$-alkyl, $C_{3-4}$-alkylen group forming an annelated ring system, $C_{1-4}$-alkoxy, phenoxy, benzoxy, naphthyl, pyrimidyl, COOEt, COOBzl, $C_{3-6}$-cycloalkyl, pyrolidinyl, piperidinyl, thienyl, pyrolyl, $-CH_2-CO-NCH(CH_3)_2$, $-CH_2-CO-N(CH_2)_4$, $-CH_2-CO-N(CH_2)_4O$, benzyl (which may be substituted by up to three substituents independently selected from the group consisting of nitro, halogen, $CF_3$, thiomethyl or the corresponding sulfoxide or sulfone, thioethyl or the corresponding sulfoxide or sulfone, $C_{1-4}$-alkyl, and $C_{1-4}$-alkoxy), and phenyl (which may be substituted by up to three substituents independently selected from the group consisting of nitro, halogen, $CF_3$, thiomethyl, thioethyl, $C_{1-4}$-alkyl, and $C_{1-4}$-alkoxy).

**5.** Compounds of formula I according to claim 1 wherein u, v, and w are zero and K is not an hydroxy, benzoxy, phenoxy or alkoxy moiety.

**6.** Compounds of formula I according to claim 1 wherein u and v are zero and K is not an hydroxy or alkoxy moiety.

7. Compounds of formula I according to claim 1 wherein u, v and w are 1 and K is a hydroxy, alkoxy, phenoxy or benzyloxy moiety.

8. Compounds of formula I according to claim 1 wherein u and v are 1, w is O and K is a hydroxy, alkoxy, phenoxy or benzyloxy moiety.

9. Compounds of formula I according to claim 1 wherein u is 1, v and w are 0 and K is a hydroxy, alkoxy, phenoxy or benzyloxy moiety.

10. Use of compounds of formula I or salts thereof for the preparetion of medicines for treating oncological diseases.

11. A pharmaceutical composition comprising a pharmaceutically acceptable carrier and a therapeutically effective amount of a compound of claim 1.

12. The method of preparing compounds of formula I according to claim 1 characterized in that they are prepared according to known methods of peptide chemistry.

**Patentansprüche**

1. Gegen Krebs wirksames Peptid mit der Formel I

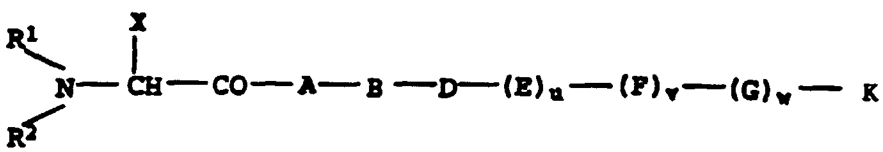

I

worin:

| | |
|---|---|
| $R^1$ | Alkyl; Cycloalkyl; Alkylsulfonyl; Fluoralkyl; oder $NR^3R^4$, worin $R^3$ und $R^4$ jeweils entweder Wasserstoff oder Alkyl sein können; |
| $R^2$ | Wasserstoff; Alkyl; Fluoralkyl; Cycloalkyl; oder |
| $R^1$-N-$R^2$ | gemeinsam |

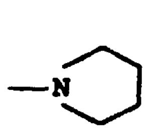

oder

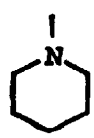

sein können

| | |
|---|---|
| A | ein Valyl-, Isoleucyl-, Leucyl-, Allo-Isoleucyl-, 3-tert-Butylalanyl-, 2-tert-Butylglycyl-, 3-Cyclohexylalanyl-, 2-Ethylglycyl-, 2-Cyclohexylglycyl-, Norleucyl- oder Norvalylrest ist; |
| B | einen N-Alkyl-valyl-, -norvalyl-, -leucyl-, -isoleucyl-, -2-tert-butylglycyl-, -3-tert-butylalanyl-, -3-cyclohexylalanyl-, -phenylalanyl- oder -2-cyclohexylglycylrest bedeutet; |
| D und E | jeweils unabhängig voneinander aus der Gruppe umfassend Prolyl, Homo-prolyl, Hydroxyprolyl, Thiazolidinyl-4-carbonyl ausgewählt sind; |
| F und G | unabhängig voneinander aus der Gruppe umfassend Prolyl-, Homo-prolyl-, Hydroxyprolyl-, Thiazolidinyl-4-carbonyl-, 1-Aminopentyl-1-carbonyl-, Valyl-, 2-tert-Butylglycyl-, Isoleucyl-, Leucyl-, 3-Cyclohexylalanyl-, Phenylalanyl-, N-Methyl-phenylalanyl-, Tetrahydroisoquinolyl-2-carbonyl-, |

3-Thiazolylalanyl-, 3-Thienylalanyl-, Histidyl-, 1-Aminoindyl-1-carbonyl-, 3-Pyridylalanyl-, 3-tert-Butylalanyl-, 2-Cyclohexylglycyl-, Norvalyl-, Norleucyl- und 3-Naphthylalanylreste ausgewählt sind

X Alkyl, Cycloalkyl, $-CH_2$-Cyclohexyl oder Arylalkyl bedeutet;

E und F gemeinsam

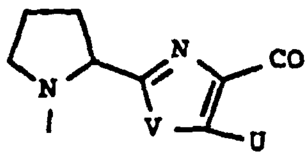

bedeuten können,

worin V Sauerstoff oder Schwefel bedeutet und U Wasserstoff, $C_{1-4}$-Alkyl, Phenyl oder Cycloalkyl bedeutet;

u, v und w unabhängig voneinander 0 oder 1 sind; und

K Hydroxy, Alkoxy, Phenoxy, Benzyloxy oder einen substituierten oder unsubstituierten Aminorest bedeutet;

sowie eines seiner Salze mit physiologisch unbedenklichen Säuren.

**2.** Verbindungen der Formel I nach Anspruch 1, worin $R^1$-N-$R^2$

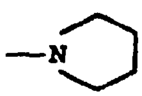

oder

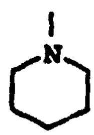

bedeutet.

**3.** Verbindungen der Formel I nach Anspruch 1, worin K einen Aminorest der Formel $R^5$-N-$R^6$ bedeutet, worin $R^5$ und $R^6$ folgende Bedeutungen haben;

$R^5$ Wasserstoff, oder Hydroxy, oder $C_{1-7}$Alkoxy, oder Benzyloxy, oder $C_{1-7}$-Alkyl, oder Fluoralkyl, oder $C_{3-7}$-Cycloalkyl, oder Benzyl, das durch bis zu drei Substituenten substituiert sein kann, wobei es sich bei diesen Substituenten unabhängig voneinander um $CF_3$, Nitro, $C_{1-7}$-Alkylsulfonyl, $C_{1-4}$-Alkoxy, Phenoxy, Benzoxy, Halogen oder $C_{1-4}$-Alkyl handeln kann,

$R^6$ H, oder $C_{1-7}$-Alkyl, oder $C_{3-7}$-Cycloalkyl, oder Fluoralkyl, oder Phenyl (das durch bis zu drei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, das ein zyklisches System bilden kann, $C_{1-4}$-Alkoxy, Phenoxy, Benzoxy, oder $C_{1-7}$-Alkyl-sulfonyl handeln kann), oder
Benzyl (das durch bis zu drei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, das ein zyklisches System bilden kann, $C_{1-4}$-Alkoxy, Phenoxy, Benzoxy, oder $C_{1-7}$-Alkyl-sulfonyl handeln kann), oder
Naphthyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Benzoxy, Phenoxy oder $C_{1-7}$-Alkyl-sulfonyl handeln kann), oder

Benzhydryl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann) oder
Biphenyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann) oder
Triphenylmethyl (das durch bis zu drei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann) oder
Benzhydrylethyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann) oder
Benzhydrylmethyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann), oder
Naphthylmethyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann), oder
Acenaphthyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann), oder
Acenaphthylmethyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann), oder
Pyridyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann), oder
Picolyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann), oder
Benzothiazolyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann), oder
Benzisothiazolyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann), oder
Benzopyrazolyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann), oder
Benzoxazolyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann), oder
Fluorenyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann), oder
Aminofluorenyl (das durch bis zu zwei Substituenten substituiert sein kann, wobei es sich bei diesen Substituenten unabhängig voneinander um $CF_3$, Nitro, Halogen, CONHBzl, $CON(Bzl)_2$, $C_{1-4}$-Alkyl, $C_{1-4}$-Alkoxy, Phenoxy, Benzoxy, oder $C_{1-7}$-Alkyl-sulfonyl handeln kann), oder
Pyrimidyl (das durch bis zu zwei Substituenten substituiert sein kann, wobei es sich bei diesen Substituenten unabhängig voneinander um $CF_3$, Nitro, Halogen, COOEt, CONHBzl, $CON(Bzl)_2$, $C_{1-4}$-Alkyl, das ein zyklisches System bilden kann, $C_{1-4}$-Alkoxy, Phenoxy, Benzoxy, oder $C_{1-7}$-Alkyl-sulfonyl handeln kann), oder
5-gliedriges Heteroaryl [das durch bis zu drei Substituenten substituiert sein kann, wobei es sich bei diesen Substituenten unabhängig voneinander um $CF_3$, Nitro, Halogen, Cyan, COOMe, COO-Et, Thiomethyl, Thioethyl, Thiophenyl, Picolyl, Acetyl, $-CH_2-COOEt$, $CONH_2$, CONHBzl, CON $(Bzl)_2$, $C_{1-4}$-Alkyl, das ein zyklisches System bilden kann, $C_{1-4}$-Alkoxy, Phenoxy, Benzoxy, Phenyl, (das durch bis zu vier Substituenten substituiert sein kann, wobei es sich bei diesen Substituenten unabhängig voneinander um Nitro, $CF_3$, Halogen oder $C_{1-4}$-Alkyl handeln kann), Benzyl (das durch

bis zu vier Substituenten substituiert sein kann, wobei es sich bei diesen Substituenten unabhängig voneinander um Nitro, $CF_3$, Halogen, $C_{1-4}$-Alkyl, Naphthyl, $C_{1-7}$-Alkyl-sulfonyl, Phenylsulfonyl oder $C_{1-4}$-Dialkylamino handeln kann) handeln kann], oder

-$CHR^7$-5-gliedriges Heteroaryl (das durch bis zu zwei Substituenten substituiert sein kann, wobei es sich bei diesen Substituenten unabhängig voneinander um $CF_3$, Nitro, Halogen, CONHBzl, $CON(Bzl)_2$, COOMe, COOEt, $COOCH(CH_3)_2$, $CONH_2$, COOBzl, $C_{1-4}$-Alkyl, $C_{1-4}$-Alkoxy, Phenoxy, Benzoxy, Phenyl, Benzyl, Naphthyl oder $C_{1-7}$-Alkyl-sulfonyl handeln kann [$R^7$ = H, gerade- oder verzweigtkettiges $C_{1-5}$-Alkyl, Benzyl; oder $R^7$ und $R^5$ bilden gemeinsam eine Gruppe $-(CH_2)_3-$ oder $-(CH_2)_4-$]).

4. Verbindungen der Formel I nach Anspruch 1 worin K $R^5$-N-$R^6$ bedeutet, welche gemeinsam Strukturen aus der Gruppe umfassend

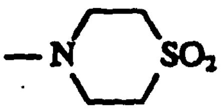
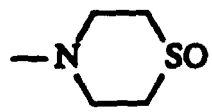
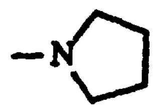
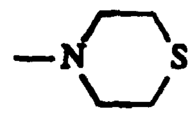
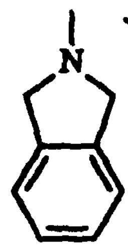
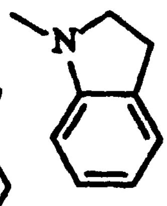
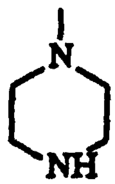
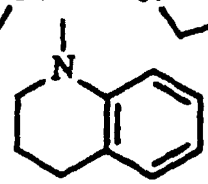
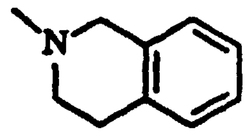
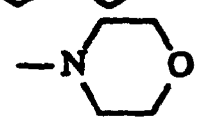
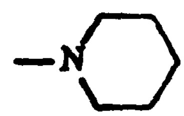
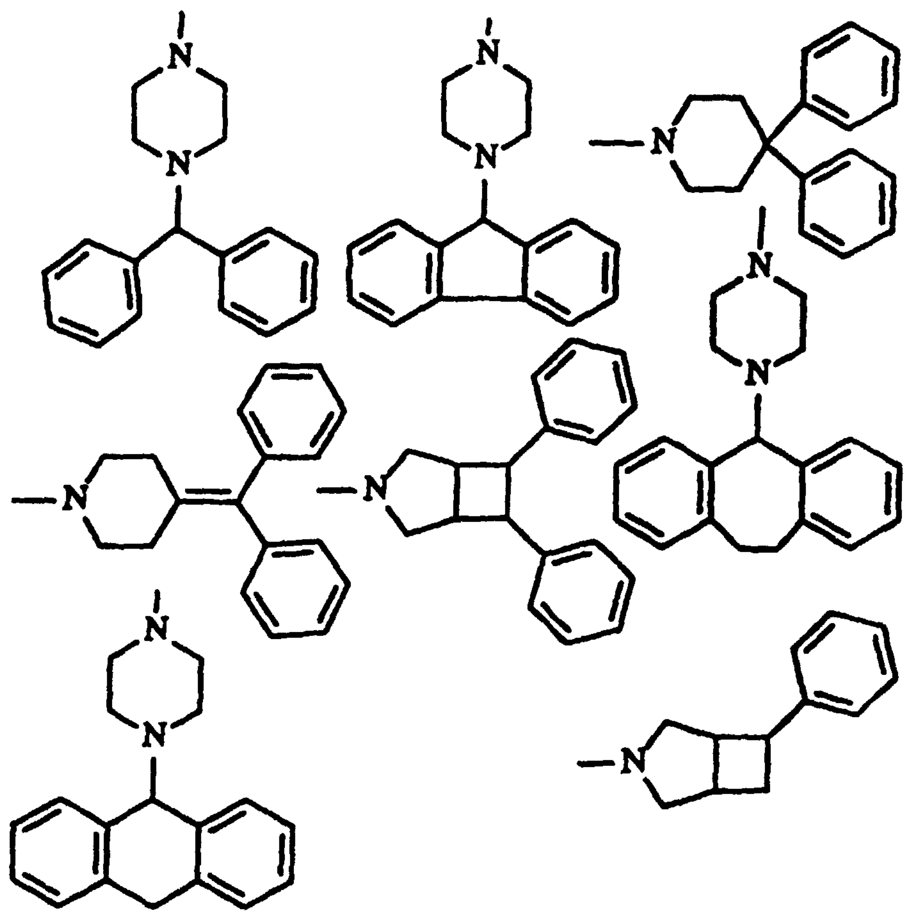

bilden können, die unsubstituiert oder durch einen oder mehrere Substituenten substituiert sein können, wobei diese Substituenten unabhängig voneinander aus der Gruppe umfassend $CF_3$, Nitro, Halogen, Oxo, Cyan, N,N-Dimethylamino, CONHBzl, $CON(Bzl)_2$, $C_{1-6}$-Alkyl, $C_{3-4}$-Alkylengruppe, die ein anelliertes Ringsystem bildet, $C_{1-4}$-Alkoxy, Phenoxy, Benzoxy, Naphthyl, Pyrimidyl, COOEt, COOBzl, $C_{3-6}$-Cycloalkyl, Pyrrolidinyl, Piperidinyl, Thienyl, Pyrrolyl, $-CH_2-CO-NCH(CH_3)_2$, $-CH_2-CO-N(CH_2)_4$, $-CH_2-CO-N(CH_2)_4O$, Benzyl (das durch bis zu drei Substituenten substituiert sein kann, wobei diese Substituenten unabhängig voneinander aus der Gruppe umfassend Nitro, Halogen, $CF_3$, Thiomethyl oder das entsprechende Sulfoxid oder Sulfon, Thioethyl oder das entsprechende Sulfoxid oder Sulfon, $C_{1-4}$-Alkyl und $C_{1-4}$-Alkoxy ausgewählt sind), sowie Phenyl (das durch bis zu drei Substituenten substituiert sein kann, die unabhängig voneinander aus der Gruppe umfassend Nitro, Halogen, $CF_3$, Thiomethyl, Thioethyl, $C_{1-4}$-Alkyl und $C_{1-4}$-Alkoxy ausgewählt sind) ausgewählt sind.

**5.** Verbindungen der Formel I nach Anspruch 1, worin u, v und w null bedeuten und K nicht einen Hydroxy-, Benzoxy-, Phenoxy- oder Alkoxyrest bedeutet.

**6.** Verbindungen der Formel I nach Anspruch 1, worin u und v null bedeuten und K nicht einen Hydroxy- oder Alkoxyrest bedeutet.

**7.** Verbindungen der Formel I nach Anspruch 1, worin u, v und w 1 bedeuten und K einen Hydroxy-, Alkoxy-, Phenoxy- oder Benzyloxyrest bedeutet.

**8.** Verbindungen der Formel I nach Anspruch 1, worin u, und v 1 bedeuten, w 0 bedeutet und K einen Hydroxy-, Alkoxy-, Phenoxy- oder Benzyloxyrest bedeutet.

**9.** Verbindungen der Formel I nach Anspruch 1, worin u 1 bedeutet, v und w 0 bedeuten und K einen Hydroxy-, Alkoxy-, Phenoxy- oder Benzyloxyrest bedeutet.

**10.** Verwendung von Verbindungen der Formel I oder deren Salzen zur Herstellung von Medikamenten für die Behandlung onkologischer Krankheiten.

**11.** Pharmazeutische Zusammensetzung umfassend einen pharmazeutisch unbedenklichen Träger und eine therapeutisch wirksame Menge einer Verbindung nach Anspruch 1.

**12.** Verfahren zur Herstellung von Verbindungen der Formel I nach Anspruch 1, dadurch gekennzeichnet, daß diese nach bekannten Verfahren der Peptidchemie hergestellt werden.

## Revendications

**1.** Un peptide doué d'activité anticancéreuse de la formule I

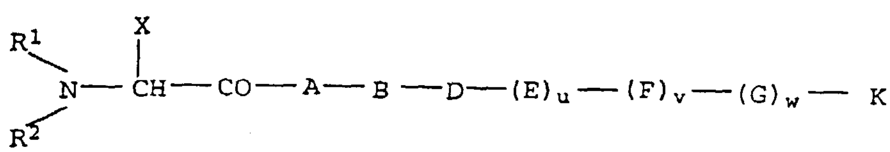

I

où

| | |
|---|---|
| $R^1$ | est un groupe alkyle, cycloalkyle, alkylsulfonyle, fluoroalkyle ou $NR^3R^4$ où $R^3$ et $R^4$ peuvent chacun être l'hydrogène ou un groupe alkyle ; |
| $R^2$ | est l'hydrogène, un groupe alkyle, fluoralkyle, cycloalkyle ou bien |
| $R^1$-N-$R^2$ | ensemble peuvent être |

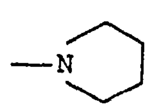

ou

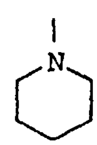

A est un résidu valyle, isoleucyle, leucyle, alloisoleucyle, 3-*tert*-butylalanyle, 2-*tert*-butylglycyle, 3-cyclohexylalanyle, 2-éthylglycyle, 2-cyclohexylglycyle, norleucyle ou norvalyle ;

B est un résidu N-alkyl-valyle, -norvalyle, -leucyle, -isoleucyle, -2-*tert*-butylglycyle, -3-tert-butylalanyle, -3-cyclohexylalanyle, -phénylalanyle ou -2-cyclohexylglycyle ;

D et E sont choisis indépendamment dans la classe formée par les résidus prolyle, homo-prolyle, hydroxyprolyle, thiazolidinyl-4-carbonyle ;

F et G sont choisis indépendamment dans la classe formée par les résidus prolyle, homo-prolyle, hydroxyprolyle, thiazolidinyl-4-carbonyle, 1-aminopentyl-1-carbonyle, valyle, 2-*tert*-butylglycyle, isoleucyle, leucyle, 3-cyclohexylalanyle, phénylalanyle, N-méthylphénylalanyle, tétrahydroisoquinolyl-2-carbonyle, 3-thiazolylalanyle, 3-thiénylalanyle, histidyle, 1-amino-indyl-1-carbonyle, 3-pyridylalanyle, 3-*tert*-butylalanyle, 2-cyclohexylglycyle, norvalyle, norleucyle et 3-naphtylalanyle ;

X est un groupe alkyle, cycloalkyle, $-CH_2$-cyclohexyle ou arylalkyle ;

E et F ensemble peuvent être

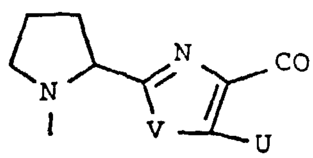

où

V est l'oxygène ou le soufre ;

U est l'hydrogène, un groupe alkyle en $C_1$-$C_4$, phényle ou cycloalkyle ;

u, v et wsont indépendamment 0 ou 1 ; et

K est un groupement hydroxyle, alcoxy, phénoxy, benzyloxy ou un groupement amino substitué ou non substitué ;

et ses sels formés avec des acides physiologiquement tolérés.

**2.** Composés de formule I selon la revendication 1, dans lesquels $R^1$-N-$R^2$ est

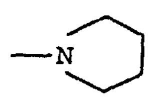

ou

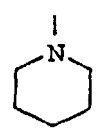

**3.** Composés de formule I selon la revendication 1, dans lesquels K est un groupement amino de la formule $R^5$-N-$R^6$ où

$R^5$ est l'hydrogène ou un groupe hydroxyle, ou alcoxy en $C_1$-$C_7$, ou benzyloxy, ou alkyle en $C_1$-$C_7$, ou fluoroalkyle, ou cycloalkyle en $C_3$-$C_7$, ou benzyle qui peut être substitué par au plus trois substituants qui peuvent être indépendamment un groupe $CF_3$, nitro, alkylsulfonyle en $C_1$-$C_7$, alcoxy en $C_1$-$C_4$, phénoxy, benzoxy, halogéno ou alkyle en $C_1$-$C_4$ ;

$R^6$ est H ou un groupe alkyle en $C_1$-$C_7$, ou cycloalkyle en $C_3$-$C_7$, ou fluoroalkyle, ou phényle (qui peut être substitué par au plus trois substituants qui peuvent être indépendamment un groupe $CF_3$, nitro, halogéno, CONHBzl, $CON(Bzl)_2$, alkyle en $C_1$-$C_4$ qui peut former un système cyclique, alcoxy en $C_1$-$C_4$, phénoxy, benzoxy ou alkylsulfonyle en $C_1$-$C_7$), ou benzyle (qui peut être substitué par au plus trois substituants qui peuvent être indépendamment un groupe $CF_3$, nitro, halogéno, CONHBzl, $CON(Bzl)_2$, alkyle en $C_1$-$C_4$ qui peut former un système cyclique, alcoxy en $C_1$-$C_4$, phénoxy, benzoxy ou alkylsulfonyle en $C_1$-$C_7$), ou

naphtyle (qui peut être substitué par au plus deux substituants qui peuvent être indépendamment un groupe $CF_3$, nitro, halogéno, CONHBzl, $CON(Bzl)_2$, alkyle en $C_1$-$C_4$, alcoxy en $C_1$-$C_4$, benzoxy, phénoxy ou alkylsulfonyle en $C_1$-$C_7$), ou

benzhydryle (qui peut être substitué par au plus deux substituants qui peuvent être indépendamment un groupe $CF_3$, nitro, halogéno, CONHBzl, $CON(Bzl)_2$, alkyle en $C_1$-$C_4$, alcoxy en $C_1$-$C_4$,

phénoxy, benzoxy ou alkylsulfonyle en $C_1$-$C_7$), ou
biphénylyle (qui peut être substitué par au plus deux substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou
triphénylméthyle (qui peut être substitué par au plus trois substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou
benzhydryléthyle (qui peut être substitué par au plus deux substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou
benzhydrylméthyle (qui peut être substitué par au plus deux substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou
naphtylméthyle (qui peut être substitué par au plus deux substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou acénaphtyle (qui peut être substitué par au plus deux substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou
acénaphtylméthyle (qui peut être substitué par au plus deux substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou
pyridyle (qui peut être substitué par au plus deux substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou
picolyle (qui peut être substitué par au plus deux substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou
benzothiazolyle (qui peut être substitué par au plus deux substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou
benzisothiazolyle (qui peut être substitué par au plus deux substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou
benzopyrazolyle (qui peut être substitué par au plus deux substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou
benzoxazolyle (qui peut être substitué par au plus deux substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou
fluorényle (qui peut être substitué par au plus deux substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou
aminofluorényle (qui peut être substitué par au plus deux substituants qui peuvent être indépendamment un groupe $CF_3$, nitro, halogéno, CONHBzl, CON(Bzl)$_2$, alkyle en $C_1$-$C_4$, alcoxy en $C_1$-$C_4$, phénoxy, benzoxy ou alkylsulfonyle en $C_1$-$C_7$), ou
pyrimidyle (qui peut être substitué par au plus deux substituants qui peuvent être indépendamment un groupe $CF_3$, nitro, halogéno, COOEt, CONHBzl, CON(Bzl)$_2$, alkyle en $C_1$-$C_4$ qui peut former un système cyclique, alcoxy en $C_1$-$C_4$, phénoxy, benzoxy ou alkylsulfonyle en $C_1$-$C_7$), ou
hétéroaryle pentagonal [qui peut être substitué par au plus trois substituants qui peuvent être indépendamment un groupe $CF_3$, nitro, halogéno, cyano, COOMe, COOEt, thiométhyle, thioéthyle, thiophényle, picolyle, acétyle, -$CH_2$-COOEt, $CONH_2$, CONHBzl, CON(Bzl)$_2$, alkyle en $C_1$-$C_4$ qui peut former un système cyclique, alcoxy en $C_1$-$C_4$, phénoxy, benzoxy, phényle (qui peut être substitué par au plus quatre substituants qui peuvent être indépendamment un groupe nitro, $CF_3$, halogéno ou alkyle en $C_1$-$C_4$), benzyle (qui peut être substitué par au plus quatre substituants qui peuvent être indépendamment un groupe nitro, $CF_3$, halogéno, alkyle en $C_1$-$C_4$, naphtyle, alkylsulfonyle en $C_1$-$C_7$, phénylsulfonyle ou dialkylamino en $C_1$-$C_4$)], ou
-$CHR^7$-hétéroaryle pentagonal (qui peut être substitué par au plus deux substituants qui peuvent

être indépendamment un groupe $CF_3$, nitro, halogéno, CONHBzl, $CON(Bzl)_2$, COOMe, COOEt, $COOCH(CH_3)_2$, $CONH_2$, COOBzl, alkyle en $C_1$-$C_4$, alcoxy en $C_1$-$C_4$, phénoxy, benzoxy, phényle, benzyle, naphtyle ou alkylsulfonyle en $C_1$-$C_7$ [$R^7$ = H, groupe alkyle en $C_1$-$C_5$ linéaire ou ramifié, groupe benzyle ; ou bien $R^7$ et $R^5$ forment ensemble un groupe $-(CH_2)_3-$ ou $-(CH_2)_4-$]).

**4.** Composés de formule I selon la revendication 1, dans lesquels K est $R^5$-N-$R^6$ qui peut s'assembler en formant des structures choisies dans la classe constituée par

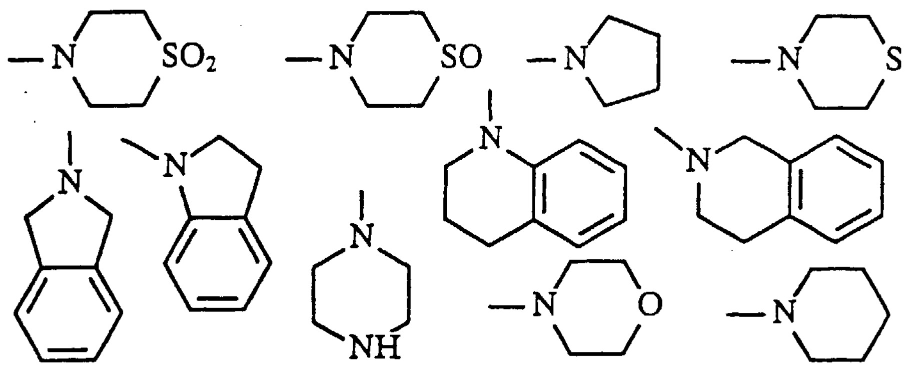

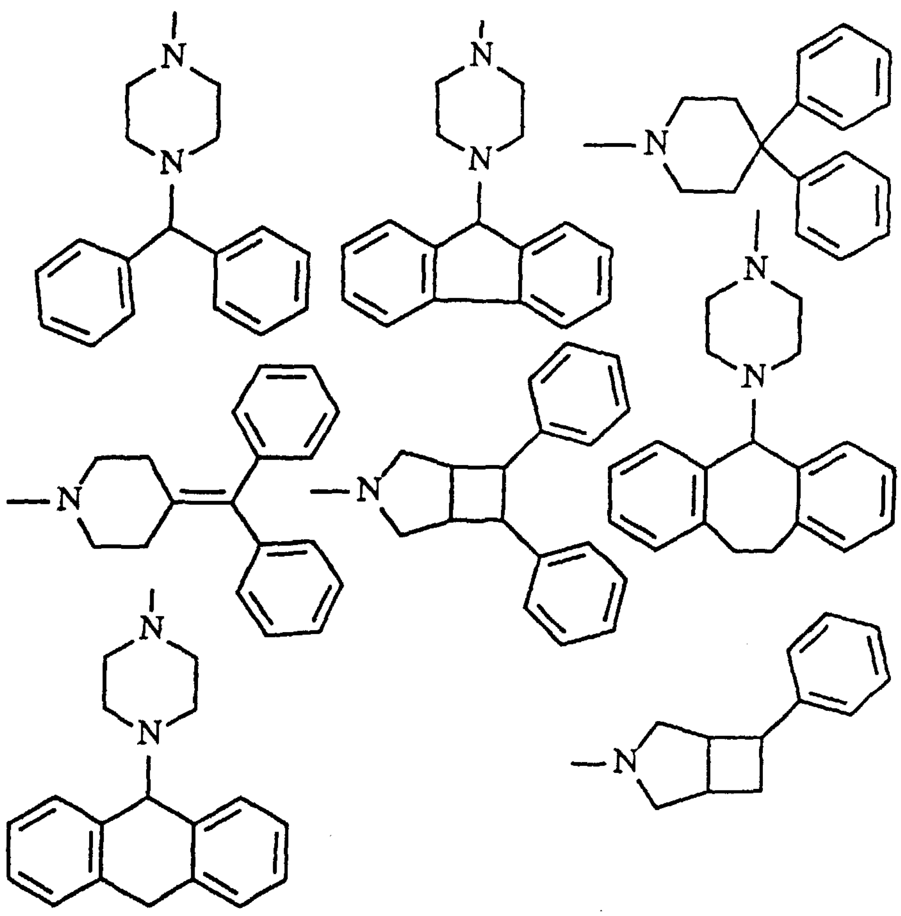

qui peuvent être non substitués ou substitués par un ou plusieurs substituants choisis indépendamment dans la classe formée par les groupes $CF_3$, nitro, halogéno, oxo, cyano, N,N-diméthylamino, CONHBzl, $CON(Bzl)_2$, alkyle en $C_1$-$C_6$, alkylène en $C_3$-$C_4$ formant un système cyclique condensé, alcoxy en $C_1$-$C_4$, phénoxy, benzoxy, naphtyle, pyrimidyle, COOEt, COOBzl, cycloalkyle en $C_3$-$C_6$, pyrrolidinyle, pipéridinyle, thiényle, pyrolyle, $-CH_2-CO-NCH(CH_3)_2$, $-CH_2-CO-N(CH_2)_4$, $-CH_2-CO-N(CH_2)_4O$, benzyle (qui peut être substitué par au plus trois substituants

choisis indépendamment dans la classe formée par les groupes nitro, halogéno, $CF_3$, thiométhyle ou le sulfoxyde ou la sulfone correspondant, thioéthyle ou le sulfoxyde ou la sulfone correspondant, alkyle en $C_1$-$C_4$ et alcoxy en $C_1$-$C_4$) et phényle (qui peut être substitué par au plus trois substituants choisis indépendamment dans la classe formée par les groupes nitro, halogéno, $CF_3$, thiométhyle, thioéthyle, alkyle en $C_1$-$C_4$ et alcoxy en $C_1$-$C_4$).

**5.** Composés de formule I selon la revendication 1, dans lesquels u, v et w sont zéro et K n'est pas un groupe hydroxyle, benzoxy, phénoxy ou alcoxy.

**6.** Composés de formule I selon la revendication 1, dans lesquels u et v sont zéro et K n'est pas un groupe hydroxyle ou alcoxy.

**7.** Composés de formule I selon la revendication 1, dans lesquels u, v et w sont 1 et K est un groupement hydroxyle, alcoxy, phénoxy ou benzyloxy.

**8.** Composés de formule I selon la revendication 1, dans lesquels u et v sont 1, w est 0 et K est un groupement hydroxyle, alcoxy, phénoxy ou benzyloxy.

**9.** Composés de formule I selon la revendication 1, dans lesquels u est 1, v et w sont 0 et K est un groupement hydroxyle, alcoxy, phénoxy ou benzyloxy.

**10.** Utilisation de composés de formule I ou de leurs sels pour la préparation de médicaments destinés au traitement de maladies oncologiques.

**11.** Une composition pharmaceutique comprenant un support pharmaceutiquement acceptable et une quantité à effet thérapeutique d'un composé de la revendication 1.

**12.** Le procédé de préparation de composés de formule I selon la revendication 1, caractérisé par le fait qu'ils sont préparés selon des procédés connus de la chimie des peptides.

Fig. 1: The Boc protective group technique on a polymeric support

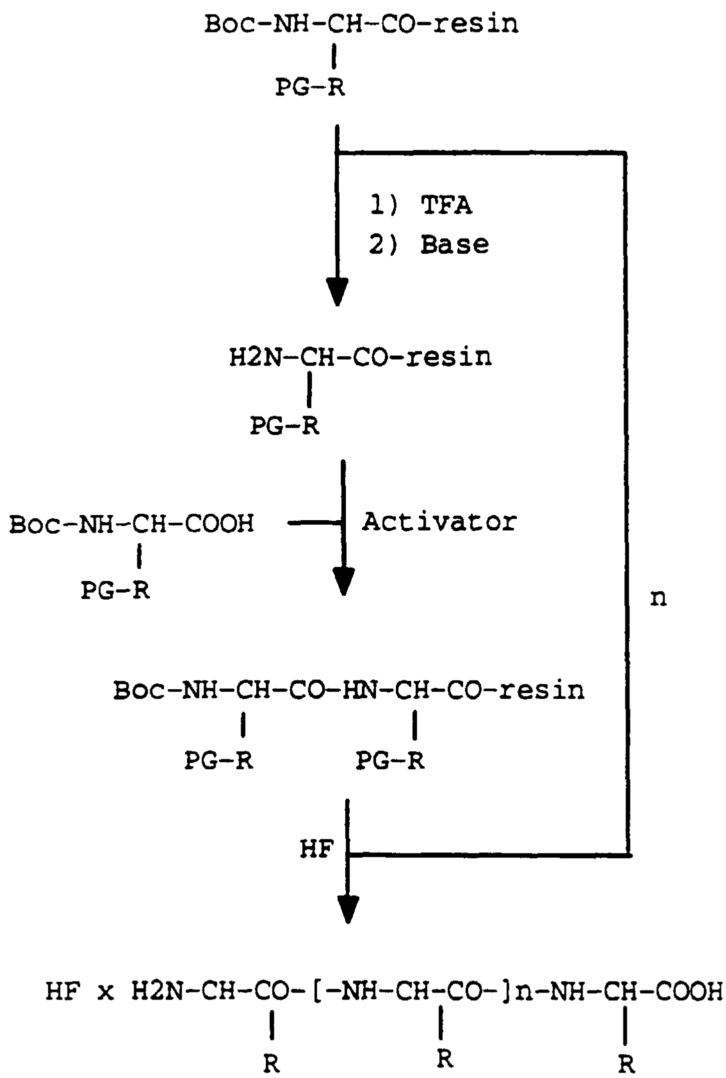


Boc = t-butyloxycarbonyl protective group
PG = side-chain protective group
R = amino-acid side chain

Fig. 2: The Fmoc protective group technique on a polymeric support

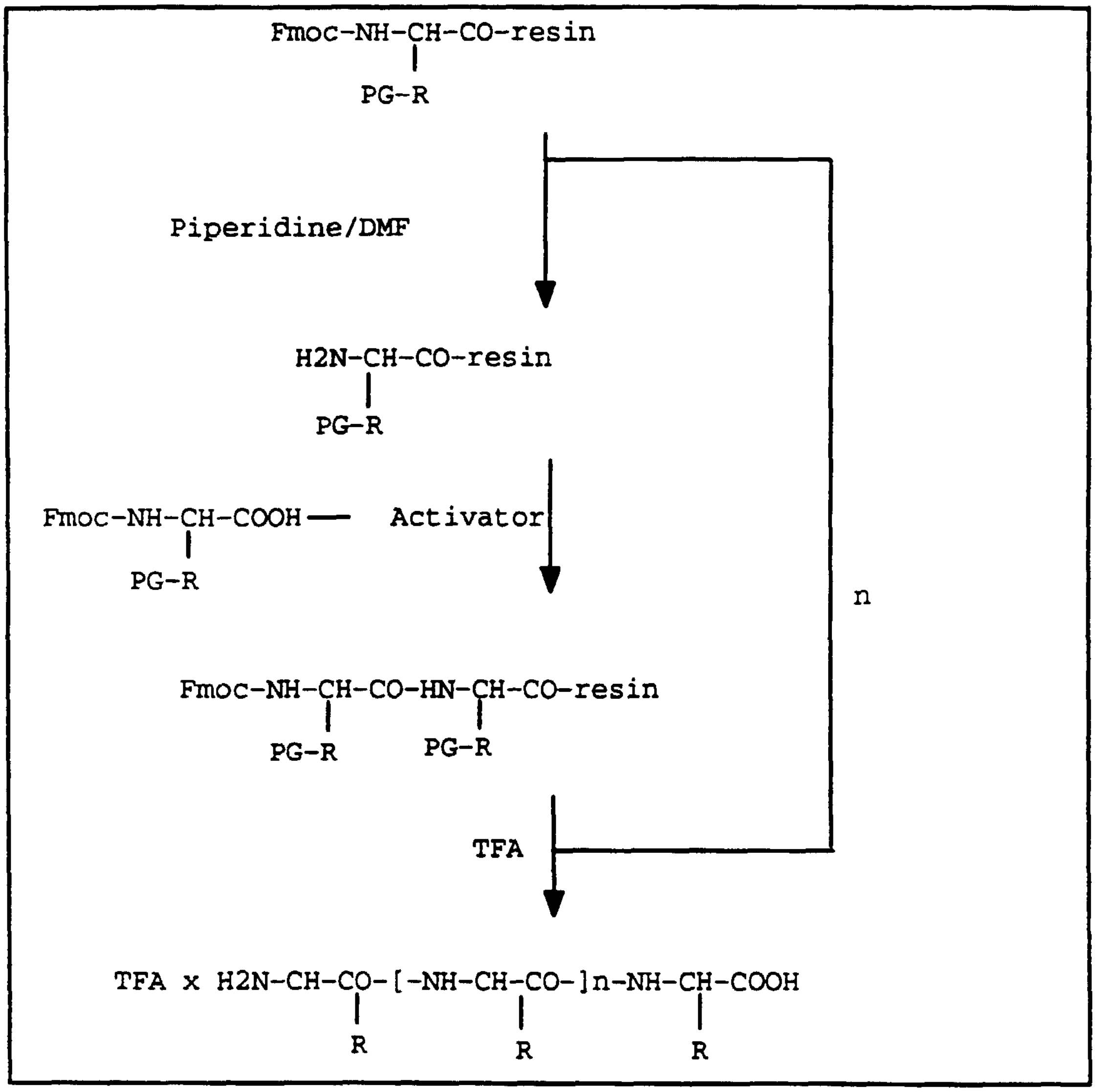


Fmoc = 9-fluorenylmethyloxycarbonyl protective group
PG = side-chain protective group
R = amino-acid side chain

SEQUENCE LISTING

(1) GENERAL INFORMATION

(i) APPLICANT:

(A) BASF Aktiengesellschaft

(B) STREET: Carl-Bosch-Strasse 38
(C) CITY: Ludwigshafen
(E) COUNTRY: Bundesrepublik Deutschland
(F) ZIP: W-6700
(G) TELEPHONE: 0621/6048526
(H) TELEFAX: 0621/6043123
(I) TELEX: 1762175170

(ii) TITLE OF INVENTION: Novel peptides, the preparation and use thereof

(iii) NUMBER OF SEQUENCES: 57

(iv) COMPUTER READABLE FORM:

(A) MEDIUM TYPE: Diskette, 3,5 inch, 2 DD
(B) COMPUTER: IBM AT-compatible, 80286 processor
(C) OPERATING SYSTEM: MS-DOS version 5.0
(D) SOFTWARE: WordPerfect version 5.1

(2) INFORMATION FOR SEQ ID NO: 1:

(i) SEQUENCE CHARACTERISTICS:

(A) LENGTH: 9 amino acids
(B) TYPE: amino acid
(D) TOPOLOGY: linear

(ii) MOLECULE TYPE: peptide
(xi) SEQUENCE DESCRIPTION: SEQ ID NO: 1:

```
Xaa Val Val Xaa Val Pro Pro Val Phe
 1               5
```

(2) INFORMATION FOR SEQ ID NO: 2:

(i) SEQUENCE CHARACTERISTICS:

(A) LENGTH: 5 amino acids
(B) TYPE: amino acid
(D) TOPOLOGY: linear

(ii)MOLECULE TYPE: peptide
(xi) SEQUENCE DESCRIPTION: SEQ ID NO: 2:

```
Xaa Val Xaa Pro Xaa
 1               5
```

(2) INFORMATION FOR SEQ ID NO: 3:

(i) SEQUENCE CHARACTERISTICS:

(A) LENGTH: 7 amino acids
(B) TYPE: amino acid
(D) TOPOLOGY: linear

(ii) MOLECULE TYPE: peptide
(xi) SEQUENCE DESCRIPTION: SEQ ID NO: 3:

```
Xaa Val Xaa Pro Pro Val Phe
 1               5
```

(2) INFORMATION FOR SEQ ID NO: 4:

(i) SEQUENCE CHARACTERISTICS:

(A) LENGTH: 7 amino acids
(B) TYPE: amino acid
(D) TOPOLOGY: linear

(ii)MOLECULE TYPE: peptide
(xi) SEQUENCE DESCRIPTION: SEQ ID NO: 4:

```
Xaa Val Xaa Pro Pro Val Xaa
 1               5
```

(2) INFORMATION FOR SEQ ID NO:5:

(i) SEQUENCE CHARACTERISTICS:

(A) LENGTH: 7 amino acids
(B) TYPE: amino acid
(D) TOPOLOGY: linear

(ii) MOLECULE TYPE: peptide
(xi) SEQUENCE DESCRIPTION: SEQ ID NO: 5:

```
Xaa Val Xaa Pro Pro Val His
 1               5
```

(2) INFORMATION FOR SEQ ID NO: 6:

(i) SEQUENCE CHARACTERISTICS:

(A) LENGTH: 7 amino acids
(B) TYPE: amino acid
(D) TOPOLOGY: linear

(ii)MOLECULE TYPE: peptide
(xi) SEQUENCE DESCRIPTION: SEQ ID NO: 6:

```
Xaa Xaa Xaa Pro Pro Val Trp
 1               5
```

(2) INFORMATION FOR SEQ ID NO: 7:

(i) SEQUENCE CHARACTERISTICS:

(A) LENGTH: 7 amino acids

(B) TYPE: amino acid
(D) TOPOLOGY: linear

(ii)MOLECULE TYPE: peptide
(xi) SEQUENCE DESCRIPTION: SEQ ID NO: 7:

```
Xaa Val Xaa Pro Pro Xaa Phe
 1               5
```

(2) INFORMATION FOR SEQ ID NO: 8:

(i) SEQUENCE CHARACTERISTICS:

(A) LENGTH: 7 amino acids
(B) TYPE: amino acid
(D) TOPOLOGY: linear

(ii)MOLECULE TYPE: peptide
(xi) SEQUENCE DESCRIPTION: SEQ ID NO: 8:

```
Xaa Val Xaa Pro Pro Ile Phe
 1               5
```

(2) INFORMATION FOR SEQ ID NO: 9:

(i) SEQUENCE CHARACTERISTICS:

(A) LENGTH: 7 amino acids
(B) TYPE: amino acid
(D) TOPOLOGY: linear

(ii) MOLECULE TYPE: peptide
(xi) SEQUENCE DESCRIPTION: SEQ ID NO: 9:

```
Xaa Val Xaa Pro Xaa Val Phe
 1               5
```

(2) INFORMATION FOR SEQ ID NO: 10:

(i) SEQUENCE CHARACTERISTICS:

(A) LENGTH: 7 amino acids
(B) TYPE: amino acid
(D) TOPOLOGY: linear

(ii) MOLECULE TYPE: peptide
(xi) SEQUENCE DESCRIPTION: SEQ ID NO: 10:

```
Xaa Val Xaa Xaa Pro Val Phe
 1               5
```

(2) INFORMATION FOR SEQ ID NO: 11:

(i) SEQUENCE CHARACTERISTICS:

(A) LENGTH: 7 amino acids
(B) TYPE: amino acid
(D) TOPOLOGY: linear

(ii) MOLECULE TYPE: peptide
(xi) SEQUENCE DESCRIPTION: SEQ ID NO: 11:

```
Xaa Val Xaa Pro Pro Val Phe
 1               5
```

(2) INFORMATION FOR SEQ ID NO: 12:

(i) SEQUENCE CHARACTERISTICS:

(A) LENGTH: 7 amino acids
(B) TYPE: amino acid
(D) TOPOLOGY: linear

(ii)MOLECULE TYPE: peptide
(xi) SEQUENCE DESCRIPTION: SEQ ID NO: 12:

```
Xaa Ile Xaa Pro Pro Val Phe
 1               5
```

(2) INFORMATION FOR SEQ ID NO: 13:

(i) SEQUENCE CHARACTERISTICS:

(A) LENGTH: 7 amino acids
(B) TYPE: amino acid
(D) TOPOLOGY: linear

(ii)MOLECULE TYPE: peptide
(xi) SEQUENCE DESCRIPTION: SEQ ID NO: 13:

```
Xaa Xaa Xaa Pro Pro Val Phe
 1               5
```

(2) INFORMATION FOR SEQ ID NO: 14:

(i) SEQUENCE CHARACTERISTICS:

(A) LENGTH: 7 amino acids
(B) TYPE: amino acid
(D) TOPOLOGY: linear

(ii) MOLECULE TYPE: peptide
(xi) SEQUENCE DESCRIPTION: SEQ ID NO: 14:

Xaa Leu Xaa Pro Pro Val Phe
1 5

(2) INFORMATION FOR SEQ ID NO: 15:

(i) SEQUENCE CHARACTERISTICS:

(A) LENGTH: 7 amino acids
(B) TYPE: amino acid
(D) TOPOLOGY: linear

(ii) MOLECULE TYPE: peptide
(xi) SEQUENCE DESCRIPTION: SEQ ID NO: 15:

Xaa Val Xaa Pro Pro Phe Phe
1 5

(2) INFORMATION FOR SEQ ID NO: 16:

(i) SEQUENCE CHARACTERISTICS:

(A) LENGTH: 6 amino acids
(B) TYPE: amino acid
(D) TOPOLOGY: linear

(ii) MOLECULE TYPE: peptide
(xi) SEQUENCE DESCRIPTION: SEQ ID NO: 16:

Xaa Val Xaa Pro Pro Val
1 5

(2) INFORMATION FOR SEQ ID NO: 17:

(i) SEQUENCE CHARACTERISTICS:

(A) LENGTH: 6 amino acids
(B) TYPE: amino acid
(D) TOPOLOGY: linear

(ii) MOLECULE TYPE: peptide
(xi) SEQUENCE DESCRIPTION: SEQ ID NO: 17:

Xaa Val Xaa Pro Pro Xaa
1 5

(2) INFORMATION FOR SEQ ID NO: 18:

(i) SEQUENCE CHARACTERISTICS:

(A) LENGTH: 6 amino acids

(B) TYPE: amino acid
(D) TOPOLOGY: linear

(ii) MOLECULE TYPE: peptide
(xi) SEQUENCE DESCRIPTION: SEQ ID NO: 18:

```
Xaa Val Xaa Pro Pro Val
 1               5
```

(2) INFORMATION FOR SEQ ID NO: 19:

(i) SEQUENCE CHARACTERISTICS:

(A) LENGTH: 6 amino acids
(B) TYPE: amino acid
(D) TOPOLOGY: linear

(ii) MOLECULE TYPE: peptide
(xi) SEQUENCE DESCRIPTION: SEQ ID NO: 19:

```
Xaa Xaa Xaa Pro Pro Val
 1               5
```

(2) INFORMATION FOR SEQ ID NO: 20:

(i) SEQUENCE CHARACTERISTICS:

(A) LENGTH: 6 amino acids
(B) TYPE: amino acid
(D) TOPOLOGY: linear

(ii) MOLECULE TYPE: peptide
(xi) SEQUENCE DESCRIPTION: SEQ ID NO: 20:

```
Xaa Val Xaa Xaa Pro Val
 1               5
```

(2) INFORMATION FOR SEQ ID NO: 21:

(i) SEQUENCE CHARACTERISTICS:

(A) LENGTH: 6 amino acids
(B) TYPE: amino acid
(D) TOPOLOGY: linear

(ii) MOLECULE TYPE: peptide
(xi)SEQUENCE DESCRIPTION: SEQ ID NO: 21:

```
Xaa Val Xaa Pro Xaa Val
 1               5
```

(2) INFORMATION FOR SEQ ID NO: 22:

(i) SEQUENCE CHARACTERISTICS:

(A) LENGTH: 4 amino acids
(B) TYPE: amino acid
(D) TOPOLOGY: linear

(ii)MOLECULE TYPE: peptide
(xi)SEQUENCE DESCRIPTION: SEQ ID NO: 22:

```
Xaa Val Xaa Xaa
 1
```

(2) INFORMATION FOR SEQ ID NO: 23:

(i) SEQUENCE CHARACTERISTICS:

(A) LENGTH: 5 amino acids
(B) TYPE: amino acid
(D) TOPOLOGY: linear

(ii) MOLECULE TYPE: peptide
(xi) SEQUENCE DESCRIPTION: SEQ ID NO: 23:

```
Xaa Xaa Xaa Pro Pro
 1               5
```

(2) INFORMATION FOR SEQ ID NO: 24:

(i) SEQUENCE CHARACTERISTICS:

(A) LENGTH: 4 amino acids
(B) TYPE: amino acid
(D) TOPOLOGY: linear

(ii) MOLECULE TYPE: peptide
(xi) SEQUENCE DESCRIPTION: SEQ ID NO: 24:

```
Xaa Val Xaa Pro
 1
```

(2) INFORMATION FOR SEQ ID NO: 25:

(i) SEQUENCE CHARACTERISTICS:

(A) LENGTH: 7 amino acids
(B) TYPE: amino acid
(D) TOPOLOGY: linear

(ii) MOLECULE TYPE: peptide
(xi) SEQUENCE DESCRIPTION: SEQ ID NO: 25:

Xaa Val Xaa Pro Pro Val Xaa
1 5

(2) INFORMATION FOR SEQ ID NO: 26:

(i) SEQUENCE CHARACTERISTICS:

(A) LENGTH: 6 amino acids
(B) TYPE: amino acid
(D) TOPOLOGY: linear

(ii) MOLECULE TYPE: peptide
(xi) SEQUENCE DESCRIPTION: SEQ ID NO: 26:

Xaa Val Xaa Pro Xaa Phe
1 5

(2) INFORMATION FOR SEQ ID NO: 27:

(i) SEQUENCE CHARACTERISTICS:

(A) LENGTH: 7 amino acids
(B) TYPE: amino acid
(D) TOPOLOGY: linear

(ii) MOLECULE TYPE: peptide
(xi) SEQUENCE DESCRIPTION: SEQ ID NO: 27:

Xaa Val Xaa Pro Pro Phe Phe
1 5

(2) INFORMATION FOR SEQ ID NO: 28:

(i) SEQUENCE CHARACTERISTICS:

(A) LENGTH: 7 amino acids
(B) TYPE: amino acid
(D) TOPOLOGY: linear

(ii) MOLECULE TYPE: peptide
(xi) SEQUENCE DESCRIPTION: SEQ ID NO: 28:

Xaa Val Xaa Pro Pro Xaa Xaa
1 5

(2) INFORMATION FOR SEQ ID NO: 29:

(i) SEQUENCE CHARACTERISTICS:

(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO: 29:

```
Xaa Val Xaa Pro Pro Leu Phe
 1               5
```

(2) INFORMATION FOR SEQ ID NO: 30:

(i) SEQUENCE CHARACTERISTICS:

(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO: 30:

```
Xaa Val Xaa Pro Pro Ile Phe
 1               5
```

(2) INFORMATION FOR SEQ ID NO: 31:

(i) SEQUENCE CHARACTERISTICS:

(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO: 31:

```
Xaa Val Xaa Pro Pro Val Ala
 1               5
```

(2) INFORMATION FOR SEQ ID NO: 32:

(i) SEQUENCE CHARACTERISTICS:

(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO: 32:

```
Xaa Val Xaa Pro Pro Val Phe
 1               5
```

(2) INFORMATION FOR SEQ ID NO: 33:

(i) SEQUENCE CHARACTERISTICS:

(A) LENGTH: 6 amino acids
(B) TYPE: amino acid
(D) TOPOLOGY: linear

(ii) MOLECULE TYPE: peptide
(xi) SEQUENCE DESCRIPTION: SEQ ID NO: 33:

```
Xaa Val Xaa Pro Pro Val
 1
```

(2) INFORMATION FOR SEQ ID NO: 34:

(i) SEQUENCE CHARACTERISTICS:

(A) LENGTH: 5 amino acids
(B) TYPE: amino acid
(D) TOPOLOGY: linear

(ii) MOLECULE TYPE: peptide
(xi) SEQUENCE DESCRIPTION: SEQ ID NO: 34:

```
Xaa Ile Xaa Pro Xaa
 1              5
```

(2) INFORMATION FOR SEQ ID NO: 35:

(i) SEQUENCE CHARACTERISTICS:

(A) LENGTH: 6 amino acids
(B) TYPE: amino acid
(D) TOPOLOGY: linear

(ii) MOLECULE TYPE: peptide
(xi) SEQUENCE DESCRIPTION: SEQ ID NO: 35:

```
Xaa Val Xaa Pro Xaa Val
 1              5
```

(2) INFORMATION FOR SEQ ID NO: 36:

(i) SEQUENCE CHARACTERISTICS:

(A) LENGTH: 7 amino acids
(B) TYPE: amino acid
(D) TOPOLOGY: linear

(ii) MOLECULE TYPE: peptide
(xi) SEQUENCE DESCRIPTION: SEQ ID NO: 36:

```
Xaa Val Xaa Pro Xaa Leu Phe
 1               5
```

(2) INFORMATION FOR SEQ ID NO: 37:

(i) SEQUENCE CHARACTERISTICS:

(A) LENGTH: 6 amino acids
(B) TYPE: amino acid
(D) TOPOLOGY: linear

(ii) MOLECULE TYPE: peptide
(xi) SEQUENCE DESCRIPTION: SEQ ID NO: 37:

```
Xaa Val Xaa Pro Xaa Xaa
 1               5
```

(2) INFORMATION FOR SEQ ID NO: 38:

(i) SEQUENCE CHARACTERISTICS:

(A) LENGTH: 6 amino acids
(B) TYPE: amino acid
(D) TOPOLOGY: linear

(ii) MOLECULE TYPE: peptide
(xi) SEQUENCE DESCRIPTION: SEQ ID NO: 38:

```
Xaa Leu Xaa Pro Pro Xaa
 1               5
```

(2) INFORMATION FOR SEQ ID NO: 39:

(i) SEQUENCE CHARACTERISTICS:

(A) LENGTH: 6 amino acids
(B) TYPE: amino acid
(D) TOPOLOGY: linear

(ii) MOLECULE TYPE: peptide
(xi) SEQUENCE DESCRIPTION: SEQ ID NO: 39:

```
Xaa Leu Xaa Pro Pro Xaa
 1               5
```

(2) INFORMATION FOR SEQ ID NO: 40:

(i) SEQUENCE CHARACTERISTICS:

(A) LENGTH: 6 amino acids

(B) TYPE: amino acid
(D) TOPOLOGY: linear

(ii) MOLECULE TYPE: peptide
(xi) SEQUENCE DESCRIPTION: SEQ ID NO: 40:

```
Xaa Xaa Xaa Pro Pro Val
 1               5
```

(2) INFORMATION FOR SEQ ID NO: 41:

(i) SEQUENCE CHARACTERISTICS:

(A) LENGTH: 7 amino acids
(B) TYPE: amino acid
(D) TOPOLOGY: linear

(ii) MOLECULE TYPE: peptide
(xi) SEQUENCE DESCRIPTION: SEQ ID NO: 41:

```
Xaa Xaa Xaa Pro Pro Val Phe
 1               5
```